# EUROPEAN PATENT SPECIFICATION

(11) **EP 1 590 341 B1**
(45) Date of publication and mention of the grant of the patent: **17.06.2009**
(21) Application number: 04701058.2
(22) Date of filing: 09.01.2004
(51) Int. Cl.: C07D 401/12, C07D 213/75

(54) **2-AMINOPYRIDINE SUBSTITUTED HETEROCYCLES AS INHIBITORS OF CELLULAR PROLIFERATION**
2-AMINOPYRIDIN-SUBSTITUIERTEHETEROCYCLEN ALS INHIBITOREN DER ZELLULÄREN PROLIFERATION
HETEROCYCLES 2-AMINOPYRIDINES SUBSTITUES UTILISES COMME INHIBITEURS DE LA PROLIFERATION CELLULAIRE

(30) Priority: 17.01.2003 US 440805 P
(43) Date of publication of application: 02.11.2005
(73) Proprietor: Warner-Lambert Company LLC, New York, NY 10017 (US)
(72) Inventor: FLAMME, Cathlin M., Pfizer Global R & D, Ann Arbor, MI 48105 (US); McNAMARA, Dennis J., Pfizer Global R & D, Ann Arbor, MI 48105 (US); REPINE, Joseph T., Pfizer Global R & D, Ann Arbor, MI 48105 (US); TOOGOOD, Peter L., Pfizer Global R & D, Ann Arbor, MI 48105 (US); VANDERWEL, Scott N., Pfizer Global R & D, Ann Arbor, MI 48105 (US); WARMUS, Joseph S., Pfizer Global R & D, Ann Arbor, MI 48105 (US)
(74) Representative: Dekker, Henrike Cornelie Christine
(86) International application number: PCT/IB2004/000091
(87) International publication number: WO 2004/065378

(56) References cited:
- WO-A-01/19825
- WO-A-01/64655
- WO-A-01/64656
- WO-A-01/70741
- WO-A-98/33798
- WO-A-99/09030
- WO-A-99/61444
- WO-A-02/076954

## Description

### FIELD OF THE INVENTION

This invention relates to 2-aminopyridine substituted heterocycles that are selective inhibitors of the serine-threonine kinase, cyclin-dependent kinase 4. The compounds of the invention are useful for the treatment of inflammation, and cell proliferative diseases such as cancer and restenosis.

### BACKGROUND OF THE INVENTION

Cyclin-dependent kinases and related serine/threonine protein kinases are important cellular enzymes that perform essential functions in regulating cell division and proliferation. The cyclin-dependent kinase catalytic units are activated by regulatory subunits known as cyclins. At least 16 mammalian cyclins have been identified (Johnson D.G. and Walker C.L., Annu. Rev. Pharmacol. Toxicol. 1999;39:295-312). Cyclin B/Cdk1, Cyclin A/Cdk2, Cyclin E/Cdk2, Cyclin D/Cdk4, Cyclin D/Cdk6, and probably other heterodimers including Cdk3 and Cdk7 are important regulators of cell cycle progression. Additional functions of Cyclin/Cdk heterodimers include regulation of transcription, DNA repair, differentiation and apoptosis (Morgan D.O., Annu. Rev. Cell. Dev. Biol. 1997; 13261-13291).

Increased activity or temporally abnormal activation of cyclin-dependent kinases has been shown to result in the development of human tumors (Sherr C.J., Science 1996;274:1672-1677). Indeed, human tumor development is commonly associated with alterations in either the Cdk proteins themselves or their regulators (Cordon-Cardo C., Am. J. Pathol. 1995;147:545-560; Karp J. E. and Broder S., Nat. Med. 1995;1:309-320; Hall M. et al., Adv. Cancer Res. 1996;68:67-108). Naturally occurring protein inhibitors of Cdks such as p16 and p27 cause growth inhibition in vitro in lung cancer cell lines (Kamb A., Curr. Top. Microbiol. Immunol. 1998;227:139-148).

Small molecule Cdk inhibitors may also be used in the treatment of cardiovascular disorders such as restenosis and atherosclerosis and other vascular disorders that are due to aberrant cell proliferation. Vascular smooth muscle proliferation and intimal hyperplasia following balloon angioplasty are inhibited by over-expression of the cyclin-dependent kinase inhibitor protein. Moreover, the purine Cdk2 inhibitor CVT-313 (Ki = 95 nM) resulted in greater than 80% inhibition of neointima formation in rats.

Cdk inhibitors can be used to treat diseases caused by a variety of infectious agents, including fungi, protozoan parasites such as *Plasmodium falciparum*, and DNA and RNA viruses. For example, cyclin-dependent kinases are required for viral replication following infection by herpes simplex virus (HSV) (Schang L.M. et al., J. Virol. 1998;72:5626) and Cdk homologs are known to play essential roles in yeast.

Selective Cdk inhibitors can be used to ameliorate the effects of various autoimmune disorders. The chronic inflammatory disease rheumatoid arthritis is characterized by synovial tissue hyperplasia; inhibition of synovial tissue proliferation should minimize inflammation and prevent joint destruction. In a rat model of arthritis, joint swelling was substantially inhibited by treatment with an adenovirus expressing a Cdk inhibitor protein p.16. Cdk inhibitors are effective against other disorders of cell proliferation including psoriasis (characterized by keratinocyte hyperproliferation), glomerulonephritis, and lupus.

Certain Cdk inhibitors are useful as chemoprotective agents through their ability to inhibit cell cycle progression of normal untransformed cells (Chen, et al. J. Natl. Cancer Institute, 2000;92:1999-2008). Pre-treatment of a cancer patient with a Cdk inhibitor prior to the use of cytotoxic agents can reduce the side effects commonly associated with chemotherapy. Normal proliferating tissues are protected from the cytotoxic effects by the action of the selective Cdk inhibitor.

Review articles on small molecule inhibitors of cyclin dependent kinases have noted the difficulty of identifying compounds that inhibit specific Cdk proteins without inhibiting other enzymes. Thus, despite their potential to treat a variety of diseases, no Cdk inhibitors are currently approved for commercial use (Fischer, P. M., Curr. Opin. Drug Discovery 2001, 4, 623-634; Fry, D. W. & Garrett, M. D. Curr. Opin. Oncologic, Endocrine & Metabolic Invest. 2000, 2, 40-59; Webster, K. R. & Kimball, D. Emerging Drugs 2000, 5, 45-59; Sielecki, T. M. et al. J. Med. Chem. 2000, 43, 1-18.).

WO 98/33798 discloses a class of pyrido[2,3-d]pyrimidines that display selectivity for Cdks versus other protein kinases. These compounds are distinct from the 6-aryl-pyrido[2,3-d]pyrimidines (WO 96/15128; WO 96/34867), which display the opposite selectivity, inhibiting tyrosine kinases in preference to cyclin-dependent kinases. Moreover, they represent a new structural class when compared to either the pyrimidines and 3,4-dihydropyrimidines of international patent application WO 99/61444 or the naphthyridones described in WO 99/09030.

WO 01/70741 disclosed one class of compounds, 5-alkyl-pyrido[2,3-d]pyrimidines, that exhibit selectivity for Cdk4 inhibition. A further class of Cdk4 selective compound was disclosed in U.S. Pat. App. Ser. No. 10/345,778). However, there exists a need for other small molecular weight, highly selective inhibitors of Cdk4 that are orally bioavailable and useful for treating a wide variety of cell proliferative diseases and disorders, cancer, infections, autoimmune diseases, gout, kidney disease, and neurodegenerative diseases and disorders.

### SUMMARY OF THE INVENTION

This invention provides compounds of the formula I: 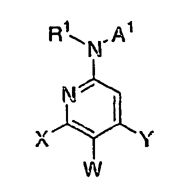 wherein:
A¹ is a monocyclic or bicyclic heteroaromatic ring system selected from: 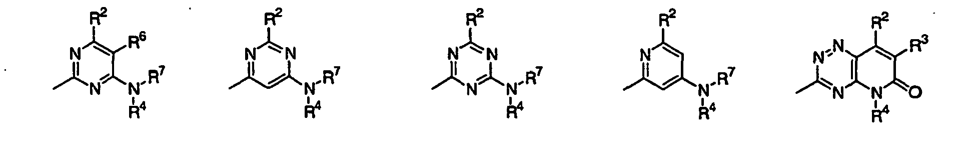 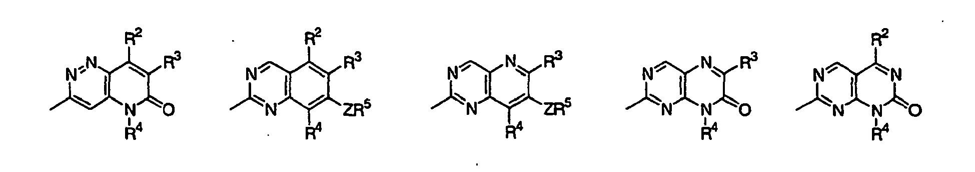 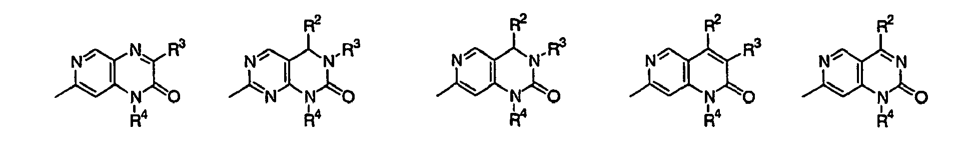 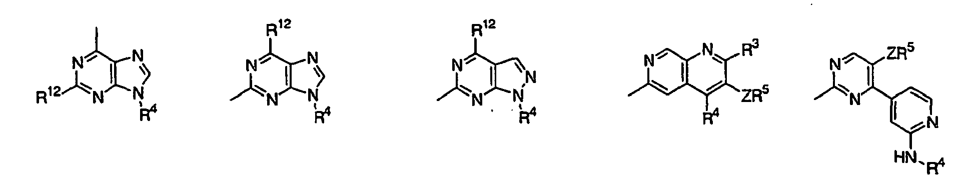
wherein:
R¹ is, in each instance, independently, hydrogen, C₁-C₆ alkyl, C₁-C₆ alkenyl, C₁-C₆ acyl, aryloxycarbonyl, alkyloxycarbonyl, or trialkylsilyl;
R², R³, R⁴, R⁵, R⁸, R⁹, R¹⁰ and R¹¹ are, in each instance, independently selected from hydrogen, C₁-C₁₀ alkyl, C₁-C₁₀ alkyl amino, C₃-C₇ cycloalkyl, aryl, heteroaryl, and heterocyclyl;
R⁶ is independently, in each instance, selected from hydrogen, halogen, nitrile, nitro, C₁-C₈ alkyl, C₂-C₈ alkenyl, C₂-C₈ alkynyl, alkylcarbonyl, alkoxycarbonyl, C₃-C₇ cycloalkyl, nitro, OR⁸, SR⁸, NR⁸R⁹, N(O)R⁸R⁹, P(O)(Ok⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R,⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-aryl, (CR⁸R⁹)ₙ-heteroaryl, -T(CH₂)ₘQR⁸, -C(O)T(CH₂)ₘQR⁸, NR⁸C(O)T(CH₂)ₘQR⁸, and -CR⁸=CR⁹C(O)R¹⁰;
R⁷ is independently, in each instance, hydrogen, C₁-C₁₀ acyl, alkyloxycarbonyl, aryloxycarbonyl, C₁-C₈ alkyl, or C₂-C₈ alkenyl;
R¹² is independently, in each instance, hydrogen, C₁-C₁₀ acyl, arylalkyl, alkylamino, arylamino, or alkylamino;
R⁸ and R⁹ may optionally form a carbocyclic group containing 3-7 members preferably 5-6 members, up to four of which are optionally heteroatoms independently selected from oxygen, sulfur, and nitrogen, wherein the carbocyclic group is unsubstituted or substituted with one, two, or three groups said groups in each instance independently selected from halogen, hydroxy, hydroxyalkyl, nitrile, lower alkyl, lower alkoxy, alkoxycarbonyl, alkylcarbonyl, alkylcarbonylamino, aminoalkyl, trifluoromethyl, N-hydroxyacetamide, trifluoromethylalkyl, amino, or mono or dialkylamino, (CH₂)ₙC(O)NR¹⁰R¹¹, and O(CH₂)ₙC(O)OR¹⁰;
T is, in each instance, independently, O, S, NR⁹, N(O)R9, or CR⁹R¹⁰;
Q is, in each instance, independently, O, S, NR⁹, N(O)R⁹, CO₂, O(CH₂)ₙ-heteroaryl, O(CH₂)ₙS(O)ₘR⁹, or (CH₂)-heteroaryl;
X and Y are in each instance independently selected from hydrogen, halogen, nitrile, C₁-C₆ alkyl, C₁-C₆ alkylcarbonyl, C₁-C₆ alkoxycarbonyl, nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(Q)(OR¹⁰)(OR¹¹), (CR⁸R⁹)ₙ-aryl, (CR⁸R⁹)ₙ-heteroaryl, -T(CH₂)ₘQR⁸, -C(O)T(CH₂)ₘQR⁸, NR⁸C(O)T(CH₂)ₘQR⁸, and -CR⁸=CR⁹C(O)R¹⁰;
W is selected from hydrogen, halogen, C₁-C₈ alkyl, C₃-C₇ cycloalkyl, C₁-C₈ alkoxy, C₁-C₈ alkoxyalkyl, C₁-C₈ haloalkyl, C₁-C₈ hydroxyalkyl, C₂-C₈ alkenyl, C₂-C₈ alkynyl, nitrile, nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-aryl, (CR⁸R⁹)ₙ-heteroaryl, -T(CH₂)ₘQR⁸, -C(O)T(CH₂)ₘQR⁸, NR⁸C(O)T(CH₂)ₘQR⁹, and -CR⁸=CR⁹C(O)R¹⁰;
W and one of X or Y may optionally together with the carbon to which they are attached may form an aromatic ring that is fused to the pyridine ring depicted in the structural formula I, wherein said aromatic ring may contain up to three heteroatoms and optionally substituted by up to 4 groups independently selected from halogen, hydroxy, hydroxyalkyl, lower alkyl, lower alkoxy, alkoxycarbonyl, alkylcarbonyl, alkylcarbonylamino, and aminoalkyl, aminoalkylcarbonyl, trifluoromethyl, trifluoromethylalkyl, trifluoromethylalkylaminoalkyl, amino, mono- or dialkylamino, N-hydroxyacetamido, aryl, heteroaryl, carboxyalkyl, nitrile, NR^{S}SO₂R⁹, C(O)NR⁸R⁹, NR⁸C(O)R⁹, C(O)OR⁸, C(O)NR⁸SO₂R⁹, (CH₂)ₙS(O)ₙR⁸; (CH₂)ₙ-heteroaryl, O(CH₂)ₙ-heteroaryl, (CH₂)ₙC(O)NR⁸R⁹, O(CH₂)ₙC(O)OR⁸, (CH₂)ₙSO₂NR⁸R⁹, and C(O)R⁸_{:}
Z is in each instance, independently, O or NR⁶;
m is an integer of from 1-6,
n is an integer of from 0-6, and
the pharmaceutically acceptable salts, esters, amides, and prodrugs thereof.

This invention identifies 2-aminopyrimidine substituted heterocyclic compounds that are useful for treating uncontrolled cell proliferative diseases, including, but not limited to, proliferative diseases such as cancer, restenosis and rheumatoid arthritis. In addition, these compounds and salts thereof are useful for treating inflammation and inflammatory diseases. In addition, these compounds have utility as antiinfective agents. Moreover, these compounds and salts thereof have utility as chemoprotective agents. Many of the compounds of the invention display unexpected improvements in selectivity for the serine/threonine kinase, cyclin-dependent kinase 4. The compounds are readily synthesized and can be administered to patients by a variety of methods.

Compounds of formula I may contain chiral centers and therefore may exist in different enantiomeric and diastereomeric forms. This invention relates to all optical isomers and all stereoisomers of compounds of the formula I, both as racemic mixtures and as individual enantiomers and diastereoisomers of such compounds, and mixtures thereof, and to all pharmaceutical compositions and methods of treatment defined above that contain or employ them, respectively.

As the compounds of formula I of this invention may possess asymmetric centers, they are capable of occurring in various stereoisomeric forms or configurations. Hence, the compounds can exist in separated (+)- and (-)-optically active forms, as well as mixtures thereof. The present invention includes all such forms within its scope. Individual isomers can be obtained by known methods, such as optical resolution, optically selective reaction, or chromatographic separation in the preparation of the final product or its intermediate.

The compounds of the present invention can exist in unsolvated forms as well as solvated forms, including hydrated forms. In general, the solvated forms, including hydrated forms, are equivalent to unsolvated forms and are intended to be encompassed within the scope of the present invention.

The present invention also includes isotopically labelled compounds, which are identical to those recited in Formula I, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I of this invention and prodrugs thereof can generally be prepared by carrying out the procedures disclosed in the Schemes and/or in the Examples and Preparations below, by substituting a readily available isotopically labelled reagent for a non-isotopically labelled reagent.

The compounds of Formula I are capable of further forming pharmaceutically acceptable formulations comprising salts, including but not limited to acid addition and/or base salts, and solvates of a compound of Formula I.

This invention also provides pharmaceutical formulations comprising a therapeutically effective amount of a compound of Formula I or a therapeutically acceptable salt thereof and a pharmaceutically acceptable carrier, diluent, or excipient therefor. All of these forms are within the present invention.

By "alkyl," in the present invention is meant a straight or branched hydrocarbon radical having from 1 to 10 carbon atoms and includes, for example, methyl, ethyl, n-propyl, isopropyl, n-butyl, sec-butyl, isobutyl, tert-butyl, n-pentyl, iso-pentyl, n-hexyl, and the like.

"Alkenyl" means straight and branched hydrocarbon radicals having from 2 to 8 carbon atoms and at least one double bond and includes, but is not limited to, ethenyl, 3-buten-1-yl, 2-ethenylbutyl, 3-hexen-1-yl, and the like. The term "alkenyl" includes, cycloalkenyl, and heteroalkenyl in which 1 to 3 heteroatoms selected from O, S, N or substituted nitrogen may replace carbon atoms.

"Alkynyl" means straight and branched hydrocarbon radicals having from 2 to 8 carbon atoms and at least one triple bond and includes, but is not limited to, ethynyl, 3-butyn-1-yl, propynyl, 2-butyn-1-yl, 3-pentyn-l-yl, and the like.

"Cycloalkyl" means a monocyclic or polycyclic hydrocarbyl group having from 3 to 8 carbon atoms, for instance, cyclopropyl, cycloheptyl, cyclooctyl, cyclodecyl, cyclobutyl, adamantyl, norpinanyl, decalinyl, norbornyl, cyclohexyl, and cyclopentyl. Also included are rings in which 1 to 3 heteroatoms replace carbons. Such groups are termed "heterocyclyl," which means a cycloalkyl group also bearing at least one heteroatom selected from O, S, N or substituted nitrogen. Examples of such groups include, but are not limited to, oxiranyl, pyrrolidinyl, piperidyl, tetrahydropyran, and morpholine.

By "alkoxy," is meant straight or branched chain alkyl groups having 1-10 carbon atoms and linked through oxygen. Examples of such groups include, but are not limited to, methoxy, ethoxy, propoxy, isopropoxy, n-butoxy, sec-butoxy, tert-butoxy, pentoxy, 2-pentyloxy, isopentoxy, neopentoxy, hexoxy, 2-hexoxy, 3-hexoxy, and 3-methylpentoxy. In addition, alkoxy refers to polyethers such as -O-(CH₂)₂-O-CH₃, and the like.

"Acyl" means an alkyl or aryl (Ar) group having from 1-10 carbon atoms bonded through a carbonyl group, i.e., R-C(O)-. For example, acyl includes, but is not limited to, a C₁-C₆ alkanoyl, including substituted alkanoyl, wherein the alkyl portion can be substituted by NR⁸R⁹ or a carboxylic or heterocyclic group. Typical acyl groups include acetyl, benzoyl, and the like.

The alkyl, alkenyl, alkoxy, and alkynyl groups described above are optionally substituted, preferably by 1 to 3 groups selected from NR⁸R⁹, phenyl, substituted phenyl, keto, amino, alkyl, thio C₁-C₆ alkyl, C₁-C₆ alkoxy, hydroxy, carboxy, C₁-C₆ alkoxycarbonyl, halo, nitrile, cycloalkyl, and a 5- or 6-membered carbocyclic ring or heterocyclic ring having 1 or 2 heteroatoms selected from nitrogen, substituted nitrogen, oxygen, and sulfur. "Substituted nitrogen" means nitrogen bearing C₁-C₆ alkyl or (CH₂)ₚPh where p is 1, 2, or 3. Perhalo and polyhalo substitution is also included.

Examples of substituted alkyl groups include, but are not limited to, 2-aminoethyl, 2-hydroxyethyl, pentachloroethyl, trifluoromethyl, 2-diethylaminoethyl, 2-dimethylaminopropyl, ethoxycarbonylmethyl, 3-phenylbutyl, methanylsulfanylmethyl, methoxymethyl, 3-hydroxypentyl, 2-carboxybutyl, 4-chlorobutyl, 3-cyclopropylpropyl, pentafluoroethyl, 3-morpholinopropyl, piperazinylmethyl, and 2-(4-methylpiperazinyl)ethyl.

Examples of substituted alkynyl groups include, but are not limited to, 2-methoxyethynyl, 2-ethylsulfanylethynyl, 4-(1-piperazinyl)-3-(butynyl), 3-phenyl-5-hexynyl, 3-diethylamino-3-butynyl, 4-chloro-3-butynyl, 4-cyclobutyl-4-hexenyl, and the like.

Typical substituted alkoxy groups include aminomethoxy, trifluoromethoxy, 2-diethylaminoethoxy, 2-ethoxycarbonylethoxy, 3-hydroxypropoxy, 6-carboxhexyloxy, and the like.

Further, examples of substituted alkyl, alkenyl, and alkynyl groups include, but are not limited to, dimethylaminomethyl, carboxymethyl, 4-dimethylamino-3-buten-1-yl, 5-ethylmethylamino-3-pentyn-1-yl, 4-morpholinobutyl, 4-tetrahydropyrinidylbutyl, 3-imidazolidin-1-ylpropyl, 4-tetrahydrothiazol-3-yl-butyl, phenylmethyl, 3-chlorophenylmethyl, and the like.

The term "anion" means a negatively charged counterion such as chloride, bromide, trifluoroacetate, and triethylammonium.

The term "aryl", as used herein, unless otherwise indicated, includes an aromatic ring system with no heteroatoms having a single ring (e.g., phenyl), multiple rings (e.g., biphenyl), or multiple condensed rings in which at least one is aromatic, (e.g., 1,2,3,4-tetrahydronaphthyl, naphthyl, anthryl, or phenanthryl), which can be mono-, di-, or trisubstituted with, e.g., halogen, lower alkyl, lower alkoxy, lower alkylthio, trifluoromethyl, lower acyloxy, aryl, heteroaryl, and hydroxy. A preferred aryl is phenyl which can be either unsubstituted or substituted with one, two or three substituents selected from the group consisting of halo, (C₁-C₄)alkyl optionally substituted with from one to three halogen atoms and (C₁-C₄)alkoxy optionally substituted with from one to three halogen atoms. The term "aryloxy", as used herein, unless otherwise indicated, means "aryl-O-", wherein "aryl" is as defined above.

The term "heteroaryl", as used herein, unless otherwise indicated, includes an aromatic heterocycle containing five or six ring members, of which from 1 to 4 can be heteroatoms selected, independently, from N, S and O, and which rings can be unsubstituted, monosubstituted or disubstituted with substituents selected, independently, from the group consisting of halo, (C₁-C₄)alkyl, and (C₁-C₄)alkoxy, said alkyl and alkoxy groups being optionally substituted with from one to three halogen atoms, wherein said halogen atom is preferably a fluorine atoms. Such heteroaryl groups include, but are not limited to, for example, thienyl, furanyl, thiazolyl, triazolyl, imidazolyl, isoxazolyl, oxazolyl, oxadiazolyl, tetrazolyl, pyridyl, thiadiazolyl, oxadiazolyl, oxathiadiazolyl, thiatriazolyl, pyrimidinyl, quinolinyl, isoquinolinyl, napthyridinyl, phthalimidyl, benzimidazolyl, and benzoxazolyl. A preferred heteroaryl is pyridine.

The term "heteroaryloxy", as used herein, unless otherwise indicated, means "heteroaryl-O", wherein heteroaryl is as defined above.

The term "leaving group", as used herein, refers to any group (X) that can depart from the carbon to which it is attached carrying with it the two electrons that comprise the bond between the leaving group and that carbon (the X-C bond). Typical leaving groups include but are not limited to: halides (e.g. F, Cl⁻, Br⁻, I⁻), esters, (e.g. acetate), sulfonate esters (e.g. mesylate, tosylate), ethers (EtO⁻, PhO⁻), sulfides (PhS⁻, MeS⁻), sulfoxides, and sulfones.

The term "one or more substituents", as used herein, refers to a number of substituents that equals from one to the maximum number of substituents possible based on the number of available bonding sites.

By the terms "halo" or "halogen" in the present invention is meant fluorine, bromine, chlorine, and iodine.

The term "cancer" includes, but is not limited to, the following cancers: cancers of the breast, ovary, cervix, prostate, testis, esophagus, stomach, skin, lung, bone, colon, pancreas, thyroid, biliary passages, buccal cavity and pharynx (oral), lip, tongue, mouth, pharynx, small intestine, colon-rectum, large intestine, rectum, brain and central nervous system, glioblastoma, neuroblastoma, keratoacanthoma, epidermoid carcinoma, large cell carcinoma, adenoma, adenocarcinoma, follicular carcinoma, undifferentiated carcinoma, papillary carcinoma, seminoma, melanoma, sarcoma, bladder carcinoma, liver carcinoma, kidney carcinoma, myeloid disorders, lymphoid disorders, Hodgkin's Disease, hairy cell leukemia, and other leukemias.

The term "treating", as used herein, refers to reversing, alleviating, inhibiting the progress of, or preventing the disorder or condition to which such term applies, or preventing one or more symptoms of such condition or disorder. The term "treatment", as used herein, refers to the act of treating, as "treating" is defined immediately above. The term "treating" as used herein may be applied to any suitable mammal. Such mammals include, but are not limited to, canines, felines, bovines, ovines, equines, humans and the like.

The term "pharmaceutically acceptable salts, esters, amides, and prodrugs" as used herein refers to those salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention.

The term "salts" refers to the relatively non-toxic, inorganic and organic acid or base addition salts of compounds of the present invention. These salts can be prepared in situ during the final isolation and purification of the compounds or by separately reacting the purified compound in its free base or free acid form with a suitable organic or inorganic acid or base and isolating the salt thus formed. In so far as the compounds of formula I of this invention are basic compounds, they are all capable of forming a wide variety of different salts with various inorganic and organic acids. Although such salts must be pharmaceutically acceptable for administration to animals, it is often desirable in practice to initially isolate the base compound from the reaction mixture as a pharmaceutically unacceptable salt and then simply convert to the free base compound by treatment with an alkaline reagent and thereafter convert the free base to a pharmaceutically acceptable acid addition salt. The acid addition salts of the basic compounds of Formula I are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Such acid addition salts may be prepared from inorganic acids. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate, laurylsulphonate and isethionate salts, and the like.

Such acid addition salts may also be prepared from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. and the like. Representative salts include acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like.

Pharmaceutically acceptable base addition salts are formed with metals or amines, such as alkali and alkaline earth metals, or of organic amines. The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

Examples of metals used as cations are sodium, potassium, magnesium, calcium, and the like. Examples of suitable amines are N,N'-dibenzylethylenediamine, chloroprocaine, choline, diethanolamine, ethylenediamine, N-methylglucamine, and procaine; see, for example, Berge et al., supra.

Pharmaceutically acceptable base addition salts may include cations based on the alkali and alkaline earth metals, such as sodium, lithium, potassium, calcium, magnesium and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, N,N'-dibenzylethylenediamine, chloroprocaine, choline, diethanolamine, ethylenediamine, N-methylglucamine, and procaine and the like; see, for example, Berge et al., supra. Also contemplated are the salts of amino acids such as arginate, gluconate, galacturonate, and the like. (See, for example, Berge S.M. et al., "Pharmaceutical Salts," J. Pharm. Sci., 1977;66:1-19 which is incorporated herein by reference.)

Examples of pharmaceutically acceptable, non-toxic esters of the compounds of this invention include C₁-C₆ alkyl esters wherein the alkyl group is a straight or branched chain. Acceptable esters also include C₅-C₇ cycloalkyl esters as well as arylalkyl esters such as, but not limited to benzyl. C₁-C₄ alkyl esters are preferred. Esters of the compounds of the present invention may be prepared according to conventional methods "March's Advanced Organic Chemistry, 5th Edition". M. B. Smith & J. March, John Wiley & Sons, 2001.

Examples of pharmaceutically acceptable, non-toxic amides of the compounds of this invention include amides derived from ammonia, primary C₁-C₆ alkyl amines and secondary C₁-C₆ dialkyl amines wherein the alkyl groups are straight or branched chain. In the case of secondary amines the amine may also be in the form of a 5- or 6-membered heterocycle containing one nitrogen atom. Amides derived from ammonia, C₁-C₃ alkyl primary amines and C₁-C₂ dialkyl secondary amines are preferred. Amides of the compounds of the invention may be prepared according to conventional methods such as "March's Advanced Organic Chemistry, 5th Edition". M. B. Smith & J. March, John Wiley & Sons, 2001.

The term "prodrug" refers to compounds that are rapidly transformed in vivo to yield the parent compound of the above formulae, for example, by hydrolysis in blood. A thorough discussion is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are hereby incorporated by reference.

Preferred compounds of the present invention are those having the formula IA: 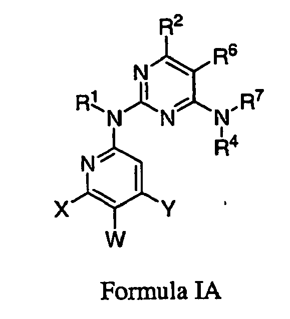 wherein:
R¹, R², R⁴, R⁵, R⁶, R⁷, R⁸, R⁹, R¹⁰, R¹¹ T, Q, W, X, Y, m and n are as defined as for Formula I.

In one preferred embodiment of the present invention R¹ and R² are independently, in each instance, hydrogen.

In a further preferred embodiment of the present invention R⁴ is alkyl.

In a most preferred embodiment of the present invention R⁶ is halogen or COR⁸.

In another preferred embodiment of the present invention W is NR⁸R⁹.

In a more preferred embodiment of the present invention X and Y are hydrogen.

In a preferred embodiment of the present invention R³ is halogen, or C₁-C₈ alkyl.

Preferred embodiments of the present invention include, but are not limited to, the compounds listed below:
4-Cyclopentylamino-2-(5-piperazin-1-yl-pyridin-2-ylamino)-pyrimidine-5-carbonitrile,
N4-Cyclopentyl-5-nitro-N2-(5-piperazin-l-yl-pyridin-2-yl)-pyrimidine-2,4-diamine,
4-Cyclopentylamino-2-(5-piperazin-1-yl-pyridin-2-ylamino)-pyrimidine-5-carbaldehyde,
4-Cyclopentylamino-2-(5-piperazin-1-yl-pyridin-2-ylamino)-pyrimidine-5-carboxylic acid ethyl ester,
4-Cyclopentylamino-2-(5-piperazin-1-yl-pyridin-2-ylamino)-pyrimidine-5-carboxylic acid methyl ester,
[4-Cyclopentylamino-2-(5-piperazin-1-yl-pyridin-2-ylamino)-pyrimidin-5-yl]-methanol,
1-[4-Cyclopentylamino-2-(5-piperazin-1-yl-pyridin-2-ylamino)-pyrimidin-5-yl]-ethanone,
3-[4-Cyclopentylamino-2-(5-piperazin-1-yl-pyridin-2-ylamino)-pyrimidin-5-yl]-but-2-enoic acid ethyl ester,
4-Amino-2-(5-piperazin-1-yl-pyridin-2-ylamino)-pyrimidine-5-carbonitrile,
5-Nitro-N2-(5-piperazin-1-yl-pyridin-2-yl)-pyrimidine-2,4-diamine,
4-Amino-2-(5-piperazin-1-yl-pyridin-2-ylamino)-pyrimidine-5-carbaldehyde,
4-Amino-2-(5-piperazin-1-yl-pyridin-2-ylamino)-pyrimidine-5-carboxylic acid ethyl ester,
4-Amino-2-(5-piperazin-1-yl-pyridin-2-ylamino)-pyrimidine-5-carboxylic acid methyl ester,
[4-Amino-2-(5-piperazin-1-yl-pyidin-2-ylamino)-pyrimidin-5-yl]-methanol,
1-[4-Amino-2-(5-piperazin-1-yl-pyridin-2-ylamino)-pyrimidin-5-yl]-ethanone,
3-[4-Amino-2-(5-piperazin-1-yl-pyridin-2-ylamino)-pyrimidin-5-yl]-but-2-enoic acid ethyl ester,
4-Cyclopentylamino-2-(5-pyrrolidin-1-yl-pyridin-2-ylamino)-pyrimidine-5-carbonitrile,
N2-[5-(3-Amino-pyrrolidin-1-yl)-pyridin-2-yl]-N4-cyclopentyl-5-nitro-pyrimidine-2,4-diamine,
4-Cyclopentylamino-2-(5-morpholin-4-yl-pyridin-2-ylamino)-pyrimidine-5-carbaldehyde,
4-Cyclopentylamino-2-(3,4,5,6-tetrahydro-2H-[1,3']bipyridinyl-6'-ylamino)-pyrimidine-5-carboxylic acid ethyl ester,
4-Cyclopentylamino-6-methyl-2-(5-piperazin-1-yl-pyridin-2-ylamino)-pyrimidine-5-carboxylic acid methyl ester,
{2-[5-(Bis-methoxymethyl-amino)-pyridin-2-ylamino]-4-cyclopentylamino-pyrimidin-5-yl}-methanol,
1-[4-Benzylamino-2-(5-piperazin-1-yl-pyridin-2-ylamino)-pyrimidin-5-yl]-ethanone,
4-[4-Cyclopentylamino-2-(5-piperazin-1-yl-pyridin-2-ylamino)-pyrimidin-5-yl]-pent-3-en-2-one,
4-Amino-2-(pyridin-2-ylamino)-pyrimidine-5-carbonitrile,
5-Nitro-N2-pyridin-2-yl-pyrimidine-2,4-diamine,
4-Amino-2-(pyridin-2-ylamino)-pyrimidine-5-carbaldehyde,
4-Amino-2-(pyridin-2-ylamino)-pyrimidine-5-carboxylic acid ethyl ester,
5-Bromo-N2-(5-piperazin-1-yl-pyridin-2-yl)-pyrimidine-2,4-diamine,
[4-Amino-2-(5-morpholin-4-yl-pyridin-2-ylamino)-pyrimidin-5-yl]-methanol,
1-[4-Amino-2-(5-morpholin-4-yl-pyridin-2-ylamino)-pyrimidin-5-yl]-ethanone,
[6-(5-Acetyl-4-amino-pyrimidin-2-ylamino)-pyridin-3-yloxy]-acetic acid,
4-Cyclopentylamino-2-(4-hydroxymethyl-5-pyrrolidin-1-yl-pyridin-2-ylamino)-pyrimidine-5-carbonitrile,
N2-[5-(3-Amino-pyrrolidin-1-yl)-6-chloro-pyridin-2-yl]-N4-cyclopentyl-5-nitro-pyrimidine-2,4-diamine,
2-(5-Bromo-pyridin-2-ylamino)-4-cyclopentylamino-pyrimidine-5-carbaldehyde,
4-Cyclopentylamino-2-(1H-pyrrolo[3,2-b]pyridin-5-ylamino)-pyrimidine-5-carboxylic acid ethyl ester,
4-Cyclopentylamino-2-(4,6-dichloro-5-piperazin-1-yl-pyridin-2-ylamino)-6-methyl-pyrimidine-5-carboxylic acid methyl ester,
2-(2-{5-[Bis-(2-methoxy-ethyl)-amino]-pyridin-2-ylamino}-4-cyclopentylamino-pyrimidin-5-yl)-2-methyl-propan-1-ol,
1-[4-Phenylamino-2-(5-piperazin-1-yl-pyridin-2-ylamino)-pyrimidin-5-yl]-ethanone,
4-[4-(3-Hydroxy-cyclopentylamino)-2-(5-piperazin-1-yl-pyridin-2-ylamino)-pyrimidin-5-yl]-pent-3-en-2-one,
4-[5-Cyano-2-(pyridin-2-ylamino)-pyrimidin-4-ylamino]-cyclohexanecarboxylic acid,
2-(4-Amino-5-nitro-pyrimidin-2-ylamino)-isonicotinic acid,
4-Amino-6-methyl-2-(pyridin-2-ylamino)-pyrimidine-5-carbaldehyde,
5-Iodo-N2-pyridin-2-yl-pyrimidine-2,4-diamine,
N-[5-Bromo-2-(5-piperazin-1-yl-pyridin-2-ylamino)-pyrimidin-4-yl]-acrylamide,
N2-(5-Piperazin-1-yl-pyridin-2-yl)-5-prop-1-ynyl-pyrimidine-2,4-diamine,
5-[2-(4-Fluoro-phenyl)-ethyl]-N2-(5-piperazin-1-yl-pyridin-2-yl)-pyrimidine-2,4-diamine,
[6-(4-Amino-5-propenyl-pyrimidin-2-ylamino)-pyridin-3-yloxy]-acetic acid,
5-Bromo-N4-cyclopentyl-N2-(5-pyrrolidin-1-yl-pyidin-2-yl)-pyrimidine-2,4-diamine,
N2-[5-(3-Amino-pyrrolidin-1-yl)-6-chloro-pyridin-2-yl]-5-bromo-N4-cyclopentyl-pyrimidine-2,4-diamine,
5-Bromo-N4-cyclopentyl-N2-(5-piperazin-1-yl-pyridin-2-yl)-pyrimidine-2,4-diamine,
5-Bromo-N4-cyclopentyl-N2-(1H-pyrrolo[3,2-b]pyridin-5-yl)-pyrimidine-2,4-diamine,
5-Bromo-N4-cyclopentyl-N2-(4,6-dichloro-5-piperazin-1-yl-pyridin-2-yl)-6-methyl-pyrimidine-2,4-diamine,
N2-{5-[Bis-(2-methoxy-ethyl)-amino]-pyridin-2-yl}-5-bromo-N4-cyclopentyl-pyrimidine-2,4-diamine,
5-Bromo-N4-phenyl-N2-(5-piperazin-1-yl-pyridin-2-yl)-pyrimidine-2,4-diamine,
3-[5-Bromo-2-(5-piperazin-1-yl-pyridin-2-ylamino)-pyrimidin-4-ylamino]-cyclopentanol,
N4-Cyclopentyl-5-iodo-N2-(5-pyrrolidin-1-yl-pyridin-2-yl)-pyrimidine-2,4-diamine,
N2-[5-(3-Amino-pyrrolidin-1-yl)-6-chloro-pyridin-2-yl]-N4-cyclopentyl-5-iodo-pyrimidine-2,4-diamine,
N4-Cyclopentyl-5-iodo-N2-(5-piperazin-1-yl-pyridin-2-yl)-pyrimidine-2,4-diamine,
N4-Cyclopentyl-5-iodo-N2-(1H-pyrrolo[3,2-b]pyridin-5-yl)-pyrimidine-2,4-diamine,
4-[6-(5-Bromo-4-cyclopentylamino-pyrimidin-2-ylamino)-pyridin-3-yl]-piperazine-1-carboxylic acid tert-butyl ester,
4-[6-(4-Cyclopentylamino-5-formyl-pyrimidin-2-ylamino)-pyridin-3-yl]-piperazine-1-carboxylic acid tert-butyl ester,
4-[6-(5-Acetyl-4-cyclopentylamino-pyrimidin-2-ylamino)-pyridin-3-yl]-piperazine-1-carboxylic acid tert-butyl ester,
2-[5-(4-tert-Butoxycarbonyl-piperazin-1-yl)-pyridin-2-ylamino]-4-cyclopentylamino-pyrimidine-5-carboxylic acid ethyl ester,
N-Cyclopentyl-N'-(5-piperazin-1-yl-pyridin-2-yl)-pyrimidine-4,6-diamine,
N-Isopropyl-N'-(5-piperazin-1-yl-pyridin-2-yl)-pyrimidine-4,6-diamine,
4-[6-(6-Cyclopentylamino-pyrimidin-4-ylamino)-pyridin-3-yl]-piperazine-1-carboxylic acid tert-butyl ester,
N-[5-(3-Amino-pyrrolidin-1-yl)-pyridin-2-yl]-N'-cyclopentyl-pyrimidine-4,6-diamine,
4-{6-[4-Cyclopentylamino-5-(1-methyl-3-oxo-but-1-enyl)-pyrimidin-2-ylamino]-pyridin-3-yl}-piperazine-1-carboxylic acid tert-butyl ester,
N-Cyclopentyl-N'-(5-piperazin-1-yl-pyridin-2-yl)-[1,3,5]triazine-2,4-diamine,
1-[4-Cyclopentylamino-2-(5-piperazin-1-yl-pyridin-2-ylamino)-pyrimidin-5-yl]-ethanone,
5-Bromo-N4-cyclopentyl-N2-(5-piperazin-1-yl-pyridin-2-yl)-pyridine-2,4-diamine,
4-Cyclopentylamino-6-(5-piperazin-1-yl-pyridin-2-ylamino)-nicotinonitrile, N4-Cyclopentyl-5-nitro-N2-(5-piperazin-1-yl-pyridin-2-yl)-pyridine-2,4-diamine,
4-Cyclopentylamino-6-(5-piperazin-1-yl-pyridin-2-ylamino)-pyridine-3-carbaldehyde,
4-Cyclopentylamino-6-(5-piperazin-1-yl-pyridin-2-ylamino)-nicotinic acid ethyl ester,
4-Cyclopentylamino-6-(5-piperazin-1-yl-pyridin-2-ylamino)-nicotinic acid methyl ester,
[4-Cyclopentylamino-6-(5-piperazin-1-yl-pyridin-2-ylamino)-pyridin-3-yl]-methanol,
1-[4-Cyclopentylamino-6-(5-piperazin-1-yl-pyridin-2-ylamino)-pyridin-3-yl]-ethanone,
3-[4-Cyclopentylamino-6-(5-piperazin-1-yl-pyridin-2-ylamino)-pyridin-3-yl]-but-2-enoic acid ethyl ester,
(5-Cyclopentyl-5,6-dihydro-pyrido[2,3-e][1,2,4]triazin-3-yl)-(5-piperazin-1-yl-pyridin-2-yl)-amine,
(8-Cyclopentyl-7-methoxy-quinazolin-2-yl)-(5-piperazin-1-yl-pyridin-2-yl)-amine,
(8-Cyclopentyl-7-methoxy-pyrido[3,2-d]pyrimidin-2-yl)-(5-piperazin-1-yl-pyridin-2-yl)-amine,
6-Acetyl-8-cyclopentyl-2-(5-piperazin-1-yl-pyridin-2-ylamino)-8H-pteridin-7-one,
3-Acetyl-1-cyclopentyl-7-(5-piperazin-1-yl-pyridin-2-ylamino)-1H-pyrido[3,4-b]pyrazin-2-one,
1-Cyclopentyl-3-ethyl-4-methyl-7-(5-piperazin-1-yl-pyridin-2-ylamino)-3,4-dihydro-1H-pyrimido[4,5-d]pyrimidin-2-one,
1-Cyclopentyl-3-ethyl-4-methyl-7-(5-piperazin-1-yl-pyridin-2-ylamino)-3,4-dihydro-1H-pyrido[4,3-d]pyrimidin-2-one,
3-Acetyl-1-cyclopentyl-4-methyl-7-(5-piperazin-1-yl-pyridin-2-ylamino)-1H-[1,6]naphthyridin-2-one,
(9-Isopropyl-6-methyl-9H-purin-2-yl)-(5-piperazin-1-yl-pyridin-2-yl)-amine,
2-[9-Isopropyl-6-(5-piperazin-1-yl-pyridin-2-ylamino)-9H-purin-2-ylamino]-ethanol,
N2-(4-Amino-cyclohexyl)-9-cyclopentyl-N6-(5-piperazin-1-yl-pyridin-2-yl)-9H-purine-2,6-diamine,
2-[9-Isopropyl-6-(5-piperazin-1-yl-pyridin-2-ylamino)-9H-purin-2-ylamino]-3-methyl-butan-1-ol,
(1-Isopropyl-4-methyl-1H-pyrazolo[3,4-d]pyrimidin-6-yl)-(5-piperazin-1-yl-pyridin-2-yl)-amine,
2-[1-Isopropyl-4-(5-piperazin-1-yl-pyridin-2-ylamino)-1H-pyrazolo[3,4-d]pyrimidin-6-ylamino]-ethanol,
N6-(4-Amino-cyclohexyl)-1-cyclopentyl-N4-(5-piperazin-1-yl-pyridin-2-yl)-1H-pyrazolo[3,4-d]pyrimidine-4,6-diamine,
2-[1-Isopropyl-4-(5-piperazin-1-yl-pyridin-2-ylamino)-1H-pyrazolo[3,4-d]pyrimidin-6-ylamino]-3-methyl-butan-1-ol,
5-Cyclopentyl-7-(1-hydroxy-ethyl)-8-methyl-3-(5-piperazin-1-yl-pyridin-2-ylamino)-5H-pyrido[3,2-c]pyridazin-6-one,
5-Cyclopentyl-8-methyl-3-(5-piperazin-1-yl-pyridin-2-ylamino)-5H-pyrido[3,2-c]pyridazin-6-one,
7-Benzyl-5-cyclopentyl-3-(5-piperazin-1-yl-pyridin-2-ylamino)-5H-pyrido[3,2-c]pyridazin-6-one,
(2-Ethyl-4-isopropyl-3-methoxy-[1,7]naphthyridin-6-yl)-pyridin-2-yl-amine,
(2,4-Diisopropyl-3-methoxy-[1,7]naphthyridin-6-yl)-(5-isopropenyl-pyridin-2-yl)-amine,
[4-(2-Ethylamino-pyridin-4-yl)-pyrimidin-2-yl]-(5-piperazin-1-yl-pyridin-2-yl)-amine,
[4-(5-Ethyl-2-methylamino-pyridin-4-yl)-pyrimidin-2-yl]-(5-morpholin-4-yl-pyridin-2-yl)-amine,
[5-Methoxy-4-(2-methylamino-pyridin-4-yl)-pyrimidin-2-yl]-(5-morpholin-4-yl-pyridin-2-yl)-amine, and
5-Fluoro-N4-isopropyl-N2-(5-piperazin-1-yl-pyridin-2-yl)-pyrimidine-2,4-diamine.

This invention provides a method of treating a disorder or condition selected from the group consisting of cell proliferative disorders, such as cancer, vascular smooth muscle proliferation associated with atherosclerosis, postsurgical vascular stenosis, restenosis, and endometriosis; infections, including viral infections such as DNA viruses like herpes and RNA viruses like HIV, and fungal infections; autoimmune diseases such as psoriasis, inflammation like rheumatoid arthritis, lupus, type 1 diabetes, diabetic nephropathy, multiple sclerosis, and glomerulonephritis, organ transplant rejection, including host versus graft disease, in a mammal, including human, comprising administering to said mammal an amount of a compound of formula I, or a pharmaceutically acceptable salt thereof, that is effective in treating such disorder or condition.

This invention further provides compounds of formula I that are useful for treating abnormal cell proliferation such a cancer. The invention provides a method of treating the abnormal cell proliferation disorders such as a cancer selected from the group consisting of cancers of the breast, ovary, cervix, prostate, testis, esophagus, stomach, skin, lung, bone, colon, pancreas, thyroid, biliary passages, buccal cavity and pharynx (oral), lip, tongue, mouth, pharynx, small intestine, colon-rectum, large intestine, rectum, brain and central nervous system, glioblastoma, neuroblastoma, keratoacanthoma, epidermoid carcinoma, large cell carcinoma, adenocarcinoma, adenocarcinoma, adenoma, adenocarcinoma, follicular carcinoma, undifferentiated carcinoma, papillary carcinoma, seminoma, melanoma, sarcoma, bladder carcinoma, liver carcinoma, kidney carcinoma, myeloid disorders, lymphoid disorders, Hodgkin's, hairy cells, and leukemia, comprising administering a therapeutically effective amound of a compound of formula I, or a pharmaceutically acceptable salt thereof, to a subject in need of such treatment.

A further embodiment of this invention is a method of treating subjects suffering from diseases caused by vascular smooth muscle cell proliferation. Compounds within the scope of the present invention effectively inhibit vascular smooth muscle cell proliferation and migration. The method comprises administering to a subject in need of treatment an amount of a compound of formula I, or a pharmaceutically acceptable salt thereof, sufficient to inhibit vascular smooth muscle proliferation, and/or migration.

This invention further provides a method of treating a subject suffering from gout comprising administering to said subject in need of treatment an amount of a compound of formula I, or a pharmaceutically acceptable salt thereof, sufficient to treat the condition.

This invention further provides a method of treating a subject suffering from kidney disease, such as polycystic kidney disease, comprising administering to said subject in need of treatment an amount of a compound of formula I, or a pharmaceutically acceptable salt thereof, sufficient to treat the condition.

Because of their inhibitory activity against Cdks and other kinases, the compounds of the present invention are also useful research tools for studying the mechanism of action of those kinases, both in vitro and in vivo.

The above-identified methods of treatment are preferably carried out by administering a therapeutically effective amount of a compound of Formula I to a subject in need of treatment. Compounds of the present invention are substituted 2-aminopyridines that are potent inhibitors of cyclin-dependent kinases 4 (Cdk4). The compounds are readily synthesized and can be administered by a variety of routes, including orally and parenterally, and have little or no toxicity. The compounds of the invention are members of the class of compounds of Formula I.

This invention provides a pharmaceutical composition comprising a therapeutically effective amount of a compound of the Formula I, or a pharmaceutically acceptable salt thereof, and a pharmaceutically acceptable carrier, diluent, or excipient therefor.

Many of the compounds of the present invention are selective inhibitors of cyclin dependent kinase Cdk4, which is to say that they inhibit Cdk4 more potently than they inhibit tyrosine kinases and other serine-threonine kinases including other cyclin-dependent kinases such as Cdk2. Despite their selectivity for Cdk4 inhibition, compounds of the invention may inhibit other kinases, albeit at higher concentrations than those at which they inhibit Cdk4. However, compounds of the present invention also may inhibit Cdk6 at similar concentrations to those necessary for inhibition of Cdk4 since Cdk6 is structurally similar to and performs similar functions to Cdk4.

Preferred embodiments of the present invention are compounds of the Formula I that inhibit Cdk4 at least about 10-fold more potently than they inhibit Cdk2.

A preferred embodiment of the present invention provides a method of inhibiting Cdk4 at a lower dose than is necessary to inhibit Cdk2 comprising administration of a preferred compound of Formula I in an amount that selectively inhibits Cdk4 over Cdk2.

The compounds of Formula I of this invention have useful pharmaceutical and medicinal properties. Many of the compounds of Formula I of this invention exhibit significant selective Cdk4 inhibitory activity and therefore are of value in the treatment of a wide variety of clinical conditions in which Cdk4 kinase is abnormally elevated, or activated or present in normal amounts and activities, but where inhibition of the Cdks is desirable to treat a cellular proliferative disorder. Such disorders include, but are not limited to those enumerated in the paragraphs below.

The compounds of the present invention are useful for treating cancer (for example, leukemia and cancer of the lung, breast, prostate, colon and skin such as melanoma) and other proliferative diseases including but not limited to psoriasis, HSV, HIV, restenosis, and atherosclerosis. To utilize a compound of the present invention to treat cancer, a patient in need of such treatment, such as one having cancer or another proliferative disease, is administered a therapeutically effective amount of a pharmaceutically acceptable composition comprising at least one compound of the present invention.

### DETAILED DESCRIPTION OF THE INVENTION

An illustration of the preparation of compounds of the present invention is shown in Schemes 1 to 7.

### Synthesis

The compounds of the invention may be prepared according to any of schemes 1-6. Typical solvents for Schemes 1, 4 and 5 are selected from the group consisting of benzene, chlorobenzene, nitrobenzene, toluene, pyridine, xylenes, acetonitrile, tetrahydrofuran, dimethyl sulfoxide, dimethylformamide, dimethylacetamide, N-methylpyrrolidinone, glyme, diglyme, ethoxyethanol, butanol, isopropanol and the like. Most preferably the solvent is selected from the group consisting of toluene, xylenes, acetonitrile, and dimethylsulfoxide.

In Scheme 1, the reaction of components III and IV generally requires their combination in a suitable solvent, preferably DMSO, toluene or pyridine and heating this mixture to a temperature of about 80°C to about 140°C. A subsequent deprotection step may be required depending on the nature of substituent R¹. To prepare compounds of Formula I according to Scheme 1, a compound A¹ bearing a leaving group (G), preferably selected from the group consisting of a halogen, aryl or alkyl sulfide, aryl or alkyl sulfoxide, aryl or alkyl sulfone, or aryl or alkyl sulfonate ester is combined with an aminopyridine derivative in a suitable solvent, preferably, DMSO, toluene, THF, or CH₃CN, and heated to reflux. It is generally preferable to use an excess of the aminopyridine. The desired products may be isolated by chromatography or by precipitation from the reaction mixture.

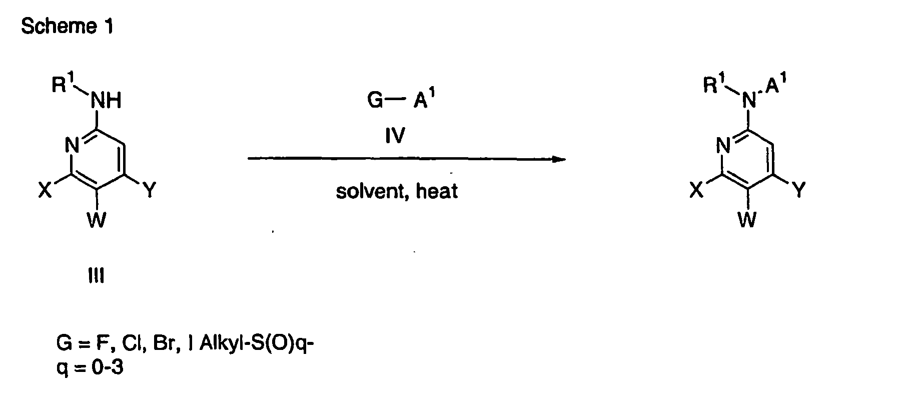

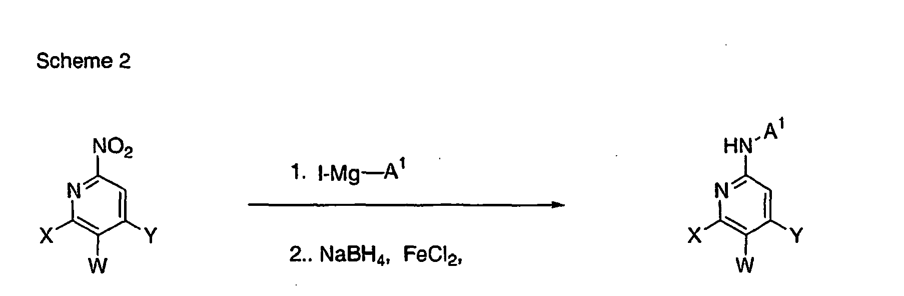

An alternative route, shown in Scheme 2, can be used to make compounds of formula I wherein R¹ is H. Conversion of an A¹-iodide to its corresponding Grignard reagent followed by coupling to a nitropyridine derivative according to the methods described by Knochel (J. Am. Chem. Soc. 2002, 124, 9390-9391) provides the desired products as shown. Thus, an aryl halide is treated with approximately one equivalent of a Grignard reagent in THF at a temperature between about -78 and 0°C for approximately 30 mins. To the resulting aryl Grignard is added one half to one third of an equivalent of a nitroarene compound at the same temperature and the reaction mixture is maintained at this temperature for a period of two to five hours. The reaction mixture is then treated with ethanol (excess), sodium borohydride (one equivalent with respect to the nitroarene) and ferric chloride (two equivalents with respect to the nitroarene). The resulting mixture is allowed to warm to room temperature until the reaction is complete (approximately two to five hours).

In some instances, it is possible to prepare compounds of Formula I by condensation of a pyridine guanidine with an appropriate partner to form a pyrimidine ring as shown in Scheme 3. In this instance, the guanidine and a corresponding ketone are combined with 3-5 equivalents of potassium carbonate in dry dimethyl formamamide and heated to about 150°C for approximately 1 hour.

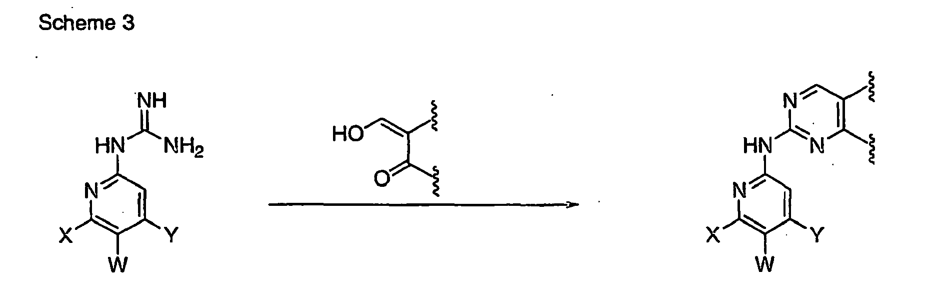

Specific examples of the process illustrated in Scheme 1 are shown below in Schemes 4 and 5 for the synthesis of compounds of Formula I wherein R¹ is H.

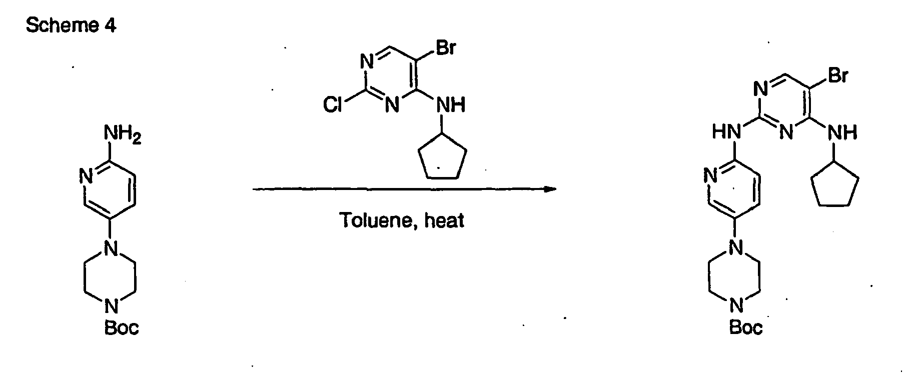

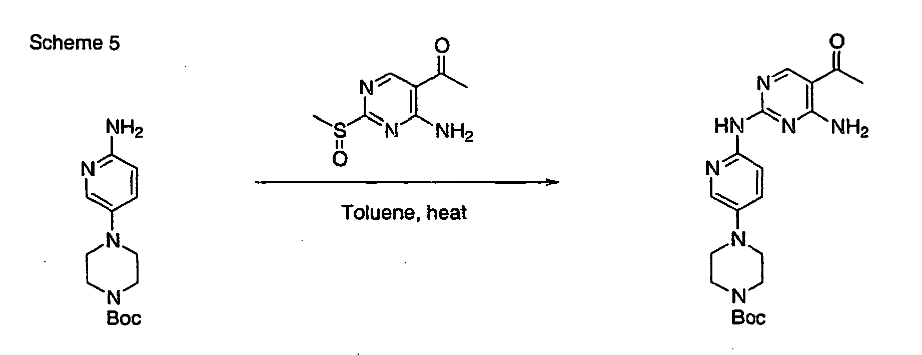

Scheme 6 illustrates an example of the synthesis of a compound of Formula I according to the general process depicted in Scheme 3.

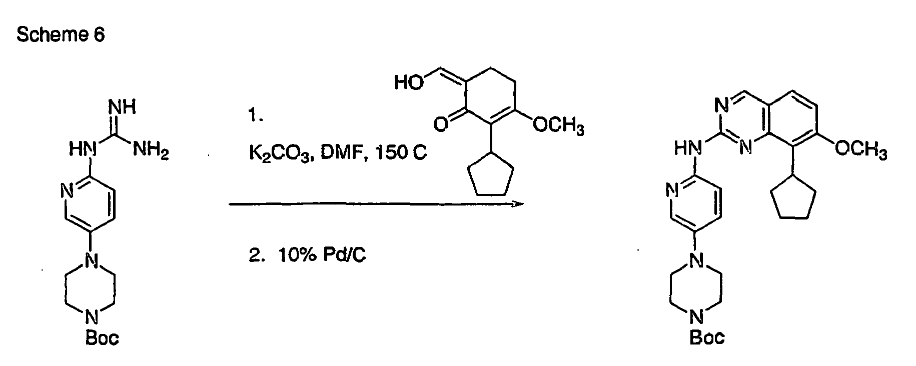

Another example of the synthesis of compounds of Formula I is demonstrated in Scheme 7 shown below. In this process, the formamide is deprotonated in the presence of a base, most preferably a strong organic base, and displaces the leaving group at room temperature.

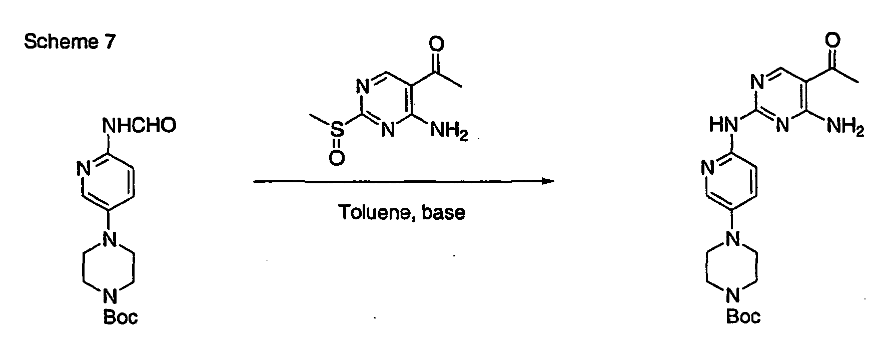

The compounds of the present invention can be formulated and administered in a wide variety of oral and parenteral dosage forms, including transdermal and rectal administration. It will be recognized to those skilled in the art that the following dosage forms may comprise as the active component, either a compound of Formula I or a corresponding pharmaceutically acceptable salt or solvate of a compound of Formula I.

This invention also comprises a pharmaceutical formulation comprising a therapeutically effective amount of a compound of Formula I together with a pharmaceutically acceptable carrier, diluent, or excipient therefor. For preparing pharmaceutical compositions with the compounds of the present invention, pharmaceutically acceptable carriers can be either a solid or liquid. Solid form preparations include powders, tablets, pills, capsules, cachets, suppositories, and dispensable granules. A solid carrier can be one or more substances which may also act as diluents, flavoring agents, binders, preservatives, tablet disintegrating agents, or an encapsulating material.

In powders, the carrier is a finely divided solid such as talc or starch which is in a mixture with the finely divided active component. In tablets, the active component is mixed with the carrier having the necessary binding properties in suitable proportions and compacted in the shape and size desired.

The formulations of this invention preferably contain from about 5% to about 70% or more of the active compound. Suitable carriers include magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. A preferred form for oral use are capsules, which include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient size molds, allowed to cool, and thereby to solidify.

Liquid form preparations include solutions, suspensions, and emulsions such as water or water/propylene glycol solutions. For parenteral injection, liquid preparations can be formulated in solution in aqueous polyethylene glycol solution, isotonic saline, 5% aqueous glucose, and the like. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water and mixing with a viscous material, such as natural or synthetic gums, resins, methylcellulose, sodium carboxymethylcellulose, or other well-known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like. Waxes, polymers, microparticles, and the like can be utilized to prepare sustained-release dosage forms. Also, osmotic pumps can be employed to deliver the active compound uniformly over a prolonged period.

The pharmaceutical preparations of the invention are preferably in unit dosage form. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The therapeutically effective dose of a compound of Formula I will vary from approximately 0.01 mg/kg to approximately 100 mg/kg of body weight per day. Typical adult doses will be approximately 0.1 mg to approximately 3000 mg per day. The quantity of active component in a unit dose preparation may be varied or adjusted from approximately 0.1 mg to approximately 500 mg, preferably about 0.6 mg to 100 mg according to the particular application and the potency of the active component. The composition can, if desired, also contain other compatible therapeutic agents. A subject in need of treatment with a compound of Formula I is administered a dosage of about 0.6 to about 500 mg per day, either singly or in multiple doses over a 24-hour period. Such treatment may be repeated at successive intervals for as long as necessary.

This invention provides a pharmaceutical composition for treating a disorder or condition selected from the group consisting of cell proliferative disorders, such as cancer, vascular smooth muscle proliferation associated with atherosclerosis, postsurgical vascular stenosis, restenosis, and endometriosis; infections, including viral infections such as DNA viruses like herpes and RNA viruses like HIV, and fungal infections; autoimmune diseases such as psoriasis, inflammation like rheumatoid arthritis, lupus, type 1 diabetes, diabetic nephropathy, multiple sclerosis, and glomerulonephritis, organ transplant rejection, including host versus graft disease.

The examples presented below are intended to illustrate particular embodiments of the invention, and are not intended to limit the scope of the specification or the claims in any way.

Those having skill in the art will recognize that the starting materials may be varied and additional steps employed to produce compounds encompassed by the present invention, as demonstrated by the following examples. The following examples are for illustrative purposes only and are not intended, nor should they be construed as limiting the invention in any manner. Those skilled in the art will appreciate that variations and modifications can be made without violating the spirit or scope of the invention.

### EXAMPLE 1

### Preparation of 4-Cyclopentylamino-2-(5-piperazin-1-yl-pyridin-2-ylamino)-pyrimidine-5-carboxylic acid ethyl ester hydrochloride salt

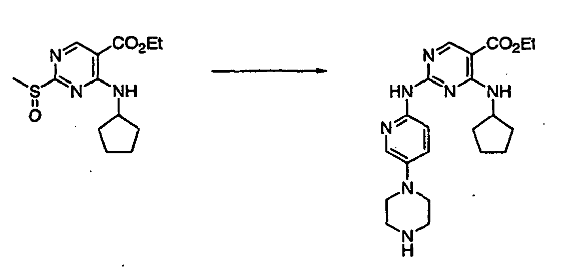

4-Cyclopentylamino-2-methanesulfinyl-pyrimidine-5-carboxylic acid ethyl ester (0.2 g, 0.67 mmol) and 4-(6-amino-pyridin-3-yl)-piperazine-1-carboxylic acid tert-butyl ester (0.75 g, 2.7 mmol) were combined in toluene (4 mL) and heated under nitrogen to 110 °C for 5 h. Succinic anhydride was added (0.27 g) and the heating bath was removed once the mixture had solidified. The mixture was allowed to cool, then diluted with dichloromethane and filtered to give 2-[5-(4-tert-butoxycarbonyl-piperazin-1-yl)-pyridin-2-ylamino]-4-cyclopentylamino-pyrimidine-5-carboxylic acid ethyl ester as a white solid. This solid was suspended in dichloromethane (5 mL) and treated with 2 M HCl in ether (5 mL) for 4 h. The solvents were evaporated to give 4-cyclopentylamino-2-(5-piperazin-1-yl-pyridin-2-ylamino)-pyrimidine-5-carboxylic acid ethyl ester hydrochloride salt as a yellow solid. mp 180 °C. MS (APCI) M⁺+1 Calc'd, 412.24; Found, 412.2.

### EXAMPLE 2

### Preparation of 4-[6-(5-Bromo-4-cyclopentylamino-pyrimidin-2-ylamino)-pyridin-3-yl]-piperazine-1-carboxylic acid tert-butyl ester

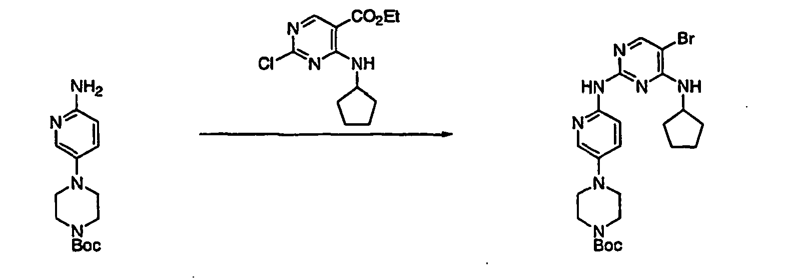

To 2-chloro-4-cyclopentylamino-pyrimidine-5-carboxylic acid ethyl ester (-2 mmol) in butanol (1.7 mL) was added 4-(6-amino-pyridin-3-yl)-piperazine-1-carboxylic acid tert-butyl ester (0.7g). This mixture was heated to 100 °C. After 2 hrs, xylenes (2 mL) was added and the temperature was raised to 140 °C. Heating was continued overnight. The mixture then was allowed to cool and diluted with ethyl acetate. The organic solution was washed twice with 1 M NaOH (aq), saturated ammonium chloride solution, then brine. After drying over magnesium sulfate, the solvents were evaporated and the residue was purified by chromatography on silica gel eluting with 35-45% ethyl acetate in hexanes to give 4-[6-(5-Bromo-4-cyclopentylamino-pyrimidin-2-ylamino)-pyridin-3-yl]-piperazine-1-carboxylic acid tert-butyl ester. MS (APCI) M⁺+1 Calc'd, 519.18; Found, 520.0.

### EXAMPLE 3

### Preparation of 4-(6-Formylamino-pyridin-3-yl)-piperazine-1-carboxylic acid tert-butyl ester

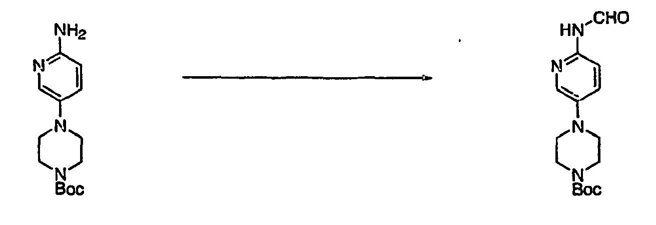

A solution of 4-(6-amino-pyridin-3-yl)-piperazine-1-carboxylic acid tert-butyl ester (278 mg, 1 mmol) and benzotriazole-1-carboxaldehyde (147 mg, 1 mmol) (Katritzky, A.R.; Chang, H.X.; Yang, B. Synthesis. 1995, 503-505) in THF (3 mL) was heated under reflux overnight under nitrogen. The solution was concentrated and the residue was dissolved in dichloromethane. The solution was washed with 1N NaOH (2X) and then water, dried (MgSO₄), and concentrated. The solid was purified by chromatography over silica gel to give 0.279 g (91.2%) of the product as a white solid, mp 178-180 °C.
MS (APCI): Calc for C₁₅H₂₂N₄O₃ (M+1), 307.2; Found, 307.2. NMR (400 MHz, CDCl₃ + D₂O) (mixture of rotamers) δ 1.46 (s, 9H), 3.08 (m, 4H), 3.57 (m, 4H), 6.80 (d, J=8.8 Hz, 0.3H), 7.24 (dd, obscured by CHCl₃, 0.3H), 7.27 (dd, J=9.2 Hz, 3.1 Hz; 0.7H), 7.93 (d, J=2.7 Hz, 0.7 H), 7.96 (d, J=2.7 Hz, 0.3H), 8.08 (dd, J=9.0 Hz, 0.5 Hz; 0.7H), 8.39 (s, 0.7H), 9.09 (s, 0.3H).

### EXAMPLE 4

### Preparation of 4-[6-(5-Acetyl-4-amino-pyrimidin-2-ylamino)-pyridin-3-yl]-piperazine-1-carboxylic acid tert-buyl ester

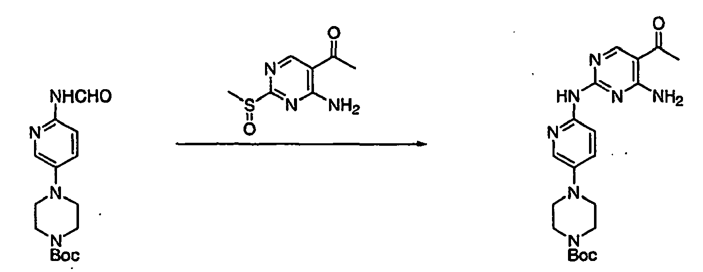

To a solution of 4-(6-formylamino-pyridin-3-yl)-piperazine-1-carboxylic acid tert-butyl ester (127 mg, 0.41 mmol) in THF (3 mL) at 0 °C, was added a solution of lithium bis(trimethylsilyl)amide in THF (1.0 M, 0.41 mL, 0.41 mmol). To the cold solution was added 1-(4-amino-2-methanesulfinyl-pyrimidin-5-yl)-ethanone (75 mg, 0.38 mmol) (WO 01/055147). The suspension was stirred at room temperature overnight, then treated with MeOH (5 mL) and CH₂Cl₂ (50 mL) and stirred for another 30 minutes. The suspension was poured into water, and the layers were separated. The organic layer was dried (MgSO₄) and concentrated to give a solid. Trituration with CH₃CN gave 26 mg (17%) of the product as a brown-yellow solid. MS (APCI): Calc for C₂₀H₂₇N₇O₃ (M+1), 414.2; Found, 414.2. NMR (400 MHz, DMSO-*d*₆ + D₂O) δ 1.36 (s, 9H), 2.39 (s, 3H), 3.02 (m, 4H), 3.42 (m, 4H), 7.36 (dd, J=9.0 Hz, 2.9 Hz, 1H), 7.96 (d, J=2.7 Hz, 1H), 8.16 (d, J=9.0 Hz, 1H), 8.69 (s, 1H).

### EXAMPLE 5

### Preparation of 4-[6-Cyclopentylamino-5-nitro-pyrimidin-2-ylamino)-pyridin-3-yl]-piperazine-1-carboxylic acid tert-butyl ester

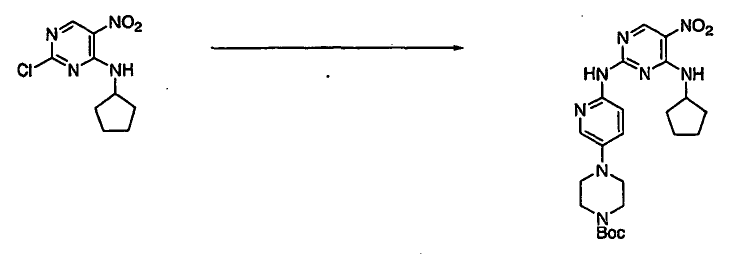

A solution of (2-chloro-5-nitro-pyrimidin-4-yl)-cyclopentyl-amine (0.91 g, 3.75 mmol) (WO 01/019825) and 4-(6-amino-pyridin-3-yl)-piperazine-1-carboxylic acid tert-butyl ester (2.30 g, 8.25 mmol) in CH₃CN (20 mL) was stirred at room temperature overnight. The solution was poured into a cold, saturated aqueous solution of NaHCO₃, and the suspension was extracted with CH₂Cl₂. The organic layer was dried (MgSO₄) and concentrated. To the residue was added CH₃CN (100 mL) and the suspension was heated under reflux for 2 hr. The suspension was cooled and filtered to give a solid. Chromatography over silica gel gave 0.805 g (44.3%) of the product as a yellow solid. MS (APCI): Calc for C₂₃H₃₂N₈O₄ (M+1), 485.3; Found, 485.1. NMR (400 MHz, CDCl₃) δ 1.47 (s, 9H), 1.6-1.8 (m, 6H), 2.1 (m, 2H), 3.13 (t, J=5.0 Hz, 4H), 3.60 (t, J=5.0 Hz, 4H), 4.53 (m, 1H), 7.34 (dd, J=9.0 Hz, 2.9 Hz, 1H), 8.07 (s, 1H), 8.34 (d, J=9.0 Hz, 1H), 8.51 (d, 1H), 9.16 (s, 1H), 9.39 (br s, 1H).

### EXAMPLE 6

### Preparation of 4-[6-(5-Amino-4-cyclopentylamino-pyrimidin-2-ylamino)-pyridin-3-yl]-piperazine-1-carboxylic acid tert-butyl ester

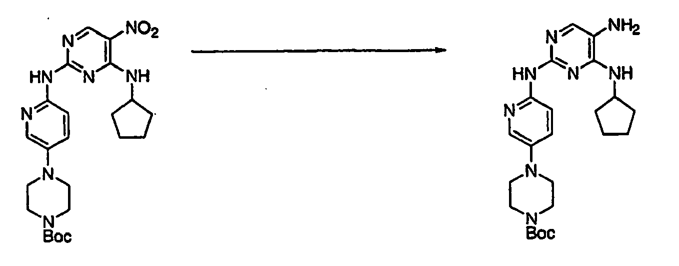

A suspension of 4-[6-cyclopentylamino-5-nitro-pyrimidin-2-ylamino)-pyridin-3-yl]-piperazine-1-carboxylic acid tert-butyl ester (90 mg, 0.2 mmol) and RaNi (100 mg) in THF (100 mL) was shaken at room temperature for 19 hr under a hydrogen atmosphere with an initial pressure of 50 psi. The suspension was filtered and the filtrate concentrated to give 0.08 g (100%) of the product as a grey solid. MS (APCI): Calc for C₂₃H₃₄N₈O₂ (M+1), 455.3; Found, 455.2. NMR (400 MHz, CDCl₃) δ 1.42 (s, 9H), 1.3-1.7 (m, 6H), 2.0 (m, 2H), 3.0 (s, 4H), 3.5 (m, 4H), 4.3 (m, 1H), 7.23 (dd, J=9.2 Hz, 2.8 Hz, 1H), 7.48 (s, 1H), 7.86 (d, J=2.4 Hz, 1H), 8.09 (br m, 1H).

### EXAMPLE 7

### Preparation of 4-[6-(8-Cyclonentyl-6-methyl-7-oxo-7, 8-dihydro-pteridin-2-ylamino)-pyridin-3-yl]-piperazine-1-carboxylic acid tert-butyl ester

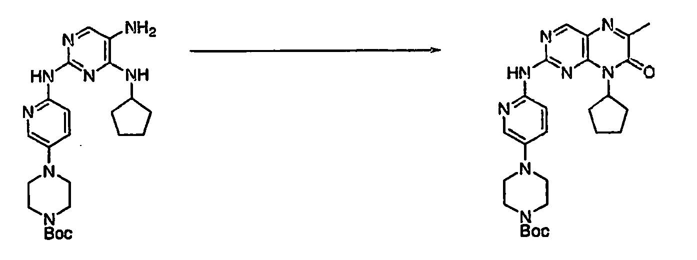

To 4-[6-(5-amino-4-cyclopentylamino-pyrimidin-2-ylamino)-pyridin-3-yl]-piperazine-1-carboxylic acid tert-butyl ester (291 mg, 0.64 mmol) and 2-oxo-propionic acid methyl ester (96 µL, 0.96 mmol) in EtOH (8 mL) was added acetic acid (4 drops), and the solution was heated under reflux for 1 hr. The resulting solid was collected by filtration and purified by chromatography over silica gel to give the product (185 mg, 56.7%) as a yellow solid. MS (APCI) Calc for C₂₆H₃₄N₈O₃ (M+1), 507.3; Found, 507.1 NMR (400 MHz, CDCl₃) δ 1.48 (s, 9H), 1.7 (m, 2H), 1.9 (m, 2H), 2.1 (m, 2H), 2.3 (m, 2H), 3.12 (t, J=4.9 Hz, 4H), 3.61 (t, J=5.0 Hz, 4H), 5.76 (m, 1H), 7.37 (dd, J=9.0 Hz, 2.9 Hz, 1H), 8.05 (d, J=2.7 Hz, 1H), 8.24 (d, J=9.0 Hz, 1H), 8.66 (br s, 1H), 8.80 (s, 1H).

### EXAMPLE 8

### Preparation of 8-Cyclonentyl-6-methyl-2-(5-piperazin-1-yl-pyridin-2-ylamino) - 8H-pteridin-7-one

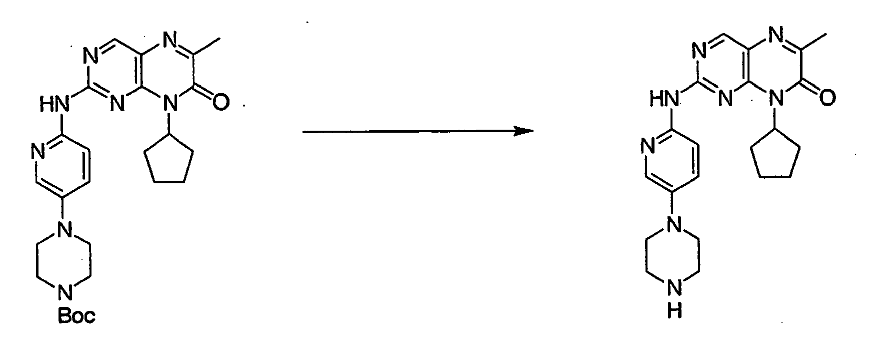

To a solution of 4-[6-(8-cyclopentyl-6-methyl-7-oxo-7, 8-dihydro-pteridin-2-ylamino)-pyridin-3-yl]-piperazine-1-carboxylic acid tert-butyl ester (230 mg, 0.452 mmol) in CH₂Cl₂ (20 mL) was added trifluoroacetic acid (4 mL), and the solution was stoppered and stirred at room temperature overnight. The reaction mixture was concentrated, and the residue was co-evaporated with CH₂Cl₂ two times. The residue was then dissolved in CH₂Cl₂, and the solution was washed with dilute ammonium hydroxide, dried (MgSO₄), and concentrated to give the product as a yellow solid (172 mg, 93.5%), mp 221-223 °C. MS (APCI) Calc for C₂₁H₂₆N₈O₁ (M+1), 407.2; Found, 407.1. NMR (400 MHz, DMSO-*d₆*) δ 1.6 (m, 2H), 1.8 (m, 2H), 1.9 (m, 2H), 2.2 (m, 2H), 2.31 (s, 3H), 2.81 (m, 4H), 3.01 (m, 4H), 5.65 (p, J=8.7 Hz, 1H), 7.40 (dd, J=9.2 Hz, 3.1 Hz, 1H), 7.83 (d, J=9.0 Hz, 1H), 7.98 (d, J=2.7 Hz, 1H), 8.71 (s, 1H), 9.98 (s, 1H).

### EXAMPLE 9

### Preparation of 4-[6-(8-Cyclopentyl-6-ethyl-7-oxo-7, 8-dihydro-pteridin-2-ylamino)-pyridin-3-yl]-piperazine-1-carboxylic acid tert-butyl ester

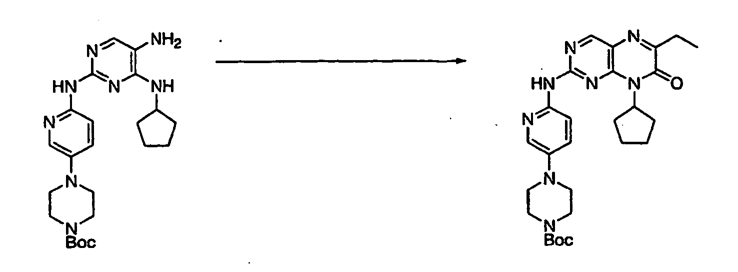

To 4-[6-(5-amino-4-cyclopentylamino-pyrimidin-2-ylamino)-pyridin-3-yl]-piperazine-1-carboxylic acid tert-butyl ester (430 mg, 0.846 mmol) and 2-oxo-butyric acid methyl ester (147 mg, 1.27 mmol) in EtOH (5 mL) was added acetic acid (4 drops), and the solution was heated under reflux for 1 hr. The cooled solution was poured into a saturated aqueous solution of sodium bicarbonate, and the suspension was extracted with CH₂Cl₂. The extracts were dried (MgSO₄) and concentrated. The residue was purified by chromatography over silica gel to give the product (201 mg, 45.7%) as a yellow solid. MS (APCI) Calc for C₂₇H₃₆N₈O₃ (M+1), 521.3; Found, 521.2. NMR (400 MHz, CDCl₃) δ 1.29 (t, J=7.3 Hz, 3H), 1.48 (s, 9H), 1.7 (m, 2H), 1.9 (m, 2H), 2.1 (m, 2H), 2.3 (m, 2H), 2.88 (q, J=7.4 Hz, 2H), 3.12 (t, J=4.9 Hz, 4H), 3.61 (t, J=5.1 Hz, 4H), 5.76 (p, J=8.9 Hz, 1H), 7.40 (dd, J=9.2 Hz, 2.8 Hz, 1H), 7.99 (d, J=2.9 Hz, 1H), 8.29 (d, J=9.3 Hz, 1H), 8.53 (br s, 1H), 8.85 (s, 1H).

### EXAMPLE 10

### Preparation of 8-Cyclopentyl-6-ethyl-2-(5-piperazin-l-yl-pyridin-2-ylamino)-8H-pteridin-7-one

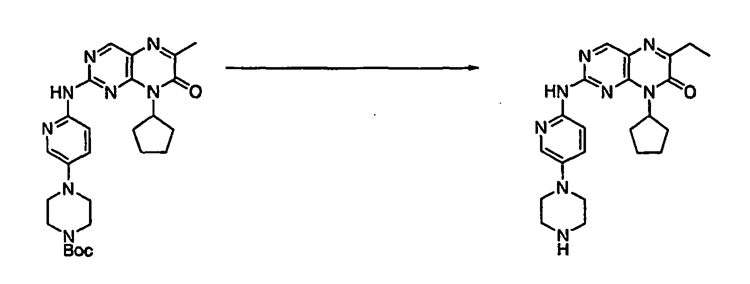

To a solution of 4-[6-(8-cyclopentyl-6-ethyl-7-oxo-7, 8-dihydro-pteridin-2-ylamino)-pyridin-3-yl]-piperazine-1-carboxylic acid tert-butyl ester (200 mg, 0.40 mmol) in CH₂Cl₂ (20 mL) was added trifluoroacetic acid (4 mL). The flask was stoppered and the reaction mixture was stirred at room temperature overnight. The reaction mixture was concentrated, and the residue was co-evaporated with CH₂Cl₂ two times. The residue was then dissolved in CH₂Cl₂, and the solution was washed with dilute ammonium hydroxide, dried (MgSO₄), and concentrated to give the product as a yellow solid (134 mg, 83.8%), mp 203-208 °C (dec). MS (APCI) Calc for C₂₂H₂₈N₈O₁ (M+1), 421.2; Found, 421.1. NMR (400 MHz, CDCl₃) δ 1.28 (t, J=7.3 Hz, 3H), 1.7 (m, 2H), 1.9 (m, 2H), 2.1 (m, 2H), 2.3 (m, 2H), 2.87 (q, J=7.4 Hz, 2H), 3.09 (m, 4H), 3.15 (m, 4H), 5.75 (p, J=8.9 Hz, 1H), 7.32 (dd, J=9.2 Hz, 3.1 Hz, 1H), 8.05 (d, J=2.9 Hz, 1H), 8.17 (d, J=9.0 Hz, 1H), 8.2 (m, 1H), 8.80 (s, 1H).

### EXAMPLE 11

### Preparation of 4-[6-(3-Cyclopentyl-7-methoxy-5,6-dihydro-quinazolin-2-ylamino)-pyridin-3-yl]-piperazine-1-carboxylic acid tert-butyl ester

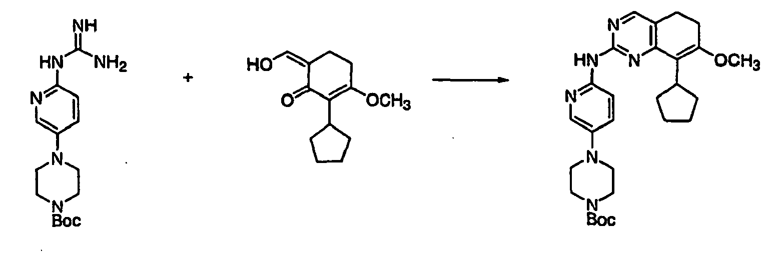

2-Cyclopentyl-6-hydroxymethylene-3-methoxy-cyclohex-2-enone (309 mg, 1.39 mmol) and 4-(6-guanidino-pyridin-3-yl)-piperazine-1-carboxylic acid tert-butyl ester (432 mg, 1.35 mmol) were combined with K₂CO₃ (562 mg, 4.07 mmol) in anhydrous DMF (7.5 mL) under nitrogen and heated to 150°C for 45 minutes. After allowing to cool, the solvent was removed in vacuo. The residue was partitioned between ethyl acetate (200 mL) and water (200 mL). The organic phase was washed with water then brine and dried over anhydrous sodium sulfate. Following removal of the drying agent and evaporation of the solvent, the residue was chromatographed in silica gel eluting with 50-100% ethyl acetate in hexanes to give 4-[6-(8-cyclopentyl-7-methoxy-5,6-dihydro-quinazolin-2-ylamino)-pyridin-3-yl]-piperazine-1-arboxylic acid tert-butyl ester as a brown solid (127 mg, 20%). MS (ESI) M⁺+1 calc'd, 507.3; Found, 507. ¹H NMR (300 MHz, CDCl₃) δ 8.30 (d, J = 9.1 Hz, 1H), 8.00 (s, 1H), 7.96 (d, J = 2.8 Hz, 1H), 7.54 (br s, 1H), 7.29 (dd, J = 9.1 Hz, 1H), 3.73 (s, 3H), 3.57-3.63 (m, 4H), 3.42-3.57 (m, 1H), 3.01-3.10 (m, 4H), 2.73 (t, J = 7.9 Hz, 2H), 2.53 (t, J = 7.8 Hz, 2H), 1.58-2.03 (m, 8H), 1.49 (s, 9H).

### EXAMPLE 12

### Preparation of 4-[6-(8-Cyclopentyl-7-methoxy-quinazolin-2-ylamino)-pyridin-3-yl]-piperazine-1-carboxylic acid tert-butyl ester

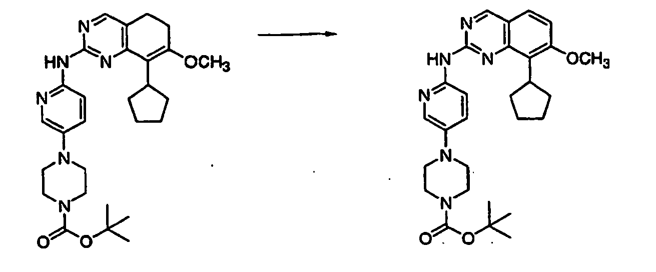

4-[6-(8-Cyclopentyl-7-methoxy-5,6-dihydro-quinazolin-2-ylamino)-pyridin-3-yl]-piperazine-1-carboxylic acid tert-butyl ester (89 mg, 0.18 mmol) and Pd/C (97 mg) in nitrobenzene (2 mL) was heated to 150 °C for 18 h, then allowed to cool The mixture was purified by flash column chromatography on silica gel eluting with 10%ethyl acetate in hexanes to remove the nitrobenzene, then eluting with 60-100% ethyl acetate in hexanes to give 4-[6-(8-cyclopentyl-7-methoxy-quinazolin-2-ylamino)-pyridin-3-yl]-piperazine-1-carboxylic acid tert-butyl ester as a light brown solid (51 mg, 24%). MS (ESI) Calc'd, 505.28; Found 505. ¹H NMR (300 MHz, CDCl₃) δ 8.98 (s, 1H), 8.60 (d, J = 9.1 Hz, 1H), 8.03 (d J = 2.8 Hz, 1H), 7.61 (d, J = 8.9 Hz, 1H), 7.37 (dd, J = 9.1, 2.9 Hz, 1H), 7.10 (d, J = 8.9 Hz, 1H), 4.24-4.39 (m, 1H), 3.97 (s, 3H), 3.58-3.67 (m, 4H), 3.07-3.15 (m, 4H), 2.12-2.29 (m, 2H), 1.70-2.03 (m, 6H), 1.50 (s, 9H).

### EXAMPLE 13

### Preparation of (8-Cyclopentyl-7-methoxy-quinazolin-2-yl)-(5-piperazin-1-yl-pyridin-2-yl)-amine

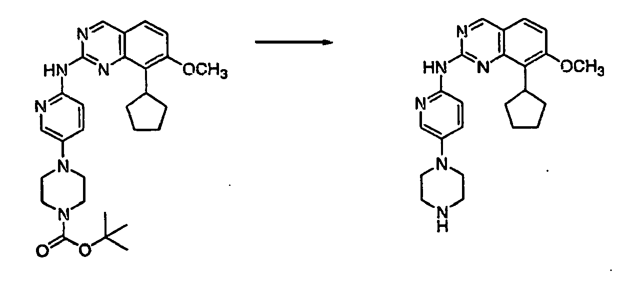

4-[6-(8-cyclopentyl-7-methoxy-quinazolin-2-ylamino)-pyridin-3-yl]-piperazine-1-carboxylic acid tert-butyl ester (25 mg, 50 µmol) was suspended in dichloromethane (2 mL) at 0 °C and trifluoroacetic acid (2 mL) was added with stirring under nitrogen. After 1 h at 0 °C, the mixture was allowed to warm to room temperature for 1.5 h. The solvents were removed in vacuo. The residue was dissolved in ethyl acetate, then washed with 1M NaOH, water, then brine. The ethyl acetate layer was dried over anhydrous sodium sulfate, filtered, then evaporated. The residue was purified by chromatography on silica gel eluting with 1:1:98 MeOH:Et₃N:CHCl₃ then with 5:1:94 MeOH:Et₃N:CHCl₃ to give (8-cyclopentyl-7-methoxy-quinazolin-2-yl)-(5-piperazin-1-yl-pyridin-2-yl)-amine as a dark yellow solid. mp > 200 °C. MS (ESI) Calc'd, 405.28; Found, 405. ¹H NMR (300 MHz, CDCl₃) δ 9.52 (s, 1H), 9.13 (s, 1H), 8.33 (d, J = 9 Hz, 1H), 8.03 (d J = 2.8 Hz, 1H), 7.80 (d, J = 8.9 Hz, 1H), 7.45 (dd, J = 9, 2.9 Hz, 1H), 7.26 (d, J = 8.9 Hz, 1H), 4.18-4.31 (m, 1H), 3.95 (s, 3H), 3.10-3.18 (m, 4H), 2.92-3.02 (m, 4H), 2.07-2.23 (m, 2H), 1.82-1.99 (m, 2H), 1.63-1.82 (m, 4H).

### EXAMPLE 14

The preparation of triazine derivatives for Formula 1 of the present invention is depicted in Scheme 8 and Examples 14 A-H below. Generally, triazine derivatives useful in the present invention are prepared from cyanuric chloride by preparing the triazine derivative, partitioning the triazine derivative into an organic solvent and further purifying and concentrating the triazine derivative before attaching it to the 2-aminopyridine core of the present invention. The triazine compounds of the present invention may be attached to the amino pyridine core by the methods described below, any of the methods of Schemes 1-7 above as well as other methods known in the art.

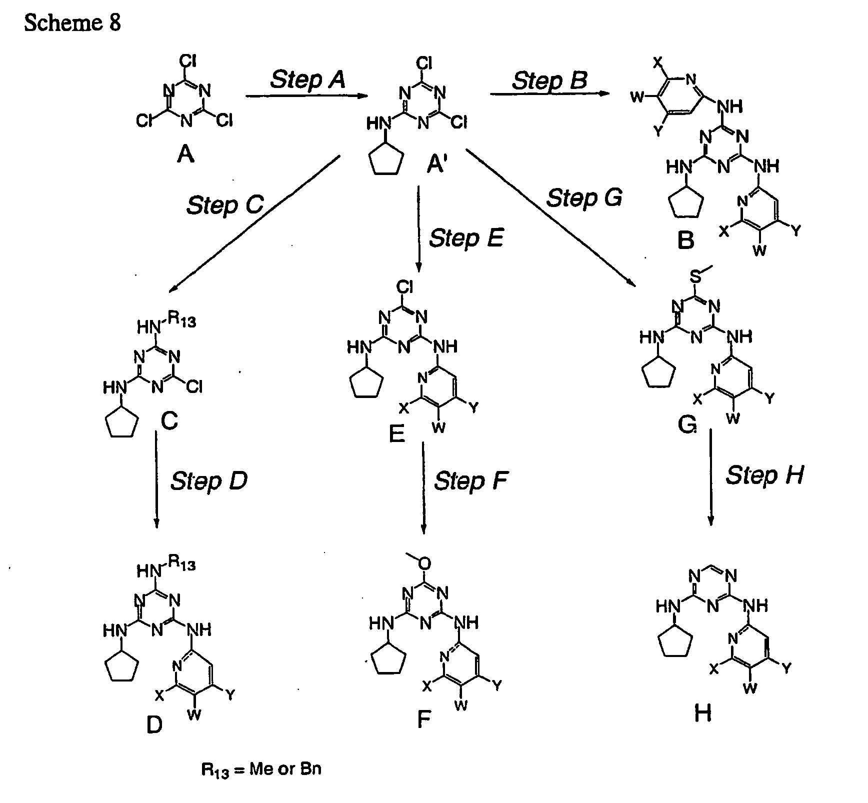

### EXAMPLE 14A

### Preparation of cyclopentyl-(4,6-dichloro-[1,3,5]triazin-2-yl)-amine (A') by Reaction Step A

To a stirring solution of cyanuric chloride (1.96g, 10.6 mmol) in THF at -12 °C was added Hunig's base (2.8 mL, 16 mmol) followed by cyclopentylamine (0.9g, 10.6 mmol) over 10 minutes. The reaction was allowed to stir at -10°C for 1 hour. The reaction mixture was diluted with water and partitioned with ethyl acetate. The organic phase was washed twice with water and once with brine. The organic layer was collected, dried over Na₂SO₄, filtered and concentrated under reduced pressure to afford an yellow oil, which was purified via silica column chromatography in 9:1 hexanes/ethyl acetate and yielded cyclopentyl-(4,6-dichloro-[1,3,5]triazin-2-yl)-amine as a dark yellow oil (2.34g, 96%).

### EXAMPLE 14B

### Preparation of N-Cyclopentyl-N',N"-bis-(6-piperazin-1-yl-pyridin-3-yl)-1,3,5-triazine-2,4,6-triamine (Compound B of Scheme 8)

To a stirring solution of cyclopentyl-(4,6-dichloro-[1,3,5]triazin-2-yl)-amine (0.60g, 2.57 mmol) prepared as in Example 14A above in 5 mL of acetonitrile was added 4-(5-amino-pyridin-2-yl piperazine-1-carboxylic acid tert-butyl ester (0.72g, 2.6 mmol) and triethylamine (0.36 mL, 2.6 mmol). The mixture was stirred at ambient temperature. After 20 minutes, solids formed. The solids were filtered off and washed several times with acetonitrile. The solids were dried in a vacuum oven for 1.5 hours at 60°C. Purification was accomplished via silica column chromotography in 7:1 dichloromethane/acetone. The desired fractions were collected and concentrated in vacuo to afford N-Cyclopentyl-N',N"-bis-(6-piperazin-1-yl-pyridin-3-yl)-1,3,5-triazine-2,4,6-triamine (Compound B) as a brown foam/solid (0.234g, 19%). Ms 517.3; Mp >290.

### EXAMPLE 14C

### Preparation of 6-Chloro-N-cyclopentyl-N'-methyl-[1,35]triazine-2,4-diamine Compound C) (PF-00089971)

To a stirring solution of cyclopentyl-(4,6-dichloro-[1,3,5]triazin-2-yl)-amine (2.76g, 11.84 mmol) Compound A' in THF (40 mL) was added methylamine hydrochloride (0.80g, 11.84 mmol) and Hunig's base (4.1 mL, 23.7 mmol). After stirring for 2.5 hours at ambient temperature, the reaction was then heated to reflux for 3.5 hours. The reaction was allowed to cool, then diluted with ethyl acetate and partitioned with water. Organics were washed twice with water and once with brine. Organics were collected, dried over Na₂SO₄, filtered and concentrated under reduced pressure to yield a yellow solid. The yellow solids were titrated with hexanes and dried under reduced pressure to yield 6-chloro-N-cyclopentyl-N'-methyl-[1,3,5]triazine-2,4-diamine (Compound C) as a white solid. MS 228.1; 1H NMR (400 MHz, METHANOL-D4) δ ppm 1.5 (m, 2 H) 1.6 (m, 2 H) 1.7 (m, 2 H) 2.0 (m, 2 H) 2.8 (m, 3 H).

### EXAMPLE 14D

### Preparation of N-Cyclopentyl-N'-methyl-N"-(4-morpholin-4-yl-phenyl)-[1,3,5]triazine-2,4,6-triamine (Compound D) (PF-00092134)

To a Radley's reaction tube was added 6-chloro-N-cyclopentyl-N'-methyl-[1,3,5]triazine-2,4-diamine (Compound D) (0.153g, 0.672 mmol) and 4-morpholin-4-yl-phenylamine (0. 120g, 0.672 mmol) and acetonitrile and the system was capped and heated to 92°C. After refluxing for 18.5 hours, the reaction mixture was concentrated and purified via silica column chromatography in 3:1 DCM/acetone to 2:1 DCM/acetone. The fractions were collected and concentrated under reduced pressure to afford N-cyclopentyl-N'-methyl-N"-(4-morpholin-4-yl-phenyl)-[1,3,5]triazine-2,4,6-triamine (Compound D). MS 370.2; Mp 218-219.

### EXAMPLE 14E

### Preparation of 6-Chloro-N-cyclopentyl-N'-(6-morpholin-4-yl-pyridin-3-yl)-[1,3,5]triazine-2,4-diamine (Compound E) (PF-00074391)

To a stirring solution of cyclopentyl-(4,6-dichloro-[1,3,5]triazin-2-yl)-amine (Compound A') (0.105g, 0.451mmol) in chloroform (5 mL) at -10 °C was added a solution of 6-morpholin-4-yl-pyridin-3-ylamine (0.081g, 0.451 mmol) in chloroform (3 mL) over 20 minutes. The reaction was allowed to stir at -10 °C for 1 hour. The reaction was allowed to warm to ambient temperature and stirred overnight. After stirring for 20 hours, the reaction mixture was diluted with dichloromethane and partitioned with water. The organic phase was washed twice with water and then collected, dried over Na₂SO₄, filtered and concentrated under reduced pressure to yield 6-chloro-N-cyclopentyl-N'-(6-morpholin-4-yl-pyridin-3-yl)-[1,3,5]triazine-2,4-diamine (Compound E) as a purple foam (0.103g, 33%). MS 376.1; MP 218-219.

### EXAMPLE 14F

### Preparation of N-cyclopentyl-6-methoxy-N'-(6-piperazin-1-yl-pyridin-3-yl)-[1,3,5]triazine-2,4-diamine (Compound F) (PF-000775944)

To a stirring solution of 4-[5-(4-chloro-6-cycopentylamino-[1,3,5]triazin-2-ylamino)-pyridin-2-yl]-piperazine-1-carboxylic acid tert-butyl ester (0.053g, 0.112 mmol) in methanol was bubbled in HCl gas over one minute. The mixture was stirred at ambient temperature for 1 hour. The reaction mixture was concentrated under reduced pressure. Diethyl ether was added to the resultant yellow oil and the mixture allowed to stand overnight. The resultant white solids were titrated with diethyl ether, then collected and dried on the high vacuum pump. The afforded white solids were characterized as N-yclopentyl-6-methoxy-N'-(6-piperazin-1-yl-pyridin-3yl)-[1,3,5]triazine-2,4-diamine (Compound F) hydrochloride salt (0.52g, 84%). MS 371.2; Mp >290.

### EXAMPLE 14G

### Preparation of N-Cyclopentyl-6-methylsulfanyl-N'-(5-piperazin-1-yl-pyridin-2-yl)-[1,3,5]triazine-2,4-diamine (Compound G) (PF-00192021)

Part 1: To a stirring solution of cyclopentyl-(4,6-dichloro-[1,3,5]triazin-2-yl)-amine Compound A' (1.38g, 5.92 mmol) in anhydrous DMSO (20 mL) was added sodium thiomethoxide (0.87g, 12 mmol). After stirring at ambient temperature for 22 hours, the reaction mixture was poured into water and partitioned with ethyl acetate. The organic layer was washed five times with water. The organic layer was collected, dried over Na₂SO₄, filtered and concentrated under reduced pressure. The afforded orange oil was coevaporated several times with hexanes to afford (4,6-bis-methylsulfanyl-[1,3,5]triazin-2-yl)-cyclopentyl-amine (Compound G) as an orange oil (1.50g, 99%). MS 487.2. 1H NMR (400 MHz, METHANOL-D4) δ ppm 1.5 (s, 9 H) 1.5 (m, 2 H) 1.6 (m, 2 H) 1.7 (m, 2 H) 2.0 (m, 2 H) 2.5 (m, 3 H) 3.1 (m, 4 H) 3.6 (m, 4 H) 4.3 (m, 1 H) 7.4 (m, 1 H) 7.9 (m, 1 H) 8.2 (m, 1 H).

Part 2: To a stirring solution of (4,6-bis-methylsulfanyl-[1,3,5]triazin-2-yl)-cyclopentyl-amine ( Compound G) (1.49g, 5.81 mmol) in dichloromethane (90 mL) was added 2-benzenesulfonyl-3-phenyl-oxaziridine (1.52g, 5.81 mmol) and allowed to stir at ambient temperature. After stirring for 21 hours the reaction mixture was concentrated and the afforded yellow oil was purified via silica column chromatography in 100% dichloromethane to 9:1 dichloromethane/acetone. The desired fractions were collected to afford cyclopentyl-(4-methanesulfinyl-6-methylsulfanyl-[1,3,5]triazin-2-yl)-amine (1.23g, 78%). MS 387.2, mp >290.

Part 3: To a stirring suspension of 4-(6-formylamino-pyridin-3-yl)-piperazine-1-carboxylic acid tert-butyl ester (0.20g, 0.64 mmol) in toluene (10 mL) at 0 °C was added LiHMDS (0.64 mL, 0.64 mmol) and allowed to warm to ambient temperature. After stirring for 45 minutes, cyclopentyl-(4-methanesulfinyl-6-methylsulfanyl-[1,3,5]triazin-2-yl)-amine (0.158g, 0.58 mmol) was added and the mixture was stirred at ambient temperature for 3 hours. The reaction was then heated to 70°C for 1 hour, then quenched with methanol (10mL) and allowed to stir at ambient temperature for 20 minutes. The reaction mixture was concentrated under reduced pressure and diluted with dichloromethane and partitioned with water. Organics were washed twice with water, then collected, dried over Na₂SO₄, filtered and concentrated under reduced pressure to yield a yellow oil. Purification was performed via silica column chromatography in 7:1 dichloromethane/acetone. The desired fractions were collected which yielded a light yellow foam. The yellow foam was dissolved in ethyl acetate and hexanes were added to afford 4-[6-(4-cyclopentylamino-6-methylsulfanyl-[1,3,5]triazin-2-ylamino)-pyridin-3-yl]-piperazine-1-carboxylic acid tert-butyl ester as a white solid (0. 140g, 50%).

Part 4: To a stirring suspension of 4-[6-(4-cyclopentylamino-6-methylsulfanyl-[1,3,5]triazin-2-ylamino)-pyridin-3-yl]-piperazine-1-carboxylic acid tert-butyl ester (0.040g, 0.082 mmol) in methanol (5 mL) was added approximately 2 mL of a HCl/methanol and allowed to stir at ambient temperature. After 4 hours the reaction mixture was concentrated under reduced pressure to yield a yellow oil. Diethyl ether was added which yielded yellow solids. The solids were washed with ether and then dried in a vacuum oven at 60°C to afford N-cyclopentyl-6-methylsulfanyl-N'-(5-piperazin-1-yl-pyridin-2-yl)-[1,3,5]triazine-2,4-diamine hydrochloride salt (0.024g, 76%).

### EXAMPLE 14H

### Preparation of 4-[6-(4-Cyclopentylamino-[1,3,5]triazin-2-ylamino)-pyridin-3-yl]-piperazine-1-carboxylic acid tert-butyl ester

Part 1: A 100 mL RBF fitted with a reflux condensor was charged with 4-[6-(4-cyclopentylamino-6-methylsulfanyl-[1,3,5]triazin-2-ylamino)-pyridin-3-yl]-piperazine-1-carboxylic acid tert-butyl ester (0.09g, 0.191 mmol) and the system was flushed with nitrogen for 20 minutes. The system was charged with THF (4 mL) and ethanol (4 mL) and heated to 85C. Raney-Nickel was added via a plastic spoon and continued to heat at 85C. After two hours another scoop of Raney-Nickel was added and continued to reflux. After 4 hours the reaction was allowed to cool and the mixture was filtered over a bed of Celite. The filtrate was collected and dried under reduced pressure, which afforded 4-[6-(4-cyclopentylamino-[1,3,5]triazin-2-ylamino)-pyridin-3-yl]-piperazine-1-carboxylic acid tert-butyl ester as a white solid (0.040g, 48%).. MS 441.3. 1H NMR (400 MHz, METHANOL-D4) δ ppm 1.5 (s, 9 H) 1.5-1.9 (m, 6 H) 2.0 (m, 2 H) 3.1 (m, 4 H) 3.6 (m, 4 H) 4.3 (m, 1 H) 7.4 (m, 1 H) 8.0 (m, 1 H) 8.1 (m, 1 H) 8.2 (m, 1H).

### Part 2: Preparation of N-Cyclopentyl-N'-(5-piperazin-1-yl-pyridin-2-yl)-[1,3,5]triazine-2,4-diamine

To a stirring suspension of 4-[6-(4-cyclopentylamino-[1,3,5]triazin-2-ylamino)-pyridin-3-yl]-piperazine-1-carboxylic acid tert-butyl ester (0.030g, 0.068 mmol) in methanol (3 mL) was added appoximately 3 ml of a HCl/methanol solution and allowed to stir at ambient temperature. After stirring for 4 hours the reaction mixture was concentrated under reduced pressure to yield a yellow oil. To the oil was added diethyl ether and allowed to stand for 2 hours. The afforded solids were rinsed with diethyl ether, collected and dried in a vacuum oven overnight to afford N-cyclopentyl-N'-(5-piperazin-1-yl-pyridin-2-yl)-[1,3,5]triazine-2,4-diamine hydrochloride salt (0.032g). MS 341.5; mp, >290.

### EXAMPLE 15

The preparation of 3-Benzyl-1-cyclopentyl-7-(3,4,5,6-tetrahydro-2H-[1,3']bipyridinyl-6'-ylamino)-3,4-dihydro-1H-pyrimido[4,5-d]pyrimidin-2-one (8) is depicted in Scheme 9 below and described in the following experimental procedure.

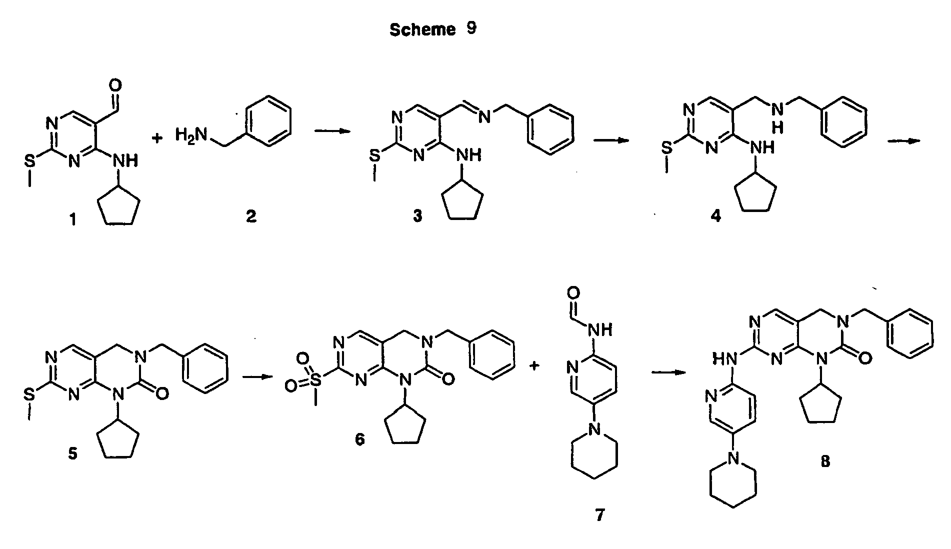

### Step 1

### Preparation of [5-(Benzylimino-methyl)-2-methylsulfanyl-pyrimidin-4-yl]-cyclopentyl-amine (3)

A solution of 4-cyclopentylamino-2-methylsulfanyl-pyrimidine-5-carboxaldehyde (1, 4.75 g, 0.02 mmol) and benzylamine (2, 2.29 mL, 0.021 mmol) in toluene (75 mL) was heated under reflux with a Dean-Stark trap overnight. The solution was cooled and concentrated to give the product as a gum. MS, 327.2.

### Step 2

### Preparation of [5-(Benzylamino-methyl)-2-methylsulfanyl-pyrimidin-4-yl]-cyclopentyl-amine (4)

To a solution of 3 (assumed 0.02 mmol) in methanol (50 mL), cooled by an ice-bath, was added portionwise sodium borohydride (1.13 g, 0.03 mmol). The solution was stirred at room temperature under nitrogen overnight. The solution was concentrated to give a gum which was dissolved in dichloromethane, washed with 1 N sodium hydroxide, dried over magnesium sulfate, and concentrated to give the product as a yellow oil (4.70 g, 71.5%). MS 329.2.

### Step 3

### Preparation of 3-Benzyl-1-cycloyentyl-7-methylsulfanyl-3,4-dihydro-1H-pyrimido[4,5-d]pyrimidin-2-one (5)

To a solution of 4 (0.33 g, 1.0 mmol) and triethylamine (0.61 mL, 4.4 mmol) in dichloromethane (10 mL), cooled in an ice-bath, was added portionwise triphosgene (0.33 g, 1.1 mmol). The ice-bath was removed and the reaction was stirred for two hours. The reaction was diluted with dichloromethane, washed with water, dried over magnesium sulfate, and concentrated. Chromatography over silica gel using ethyl acetate/ hexane gave the product as a colorless gum (0.295 g, 83.4%). MS 355.1.

### Step 4

### 3-Benzyl-1-cyclopentyl-7-methanesulfonyl-3,4-dihvdro-1H-pyrimido[4,5-d]pyrimidin-2-one (6)

To a solution of 5 (0.262 g, 0.738 mmol) in dichloromethane (10 mL) was added 3-chloroperoxybenzoic acid (0.340 g, 75% pure, 1.48 mmol), and the solution was stirred overnight. More 3-chloroperoxybenzoic acid (0.116 g, 75% pure, 0.504 mmol) was added, and the solution was stirred another 4 hours. The solution was diluted with dichloromethane, washed with saturated aqueous sodium bicarbonate, dried over magnesium sulfate, and concentrated. Chromatography over silica gel using ethyl acetate/ hexane gave the product as a colorless gum (0.152 g, 53.3%). MS 387.1.

### Step 5

### Preparation of 3-Benzyl-1-cyclopentyl-7-(3,4,5,6-tetrahydro-2H-[1,3']bipyridinyl-6'-ylamino)-3,4-dihydro-1H-pyrimido[4,5-d]pyrimidin-2-one (8)

To a suspension of N-(3,4,5,6-tetrahydro-2H-[1,3']bipyridinyl-6'-yl)-formamide (7, 0.196 g, 0.955 mmol) in toluene (4 mL), cooled by an ice-bath, was added a solution of lithium hexamethyldisilazide in THF (1 M, 0.96 mL, 0.96 mmol). The suspension was cooled in an ice-bath and treated with a solution of **6** (0.336 g, 0.869 mmol) in toluene (5 mL). The reaction was heated at 70 °C for five hours, cooled to room temperature, and then treated with methanol (1 mL). After one hour, the reaction was diluted with dichloromethane, washed with water, dried over magnesium sulfate, and concentrated. Acetonitrile (25 mL) was added to the residue and the resulting solid was collected by filtration. Recrystallization from boiling acetonitrile gave the product as an off-white solid (0.219 g, 52.0%), mp 194-196 °C.
Anal. Calc. For C₂₈H₃₃N₇O₁: C, 69.54; H, 6.88; N, 20.27
Found: C, 69.38; H, 7.01; N, 20.31

3-Benzyl-1-cyclopentyl-7-(5-piperazin-1-yl-pyridin-2-ylamino)-3,4-dihydro-1H-pyrimido[4,5-d]pyrimidin-2-one was prepared analogously.

### EXAMPLE 16

The preparation of 2,4 diamino pyridines of Formula I of the present invention is depicted in Scheme 10 below:

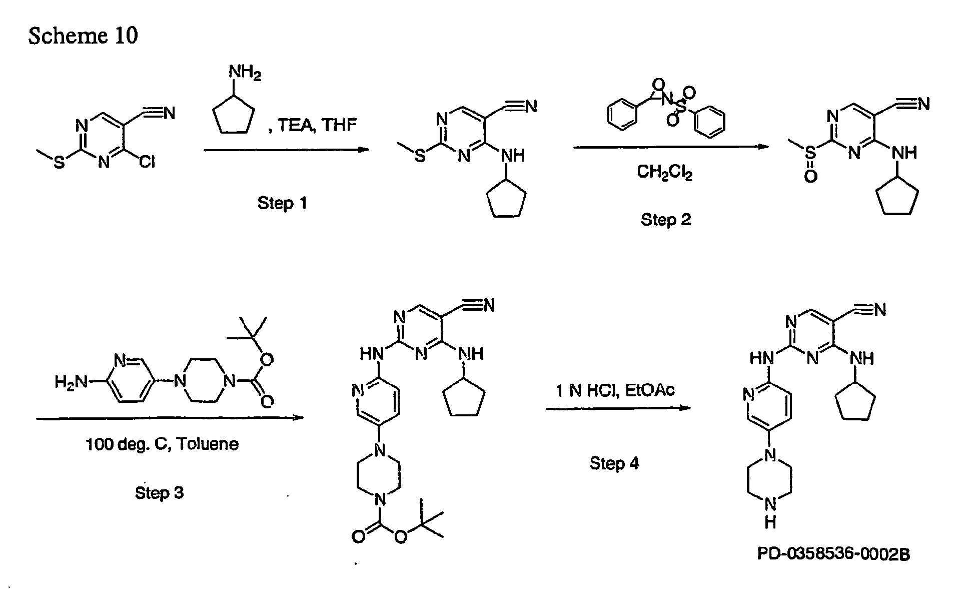

### Preparation of 4-Cyclopentylamino-2-(5-piperazin-1-yl-pyridin-2-ylamino)-pyrimidine-5-carbonitrile

### Step 1

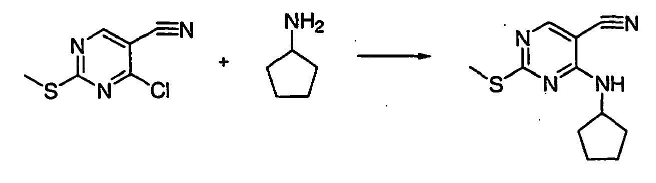

4-Chloro-2-methylsulfanyl-pyrimidine-5-carbonitrile (10.45 g, 56.3 mmol), triethylamine (11.4 g, 112.6 mmol) and cyclo-pentyl amine (5.03 g, 59.1 mmol) were dissolved in THF (100 mL). A precipitate formed almost immediately, as the reaction stirred at room temperature. After 1 hour, the precipitate was filtered and the solvent evaporated *in vacuo* to give 4-cyclopentylamino-2-methylsulfanyl-pyrimidine-5-carbonitrile (13.18 g, 99 %) as a pale orange solid.

### Step 2

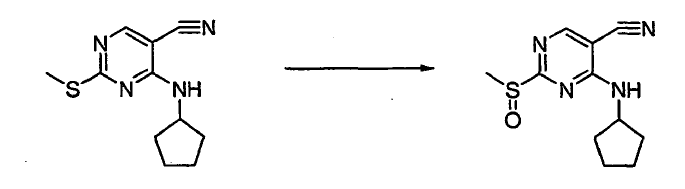

4-Cyclopentylamino-2-methylsulfanyl-pyrimidine-5-carbonitrile (2.0 g, 8.5 mmol) was dissolved in dichloromethane (25 mL), 2-benzenesulfonyl-3-phenyl-oxaziridine (2.9 g, 11.1 mmol) was added and stirred at room temperature for 6 hours. The reaction mixture was loaded directly onto a silica gel column eluting with EtOAc and hexanes to give 4-cyclopentylamino-2-methanesulfinyl-pyrimidine-5-carbonitrile (1.33 g, 62.3 %) as a white solid.

### Step 3

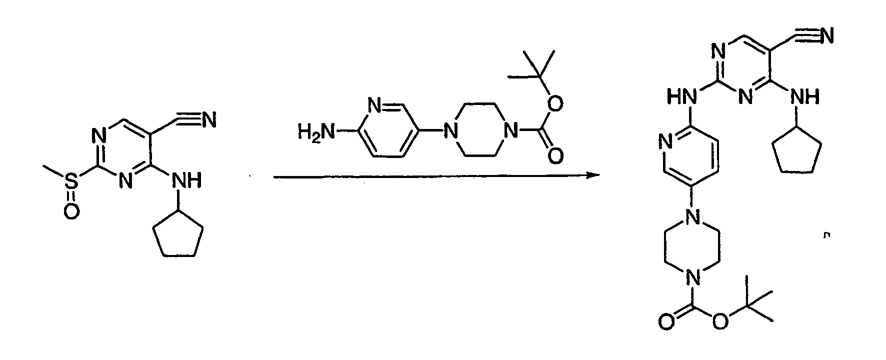

4-Cyclopentylamino-2-methanesulfinyl-pyrimidine-5-carbonitrile (0.352 g, 1.406 mmol) and 4-(6-amino-pyridin-3-yl)-piperazine-1-carboxylic acid tert-butyl ester (0.540 g, 1.94 mmol) were dissolved in Toluene (3 mL) and heated to 100 °C for 18 hours and allowed to cool to room temperature overnight. The reaction mixture was cooled and purified on a SiO₂ column to give an orange oil that was triturated with hexanes to provide 4-[6-(5-cyano-4-cyclopentylamino-pyrimidin-2-ylamino)-pyridin-3-yl]-piperazine-1-carboxylic acid tert-butyl ester (0.085 g, 13.0 %) as an orange solid.

### Step 4

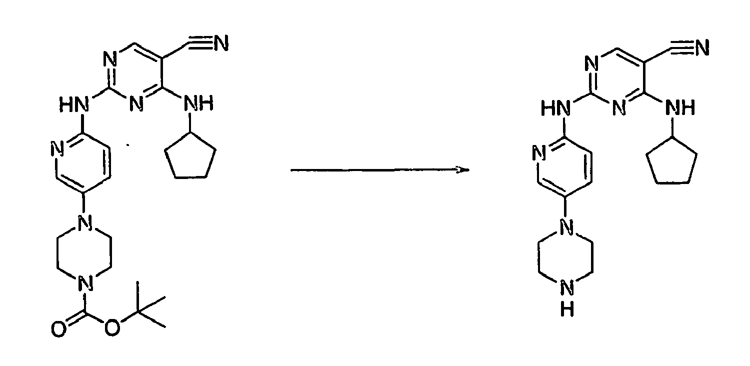

4-[6-(5-Cyano-4-cyclopentylamino-pyrimidin-2-ylamino)-pyridin-3-yl]-piperazine-1-carboxylic acid tert-butyl ester (0.060 g, 0.129 mmol) was dissolved in EtOAc (3mL) and 3 mL of 1 N HCl was added and the reaction mixture was stirred at room temperature for 18 hours. The reaction mixture was concentrated to give a crude dark tan mixture. This was dried in an oven vacuum at 60°C for 16 hours to give 4-Cyclopentylamino-2-(5-piperazin-1-yl-pyridin-2-ylamino)-pyrimidine-5-carbonitrile (0.034 g, 72.2 %) as a dark solid.

### EXAMPLE 16

The general scheme for preparing 4 amino and 4 methyl amino compounds of Formula I of the present invention is depicted below in Examples 16A through 160.

### EXAMPLE 16A

Preparation of 5-Bromo-N-4-cyclopentyl-N-2-(5-piperazin-1-yl-pyridin-2-yl)-pyrimidine-2,4-diamine was prepared as below. 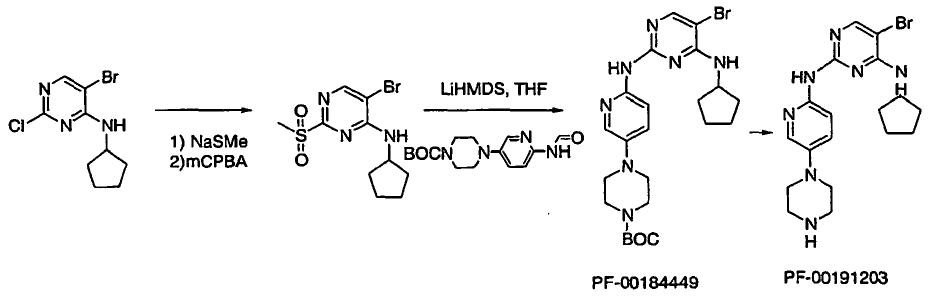 1H NMR (400 MHz, METHANOL-D4) δ ppm 1.66 (m, 4 H) 1.82 (m, 2 H) 2.10 (m, 2 H) 3.40 (dd, *J*=6.59, 2.93 Hz, 4 H) 3.45 (m, 4 H) 4.57 (m, 1 H) 7.19 (d, *J*=9.04 Hz, 1 H) 7.75 (dd, *J*=9.16, 3.05 Hz, 1 H) 8.06 (d, *J*=2.69 Hz, 1 H) 8.17 (s, 1 H).

### EXAMPLE 16B

Preparations of 5-Bromo-N-4-cyclopentyl-N-4-methyl-N-2-(4-morpholin-4-yl-phenyl)-pyrimidine-2,4-diamine, 4-{4-[5-Bromo-4-(cyclopentyl-methyl-amino)-pyrimidin-2-ylamino]-phenyl}-piperazine-1-carboxylic acid tert-butyl ester and 5-Bromo-N-4-cyclopentyl-N-4-methyl-N-2-(4-piperazin-1-yl-phenyl)-pyrimidine-2,4-diamine were prepared as depicted below.

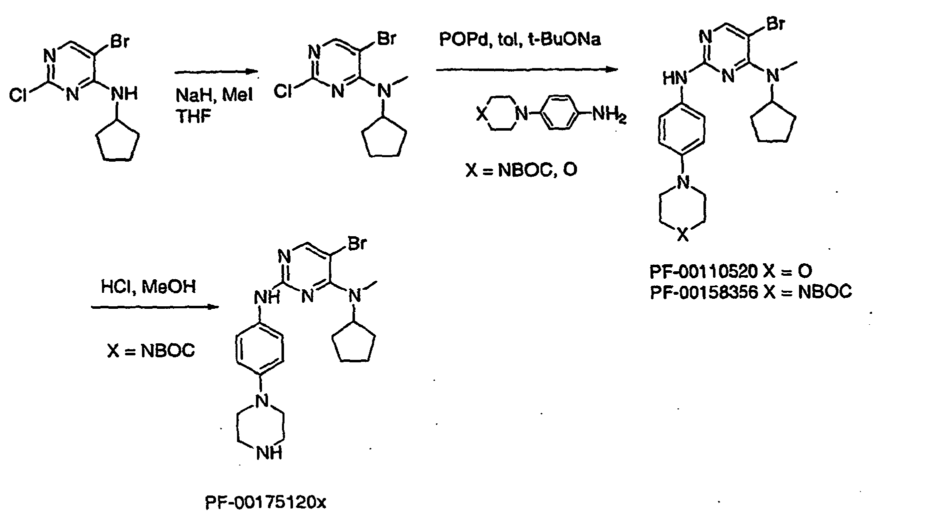

### EXAMPLE 16C

Preparation of 5-Bromo-N-4-cyclopentyl-N-4-methyl-N-2-(6-morpholin-4-yl-pyridin-3-yl)-pyrimidine-2,4-diamine was prepared as below.

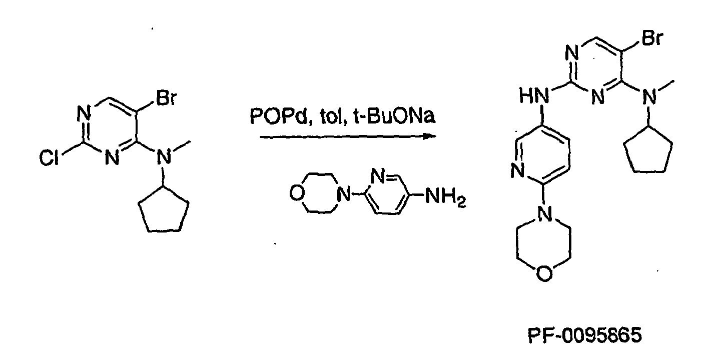

### EXAMPLE 16D

Preparation of 5-Bromo-N-4-cyclopentyl-N-4-methyl-N-2-(5-piperazin-1-yl-pyridin-2-yl)-pyrimidine-2,4-diamine was prepared as depicted below.

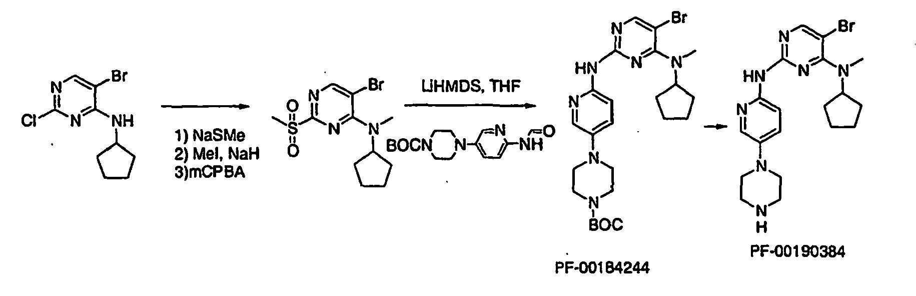

Examples 16E-16M depict the methods for preparing the intermediates for the compounds of the present Example 16.

### EXAMPLE 16E

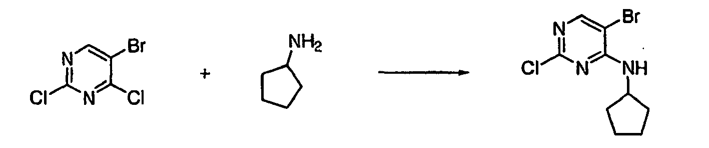

5-Bromo-2,4-dichloro-pyrimidine (18.21 g, 79.9 mmol) was dissolved in THF and place in a 0°C ice bath. Triethylamine (23.77 g, 235 mmol) was added. Reaction became light yellow. Following this, cyclopentyl amine (3.67 g, 78.31 mmol) was added. The ice bath was removed and the reaction was stirred overnight. Reaction was filtered and concentrated. Purified on silica gel using 20:1 Hexane/BtOAc to afford 16.09 g(74%) of (5-bromo-2-chloro-pyrimidin-4-yl)-cyclopentyl-amine.

### EXAMPLE 16F

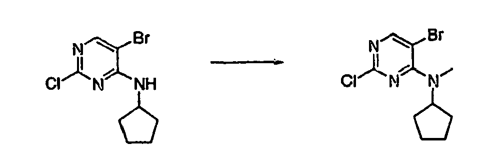

(5-Bromo-2-chloro-pyrimidin-4-yl)-cyclopentyl-amine (0.872 g, 3.15 mmol) was dissolved in THF and NaH (3.5 mmol) added, followed by MeI (0.492 g, 3.5 mmol). After 1h, added 150 mg NaH and 0.1 mL MeI. After 30 min, reaction slowly quenched with H₂O and extracted into EtOAc (3x). Reaction dried over Na₂SO₄ and concentrated in vacuo. Purified using 9:1 hexane/EtOAc by filtration through SiO₂ to afford 0.767 g (84%) yield of (5-Bromo-2-chloro-pyrimidin-4-yl)-cyclopentyl-methyl-amine.

### EXAMPLE 16G

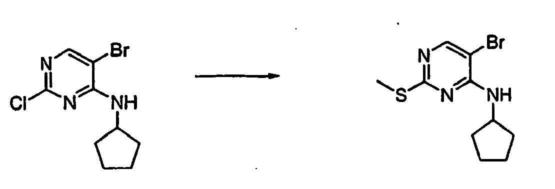

(5-Bromo-2-chloro-pyrimidin-4-yl)-cyclopentyl-amine (1.53 g, 5.53 mmol) was dissolved in DMSO and sodium thiomethoxide (0.969 g, 13.83 mmol) was added and the reaction stirred overnight, then poured into water and extracted 1x with EtOAc. Organic washed 3x with H₂O. Organic was dried over Na₂SO₄ and concentrated to afford 1.15 g (72%) of (5-bromo-2-methanesulfonyl-pyrimidin-4-yl)-cyclopentyl-amine.

### EXAMPLE 16H

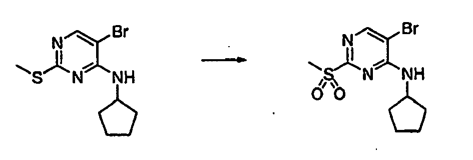

(5-Bromo-2-methylsulfanyl-pyrimidin-4-yl)-cyclopentyl-amine (0.406 g, 1.409 mmol) was dissolved in CH₂Cl₂ and cooled to 0C. mCPBA (70% purity, 1.042 g, 4.23 mmol) was added and the reaction was warmed to room temperature. The reaction was stirred for 2 h, then washed 1x with a saturated Na₂S₃O₅ solution, twice with saturated NaHO₃ and dried over Na₂SO₄ and concentrated in vacuo. Purification on silica gel using 3:1 Hexane/EtOAc to 1:1 Hexane/BtOAc afforded 0.249 g (55%) of (5-bromo-2-methylsulfanyl-pyrimidin-4-yl)-cyclopentyl-amine.

### EXAMPLE 16I

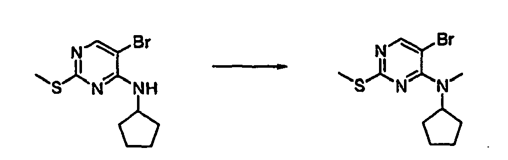

NaH (60% in oil, 0.100 g, 2.5 mmol) was added to(5-bromo-2-methylsulfanyl-pyrimidin-4-yl)-cyclopentyl-amine (0.654 g, 2.27 mmol) in THF and stirred for 30 min, MeI (0.354 g, 2.5 mmol) was added and allowed to stir for 1 h. After stirring 12h, added 60 mg NaH and -30 microliters MeI. Reaction stirred 1h, then quenched slowly with water and extracted 3x with EtOAc. Combined organics were dried over Na₂SO₄ and concentrated. Purified using silica gel, eluting with 95:5 Hexane/EtOAc to provide (5-bromo-2-methanesulfonyl-pyrimidin-4-yl)-cyclopentyl-methyl-amine.

### EXAMPLE 16J

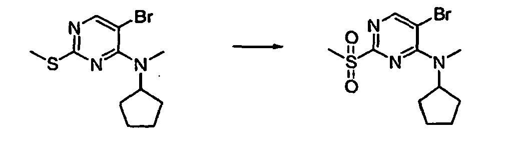

(5-Bromo-2-methanesulfonyl-pyrimidin-4-yl)-cyclopentyl-methyl-amine (0.429 g, 1.12 mmol) was dissolved in DCM at 0°C and mCPBA (0.245 g, 1.14 mmol) was added. Reaction warmed to room temperature and stirred overnight. Reaction was quenched with DMSO until homogeneous, then the organic was washed 2x with saturated NaHCO₃ and 1X with water and dried over Na₂SO₄ and conc. Purified using silica gel, 95:5 Hexans/EtOAc to 75:25 Hexane/EtOAc over 10 min to obtain 0.379 g (79 %) (5-bromo-2-methylsulfanyl-pyrimidin-4-yl)-cyclopentyl-methyl-amine.

### EXAMPLE 16K

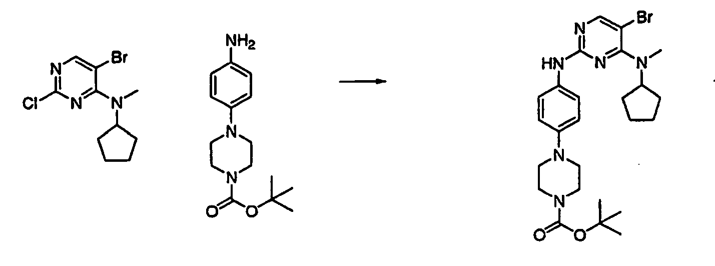

PF-00158356 (PF-0095865, PF-00153743 and PF-00110520 were made in a similar manner)

(5-Bromo-2-chloro-pyrimidin-4-yl)-cyclopentyl-methyl-amine (0.334 g, 1.149 mmol), POPd (0.029 g, 0.057 mmol), sodium tbutoxide (0.155 g, 1.61 mmol) and 4-(4-amino-phenyl)-piperazine-1-carboxylic acid tert-butyl ester (0.351 g, 1.26 mmol) were combined in toluene and heated to 95C for 17 h. The reaction was concentrated and purified using silica gel and 4:1 hexane/EtOAc to 1:1 hexane/EtOAc over 15 min to afford 0.195 g (32%) 4-{4-[5-bromo-4-(cyclopentyl-methyl-amino)-pyrimidin-2-ylamino]-phenyl}-piperazine-1-carboxylic acid tert-butyl ester.

### EXAMPLE 16L

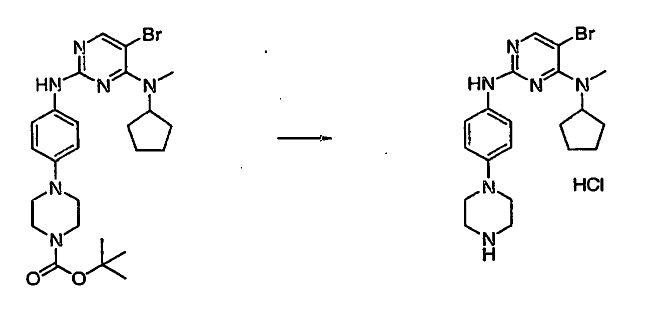

PF-00175120 (PF-0087361, PD-0338546-0002B, PF-00190384 and PF-00191203 were made in a similar manner).

4-{4-[5-Bromo-4-(cyclopentyl-methyl-amino)-pyrimidin-2-ylamino]-phenyl}-piperazine-1-carboxylic acid tert-butyl ester (0.147 g 0.277 mmol) was suspended in MeOH and MeOH saturated with HCl gas was added and the reaction stirred at room temperature 1 h. The reaction was concentrated in vacuo and placed under high vacuum 12h. The purple solid was triturated with Et₂O, collected and dried in vacuum oven at 60°C for 72 h to afford 0.137 g (98%) of 5-bromo-N-4-cyclopentyl-N-4-methyl-N-2-(4-piperazin-1-yl-phenyl)-pyrimidine-2,4-diamine.

### EXAMPLE 16M

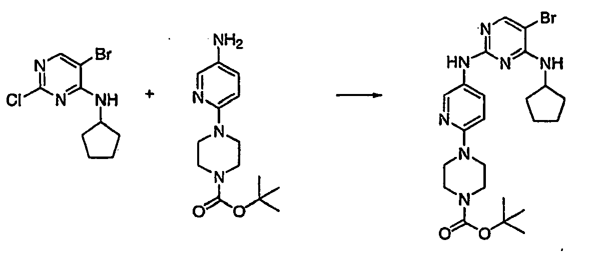

5-Bromo-N-4-cyclopentyl-N-2-(6-morpholin-4-yl-pyridin-3-yl)-pyrimidine-2,4-diamine was made in a similar manner.

(5-Bromo-2-chloro-pyrimidin-4-yl)-cyclopentyl-amine (0.155 g, 0.560 mmol) and 4-(5-amino-pyridin-2-yl)-piperazine-1-carboxylic acid tert-butyl ester (0.156 g, 0.560 mmol) were dissolved in toluene and heated to 115C for 48h. The reaction was cooled and concentrated. Purification on silica gel using 4:1 hexane/EtOAc provided 0.130 g (45%) of 4-[5-(5-bromo-4-cyclopentylamino-pyrimidin-2-ylamino)-pyridin-2-yl]-piperazine-1-carboxylic acid tert-butyl ester.

### EXAMPLE 17

### Biological Assays

To determine the inhibitory potency and selectivity of compounds of the present invention against Cdk4 and related kinases, compounds were evaluated in standard assays routinely used to measure inhibition of cyclin-dependent kinase enzymes and other protein kinases (see for example D. W. Fry et al., J. Biol. Chum. 2001, 276, 16617-16623). The assays were carried out as described below.

### Assay for inhibition of Cdk2/Cyclin A

Cdk2 enzyme assays for IC₅₀ determinations and kinetic evaluation are performed as follows. 96-well filter plates (Millipore MADVN6550) are used. The final assay volume is 0.1 mL containing buffer A (20 mM TRIS (tris[hydroxymethyl]aminomethane) (pH 7.4), 50 mM NaCl, 1 mM dithiothreitol, 10 mM MgCl₂), 12 mM ATP containing 0.25 µCi [³²P]ATP, 20 ng Cdk2/cyclin A, 1 µg retinoblastoma protein, and the test compound at appropriate dilutions in buffer A (Buffer A alone without added test compound was employed as a control for no inhibition. Buffer A containing excess EDTA was used to determine the level of background ³²P in the absence of enzyme activity). All components except the ATP are added to the wells, and the plate is placed on a plate mixer for 2 minutes. The reaction is initiated by addition of [³²P]ATP, and the plate is incubated at 25°C for 15 minutes. The reaction is terminated by addition of 0.1 mL 20% TCA. The plate is kept at 4°C for at least 1 hour to allow the substrate to precipitate. The wells are then washed five times with 0.2 mL 10% TCA, and ³²P incorporation is determined with a beta plate counter (Wallac Inc., Gaithersburg, MD). The IC₅₀ of the test compound was determined using the median effect method (Chou, T-C and Talalay, P. Applications of the median effect principle for the assessment of low-dose risk of carcinogens and for the quantitation of synergism and antagonism of chemotherapeutic agents. In: New Avenues in Developmental Cancer Chemotherapy (Eds. Harrap, K. T. and Connors, T. A.), pp. 37-64. Academic Press, New York, 1987).

### Assay for inhibition of Cdk4/Cyclin D

The Cdk4 enzyme assay for IC₅₀ determination and kinetic evaluation is performed as follows. 96-well filter plates (Millipore MADVN6550) are used. The total volume is 0.1 mL containing buffer A (20 mM TRIS (tris[hydroxymethyl]aminomethane) (pH 7.4), 50 mM NaCl, 1 mM dithiothreitol, 10 mM MgCl₂), 25 µM ATP containing 0.25 µCi [³²P]ATP, 20 ng Cdk4, 1 µg retinoblastoma protein and the test compound at appropriate dilutions in buffer A. Buffer A alone without added test compound was employed as a control for no inhibition. Buffer A containing excess EDTA was used to determine the level of background ³²P in the absence of enzyme activity. All components except the ATP are added to the wells, and the plate is placed on a plate mixer for 2 minutes. The reaction is started by adding [³²P]ATP, and the plate is incubated at 25°C for 15 minutes. The reaction is terminated by addition of 0.1 mL 20% trichloroacetic acid (TCA). The plate is kept at 4°C for at least 1 hour to allow the substrate to precipitate. The wells are then washed five times with 0.2 mL 10% TCA, and ³²P incorporation is determined with a beta plate counter (Wallac Inc., Gaithersburg, MD). The IC₅₀ of the test compound was determined using the median effect method (Chou, T-C and Talalay, P. Applications of the median effect principle for the assessment of low-dose risk of carcinogens and for the quantitation of synergism and antagonism of chemotherapeutic agents. In: New Avenues in Developmental Cancer Chemotherapy (Eds. Harrap, K. T. and Connors, T. A.), pp. 37-64. Academic Press, New York, 1987).

### Assay for inhibition of FGFr

For FGF receptor (FGFr) tyrosine kinase assays 96-well plates (100 µL/incubation/well), and conditions are optimized to measure the incorporation of ³²P from [γ³²P]ATP into a glutamate-tyrosine co-polymer substrate. Briefly, to each well is added 82.5 µL incubation buffer B (25 mM Hepes (pH 7.0), 150 mM NaCl, 0.1% Triton X-100, 0.2 mM PMSF, 0.2 mM Na₃VO₄, 10 mM MnCl₂) and 750 µg/mL Poly (4:1) glutamate-tyrosine followed by 2.5 µL of the test compound in buffer B and 5 µL of a 7.5 µg/µL FGFr solution to initiate the reaction: Following a 10-minute incubation at 25°C, 10 mL [γ³²P]ATP (0.4 µCi plus 50 µM ATP) is added to each well, and samples are incubated for an additional 10 minutes at 25°C. The reaction is terminated by the addition of 100 µL 30% trichloroacetic acid (TCA) containing 20 mM sodium pyrophosphate and precipitation of material onto glass fiber mats (Wallac). Filters are washed three times with 15% TCA containing 100 mM sodium pyrophosphate, and the radioactivity retained on the filters is counted in a Wallac 1250 Betaplate reader. Nonspecific activity is defined as radioactivity retained on the filters following incubation of samples with buffer alone (no enzyme). Specific enzymatic activity (enzyme plus buffer) is defined as total activity minus nonspecific activity. The concentration of a test compound that inhibited specific activity by 50% (IC₅₀) is determined based on the inhibition curve.

Results from the foregoing assays for several compounds of the present invention are presented in Table 1. For comparison, data are also provided for compounds having a carbon atom instead of the nitrogen at the 1 position of the pyridine ring of compounds of Examples 8, 10 and 13. These analogs differ from the Example compounds by the replacement of the pyridyl ring nitrogen atom by CH and are distinguished from compounds of the instant invention by a superscript prime (for example the phenylamino analog of Example compound 8 is denoted 8').

**Table 1A**

| Example | Cdk4/D IC₅₀ (µM) | Cdk2/A IC₅₀ (µM) | FGFr IC₅₀ (µM) |
|---|---|---|---|
| | | | |
| 8 | 0.510 | >5 | >5 |
| 10 | 0.335 | >5 | >5 |
| 13 | 0.004 | 1.7 | 4.010 |
| | | | |

**Table 1B***

| | | | |
|---|---|---|---|
| Compound | Cdk4/D IC₅₀ (µM) | Cdk2/A IC₅₀ (µM) | PGFr IC₅₀ (µM) |
| | | | |
| 8' | 0.014 | 0.913 | 1.86 |
| 10' | 0.028 | 4.125 | 1.196 |
| 13' | 0.001 | 0.028 | NA |

| | | | |
|---|---|---|---|
| *Aniline analogs of Examples 8, 10 and 13. (NA = not available) | | | |

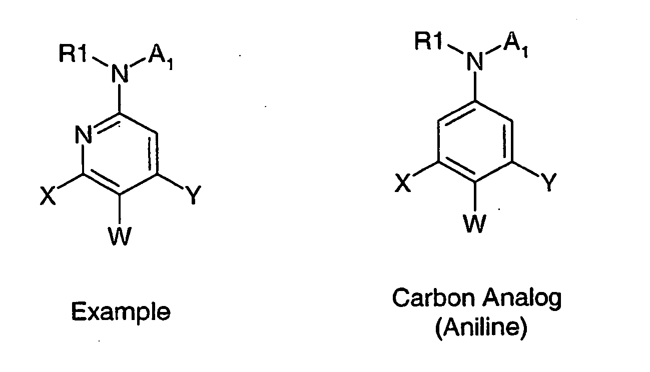

### EXAMPLE 18

### Formulations

The compounds of the present invention will typically be formulated with common excipients, diluents, and carriers to provide compositions that are well-suited for convenient administration to mammals. The following examples illustrate typical compositions that are provided in a further embodiment of this invention.

The compounds of the present invention may be freeze-dried, spray-dried, or evaporatively dried to provide a solid plug, powder, or film of crystalline or amorphous material. Microwave or radio frequency drying may be used for this purpose.

The compounds of the invention may be administered alone or in combination with other drugs and will generally be administered as a formulation in association with one or more pharmaceutically acceptable excipients. The term "excipient" is used herein to describe any ingredient other than the compound of the invention. The choice of excipient will to a large extent depend on the particular mode of administration.

### EXAMPLE 18A - ORAL ADMINISTRATION

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

### EXAMPLE 18A-1

### Tablet Formulation of the compound of Example 8

| Tablet Formulation | |
|---|---|
| Ingredient | Amount |
| Compound of Example 8 | 50 mg* |
| Lactose | 80 mg |
| Cornstarch (for mix) | 10 mg |
| Cornstarch (for paste) | 8 mg |
| Magnesium Stearate (1%) | 2 mg |
| | 150 mg |

| | |
|---|---|
| * Quantity adjusted in accordance with drug activity. | |

A compound of the present invention is mixed with the lactose and cornstarch (for mix) and blended to uniformity to a powder. The cornstarch (for paste) is suspended in 6 mL of water and heated with stirring to form a paste. The paste is added to the mixed powder, and the mixture is granulated. The wet granules are passed through a No. 8 hard screen and dried at 50°C. The mixture is lubricated with 1% magnesium stearate and compressed into a tablet. The tablets are administered to a patient at the rate of 1 to 4 each day for prevention and treatment of cancer.

### EXAMPLE 18A-2

Another composition of a typical tablet in accordance with the invention may comprise:

| Ingredient | % w/w |
|---|---|
| Compound of Example 8 | 10.00* |
| Microcrystalline cellulose | 64.12 |
| Lactose | 21.38 |
| Croscarmellose sodium | 3.00 |
| Magnesium stearate | 1.50 |

| | |
|---|---|
| * Quantity adjusted in accordance with drug activity. | |

A typical tablet may be prepared using standard processes known to a formulation chemist, for example, by direct compression, granulation (dry, wet, or melt), melt congealing, or extrusion. The tablet formulation may comprise one or more layers and may be coated or uncoated.

Examples of excipients suitable for oral administration include carriers, for example, cellulose, calcium carbonate, dibasic calcium phosphate, mannitol and sodium citrate, granulation binders, for example, polyvinylpyrrolidine, hydroxypropylcellulose, hydroxypropylmethylcellulose and gelatin, disintegrants, for example, sodium starch glycolate and silicates, lubricating agents, for example, magnesium stearate and stearic acid, wetting agents, for example, sodium lauryl sulphate, preservatives, anti-oxidants, flavours and colourants.

Solid formulations for oral administration may be formulated to be immediate and/or modified release. Modified release formulations include delayed-, sustained-, pulsed-, controlled dual-, targeted and programmed release. ' Details of suitable modified release technologies such as high energy dispersions, osmotic and coated particles are to be found in Verma et al, Pharmaceutical Technology On-line, 25(2), 1-14 (2001). Other modified release formulations are described in US Patent No. 6,106,864.

### EXAMPLE 18B - PARENTERAL ADMINISTRATION

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art. The solubility of compounds of formula (I) used in the preparation of parenteral solutions may be increased by suitable processing, for example, the use of high energy spray-dried dispersions (see WO 01/47495) and/or by the use of appropriate formulation techniques, such as the use of solubility-enhancing agents.

Formulations for parenteral administration may be formulated to be immediate and/or modified release. Modified release formulations include delayed-, sustained-, pulsed-, controlled dual-, targeted and programmed release.

To a solution of 700 mL of propylene glycol and 200 mL of water for injection is added 20.0 g of the Compound of Example 8 of the present invention. The mixture is stirred and the pH is adjusted to 5.5 with hydrochloric acid. The volume is adjusted to 1000 mL with water for injection. The solution is sterilized, filled into 5.0 mL ampoules, each containing 2.0 mL (40 mg of compound), and sealed under nitrogen. The solution is administered by injection to a patient suffering from cancer and in need of treatment.

### EXAMPLE 18C - TOPICAL ADMINISTRATION

The compounds of the invention may also be administered topically to the skin or mucosa, either dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin and propylene glycol. Penetration enhancers may be incorporated - see, for example, J Pharm Sci, 88 (10), 955-958 by Finnin and Morgan (October 1999).

Other means of topical administration include delivery by iontophoresis, electroporation, phonophoresis, sonophoresis and needle-free or microneedle injection.

Formulations for topical administration may be formulated to be immediate and/or modified release. Modified release formulations include delayed-, sustained-, pulsed-, controlled dual-, targeted and programmed release. Thus compounds of the invention may be formulated in a more solid form for administration as an implanted depot providing long-term release of the active compound.

### EXAMPLE 18D - INHALED/INTRANASAL ADMINISTRATION

The compounds of the invention can also be administered intranasally or by inhalation, typically in the form of a dry powder (either alone, as a mixture, for example, in a dry blend with lactose, or as a mixed component particle, for example, mixed with phospholipids) from a dry powder inhaler or as an aerosol spray from a pressurised container, pump, spray, atomiser (preferably an atomiser using electrohydrodynamics to produce a fine mist), or nebuliser, with or without the use of a suitable propellant, such as dichlorofluoromethane.

The pressurised container, pump, spray, atomizer, or nebuliser contains a solution or suspension of the active compound comprising, for example, ethanol (optionally, aqueous ethanol) or a suitable alternative agent for dispersing, solubilising, or extending release of the active, the propellant(s) as solvent and an optional surfactant, such as sorbitan trioleate or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

A suitable solution formulation for use in an atomiser using electrohydrodynamics to produce a fine mist may contain from 1µg to 10mg of the compound of the invention per actuation and the actuation volume may vary from 1µl to 100µl. A typical formulation may comprise a compound of this invention, propylene glycol, sterile water, ethanol and sodium chloride. Alternative solvents which may be used instead of propylene glycol include glycerol and polyethylene glycol.

Capsules, blisters and cartridges (made, for example, from gelatin or HPMC) for use in an inhaler or insufflator may be formulated to contain a powder mix of the compound of the invention, a suitable powder base such as lactose or starch and a performance modifier such as *l*-leucine, mannitol, or magnesium stearate.

In the case of dry powder inhalers and aerosols, the dosage unit is determined by means of a valve which delivers a metered amount. Units in accordance with the invention are typically arranged to administer a metered dose or "puff" appropriate for the disease state, age and size of the individual. The overall daily dose may be administered in a single dose or, more usually, as divided doses throughout the day.

Formulations for inhaled/intranasal administration may be formulated to be immediate and/or modified release. Modified release formulations include delayed-, sustained-, pulsed-, controlled dual-, targeted and programmed release.

### EXAMPLE 18E - RECTAL/INTRAVAGINAL ADMINISTRATION

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled dual-, targeted and programmed release.

### EXAMPLE 18F - OCULAR/ANDIAL ADMINISTRATION

The compounds of the invention may also be administered directly to the eye or ear, typically in the form of drops of a micronised suspension or solution in isotonic, pH-adjusted, sterile saline. Other formulations suitable for ocular and andial administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose,or a heteropolysaccharide polymer, for example, gelan gum, may be incorporated together with a preservative, such as benzalkonium chloride. Such formulations may also be delivered by iontophoresis.

Formulations for ocular/andial administration may be formulated to be immediate and/or modified release. Modified release formulation include delayed-, sustained-, pulsed-, controlled dual-, targeted, or programmed release.

The compounds of the invention may be combined with soluble macromolecular entities such as cyclodextrin or polyethylene glycol-containing polymers to improve their solubility, dissolution rate, taste-masking, bioavailability and/or stability.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta-and gamma-cyclodextrins, examples of which may be found in International Patent Applications Nos. WO 91/11172, WO 94/02518 and WO 98/55148.

### DOSAGE

For administration to human patients, the total daily dose of the compounds of the invention is typically in the range 0.1 mg to approximately 3000 mg depending, of course, on the mode of administration. For example, oral administration may require a total daily dose of from 10 mg to 3000 mg, while an intravenous dose may only require from 0.1 mg to 1000 mg/kg of body weight. The total daily dose may be administered in single or divided doses.

These dosages are based on an average human subject having a weight of about 65 to 70kg. The physician will readily be able to determine doses for subjects whose weight falls outside this range, such as infants and the elderly. The invention and the manner and process of making and using it, are now described in such full, clear, concise, and exact terms as to enable any person skilled in the art to which it pertains, to make and use the same. It is to be understood that the foregoing describes preferred embodiments of the present invention and that modifications may be made therein without departing from the spirit or scope of the present invention as set forth in the claims. To particularly point out and distinctly claim the subject matter regarded as invention, the following claims conclude this specification.

## Claims

1. A compound of the formula I: 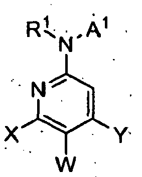 or a pharmaceutically acceptable salt, ester, or amide thereof,
wherein:
A¹ is a monocyclic or bicyclic heteroaromatic ring system selected from: 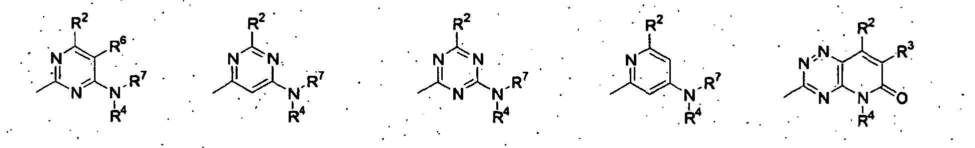 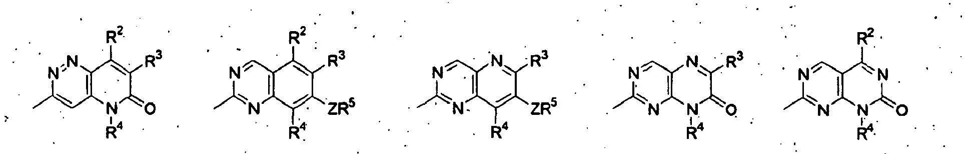 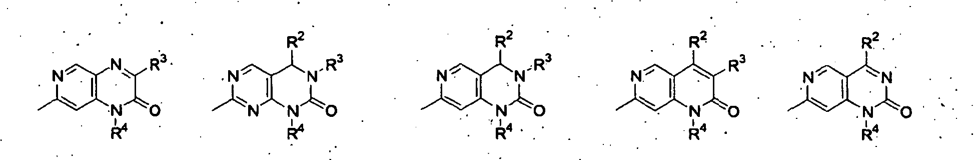 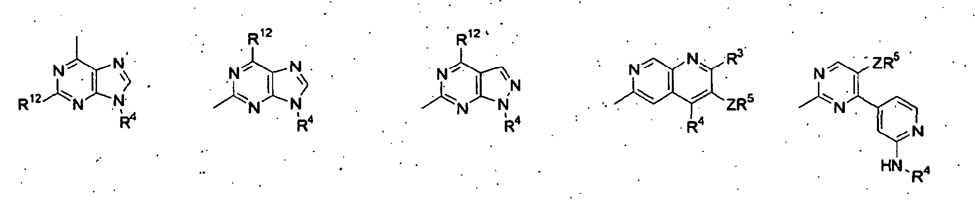
wherein:
R¹ is, in each instance, independently, hydrogen, C₁-C₆ alkyl, C₁-C₆ alkenyl, C₁-C₆ acyl, aryloxycarbonyl, C₁-C₁₀alkoxycarbonyl, or tri-C₁-C₁₀alkylsilyl;
R², R³, R⁴, R⁵, R⁸, R⁹, R¹⁰ and R¹¹ are, in each instance, independently selected from hydrogen, C₁-C₁₀alkyl, C₁-C₁₀alkylamino, C₃-C₇cycloalkyl, aryl, heteroaryl, and heterocyclyl;
R⁶ is independently, in each instance, selected from hydrogen, halogen, nitrile, nitro, C₁-C₈alkyl, C₂-C₈alkenyl, C₂-C₈alkynyl, C₁-C₁₀alkylcarbonyl, C₁-C₁₀alkoxycarbonyl, C ₃-C₇ cycloalkyl, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙ(O)(OR¹⁰)(OR¹¹), (CR⁸R⁹)ₙ-aryl, (CR⁸R⁹)ₙ-heteroaryl, T(CH₂)ₘQR⁸, -C(O)T(CH₂)ₘQR⁸, NR⁸C(O)T(CH₂)ₘQR⁸, and -CR⁸=CR⁹C(O)R¹⁰;
R⁷ is independently, in each instance, hydrogen, C₁-C₁₀acyl, C₁-C₁₀alkoxycarbonyl, aryloxycarbonyl, C₁-C₈alkyl, or C₂-C₈alkenyl,
R¹² is independently, in each instance, hydrogen, C₁-C₁₀acyl, arylC₁-C₁₀alkyl, C₁-C₁₀ alkylamino, or arylamino;
R⁸ and R⁹ may optionally form a carbocyclic group containing 3-7 members preferably 5-6 members, up to four of which are optionally heteroatoms independently selected from oxygen, sulfur, and nitrogen, wherein the carbocyclic group is unsubstituted or substituted with one, two, or three groups said groups in each instance independently selected from halogen, hydroxy, hydroxyC₁-C₁₀alkyl, nitrile, C₁-C₄alkyl, C₁-C₄alkoxy, C₁-C₁₀alkoxycarbonyl, C₁-C₁₀alkylcarbonyl, C₁-C₁₀alkylcarbonylamino, aminoC₁-C₁₀alkyl, trifluoromethyl, N-hydroxyacetamide, trifluoromethylalkyl, amino, or mono- or di-C₁-C₁₀alkylamino, (CH₂)ₙC(O)NR¹⁰R¹¹, and O(CH₂)ₙC(O)OR¹⁰;
T is, in each instance, independently, O, S, NR⁹, N(O)R⁹, or CR⁹R¹⁰;
Q is, in each instance, independently, O, S, NR⁹, N(O)R9, CO₂, O(CH₂)ₙ-heteroaryl, O(CH₂)ₙS(O)ₘR⁹, or (CH₂)-heteroaryl;
X and Y are in each instance independently selected from hydrogen, halogen, nitrile, C₁-C₆ alkyl, C₁-C₆ alkylcarbonyl, C₁-C₆ alkoxycarbonyl, nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-aryl, (CR⁸R⁹)ₙ-heteroaryl, -T(CH₂)ₘQR⁸, C(O)T(CH₂)ₘQR⁸, NR⁸C(O)T(CH₂)ₘQR⁸, and -CR⁸=CR⁹C(O)R¹⁰;
W is selected from hydrogen, halogen, C₁-C₈ alkyl, C₃-C₇ cycloalkyl, C₁-C₈ alkoxy, C₁-C₈ alkoxyalkyl, C₁-C₈ haloalkyl, C₁-C₈ hydroxyalkyl, C₂-C₈ alkenyl, C₂-C₈ alkynyl, nitrile, nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙ(O)(OR¹⁰)(OR¹¹), (CR⁸R⁹)ₙ-aryl, (CR⁸R⁹)ₙ-heteroaryl,-T(CH₂)ₘQR⁸, -C(O)T(CH₂)ₘQR⁸, NR⁸C(O)T(CH₂)ₘQR⁹, and -CR⁸=CR⁹C(O)R¹⁰;
W and one of X or Y may optionally form an aromatic ring containing up to three heteroatoms and optionally substituted by up to 4 groups independently selected from halogen, hydroxy, hydroxyC₁-C₁₀alkyl, C₁-C₄alkyl, C₁-C₄alkoxy, C₁-C₁₀alkoxycarbonyl, C₁-C₁₀alkylcarbonyl, C₁-C₁₀alkylcarbonylamino, and aminoC₁-C₁₀alkyl, aminoC₁-C₁₀alkylcarbonyl, trifluoromethyl, trifluoromethylC₁-C₁₀alkyl, trifluoromethylC₁-C₁₀alkylaminoC₁-C₁₀alkyl, amino, mono- or di-C₁-C₁₀alkylamino, N-hydroxyacetamido, aryl, heteroaryl, carboxyC₁-C₁₀alkyl, nitrile, NR⁸SO₂R⁹, C(O)NR⁸R⁹, NR⁸C(O)R⁹, C(O)OR⁸, C(O)NR⁸SO₂R⁹, (CH₂)ₙS(O)ₙR⁸, (CH₂)ₙ-heteroaryl, O(CH₂)ₙ-heteroaryl, (CH₂)ₙC(O)NR⁸R⁹, O(CH2)ₙC(O)OR⁸, (CH₂)ₙSO₂NR⁸R⁹, and C(O)R⁸;
Z is in each instance, independently, O or NR⁶;
m is an integer of from 1-6,
n is an integer of from 0-6,
the term "heterocyclyl" means a cycloalkyl group also bearing at least one heteroatom selected from O, S, N or substituted nitrogen,
the term "aryl" means an aromatic ring system with no heteroatoms having a single ring, multiple rings, or multiple condensed rings in which at least one is aromatic, which can be mono-, di-, or trisubstituted,
the term "aryloxy" means "aryl-O-", wherein "aryl" is as defined above,
the term "heteroaryl" means an aromatic heterocycle containing five or six ring members, of which from 1 to 4 can be heteroatoms selected, independently, from N, S and O, and which rings can be unsubstituted, monosubstituted or disubstituted with substituents selected, independently, from the group consisting of halo, (C₁-C₄)alkyl, and (C₁-C₄)alkoxy, optionally substituted with from one to three fluorine atoms, and
the term "heteroaryloxy" means "heteroaryl-O", wherein heteroaryl is as defined above.

2. A compound of Claim 1, having the following structure: 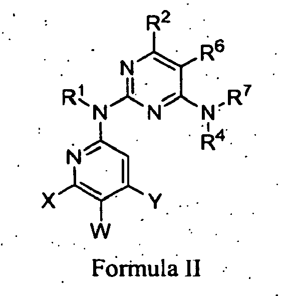 wherein:
R¹, R², R⁴, R⁵, R⁶, R⁷, R⁸, R⁹, R¹⁰, R¹¹, T, Q, W, X, Y, m and n are as defined as for Formula I.

3. A compound of claim 1 or claim 2 wherein R¹ and R² are independently, in each instance, hydrogen.

4. A compound according to claim 1 or claim2 wherein R⁴ is C₁-C₁₀alkyl.

5. A compound according to claim 1 or claim 2 wherein R⁶ is halogen or COR⁸.

6. A compound according to claim 1 or claim 2 wherein W is NR⁸R⁹.

7. A compound of claim 1 wherein Z is O.

8. A compound according to claim 1 or claim'2 wherein X and Y are hydrogen.

9. A compound according to claim 1 or claim 2 wherein R³ is halogen, or C₁-C₈ alkyl.

10. A compound according to claim 1 selected from the group consisting of:
4-Cyclopentylamino-2-(5-piperazin-1-yl-pyridin-2-ylamino)-pyrimidine-5-carbonitrile,
N4-Cyclopentyl-5-nitro-N2-(5-piperazin-1-yl-pyridin-2-yl)-pyrimidine-2,4-diamine,
4-Cyclopentylamino-2-(5-piperazin-1-yl-pyndin-2-ylamino)-pyrimidine-5-carbaldehyde,
4-Cyclopentylamino-2-(5-piperazin-1-yl-pyndin-2-ylamino)-pyrimidine-5-carboxylic acid ethyl ester,
4-Cyclopentylamino-2-(5-piperazin-1-yl-pyridin-2-ylamino)-pyrimidine-5-carboxylic acid methyl ester,
[4-Cyclopentylamino-2-(5-piperazin-1-yl-pyridin-2-ylamino)-pyrimidin-5-yl]-methanol,
1-[4-Cyclopentylamino-2-(5-piperazin-1-yl-pyridin-2-ylamino)-pyrimidin-5-yl]-ethanone,
3-[4-Cyclopentylamino-2-(5-piperazin-1-yl-pyridin-2-ylamino)-pyrimidin-5-yl]-but-2-enoic acid ethyl ester,
4-Amino-2-(5-piperazin-1-yl-pyridin-2-ylamino)-pyrimidine-5-carbonitrile,
5-Nitro-N2-(5-piperazin-1-yl-pyridin-2-yl)-pyrimidine-2,4-diamine,
4-Amino-2-(5-piperazin-1-yl-pyridin-2-ylamino)-pyrimidine-5-carbaldehyde,
4-Amino-2-(5-piperazin-1-yl-pyridin-2-ylamino)-pyrimidine-5-carboxylic acid ethyl ester,
4-Amino-2-(5-piperazin-1-yl-pyridin-2-ylamino)-pyrimidine-5-carboxylic acid methyl ester,
[4-Amino-2-(5-piperazin-1-yl-pyridin-2-ylamino)-pyrimidin-5-yl]-methanol,
1-[4-Amino-2-(5-piperazin-1-yl-pyridin-2-ylamino)-pyrimidin-5-yl]-ethanone,
3-[4-Amino-2-(5-piperazin-1-yl-pyridin-2-ylamino)-pyrimidin-5-yl]-but-2-enoic acid ethyl ester,
4-Cyclopentylamino-2-(5-pyrrolidin-1-yl-pyridin-2-ylamino)-pyrimidine-5-carbonitrile,
N2-[5-(3-Amino-pyrrolidin-1-yl)-pyridin-2-yl]-N4-cyclopentyl-5-nitro-pyrimidine-2,4-diamine,
4-Cyclopentylamino-2-(5-morpholin-4-yl-pyridin-2-ylamino)-pyrimidine-5-carbaldehyde,
4-Cyclopentylamino-2-(3,4,5,6-tetrahydro-2H-[1,3']bipyridinyl-6'-ylamino)-pyrimidene-5-carboxylic acid ethyl ester,
4-Cyclopentylamino-6-methyl-2-(5-piperazin-1-yl-pyridin-2-ylamino)-pyrimidine-5-carboxylic acid methyl ester,
{2-[5-(Bis-methoxymethyl-amino)-pyridin-2-ylamino]-4-cyclopentylamino-pyrimidin-5-yl}-methanol,
1-[4-Benzylamino-2-(5-piperazin-1-yl-pyridin-2-ylamino)-pyrimidin-5-yl]-ethanone,
4-[4-Cyclopentylamino-2-(5-piperazin-1-yl-pyridin-2-ylamino)-pyrimidin-5-yl]-pent-3-en-2-one,
4-Amino-2-(pyridin-2-ylamino)-pyrimidine-5-carbonitrile,
5-Nitro-N2-pyridin-2-yl-pyrimidine-2,4-diamine,
4-Amino-2-(pyridin-2-ylamino)-pyrimidine-5-carbaldehyde,
4-Amino-2-(pyridin-2-ylamino)-pyrimidine-5-carboxylic acid ethyl ester,
5-Bromo-N2-(5-piperazin-1-yl-pyridin-2-yl)-pyrimidine-2,4-diamine,
[4-Amino-2-(5-morpholin-4-yl-pyridin-2-ylamino)-pyrimidin-5-yl]-methanol,
1-[4-Amino-2-(5-morpholin-4-yl-pyridin-2-ylamino)-pyrimidin-5-yl]-ethanone,
[6-(5-Acetyl-4-amino-pyrimidin-2-ylamino)-pyridin-3-yloxy]-acetic acid,
4-Cyclopentylamino-2-(4-hydroxymethyl-5-pyrrolidin-1-yl-pyridin-2-ylamino)-pyrimidine-5-carbonitrile,
N2-[5-(3-Amino-pyrrolidin-1-yl)-6-chloro-pyridin-2-yl]-N4-cyclopentyl-5-nitro-pyrimidine-2,4-diamine,
2-(5-Bromo-pyridin-2-ylamino)-4-cyclopentylamino-pyrimidine-5-carbaldehyde,
4-Cyclopentylamino-2-(1H-pyrrolo[3,2-b]pyridin-5-ylamino)-pyrimidine-5-carboxylic acid ethyl ester,
4-Cyclopentylamino-2-(4,6-dichloro-5-piperazin-1-yl-pyridin-2-ylamino)-6-methyl-pyrimidine-5-carboxylic acid methyl ester,
2-(2-{5-[Bis-(2-methoxy-ethyl)-amino]-pyridin-2-ylamino}-4-cyclopentylamino-pyrimidin-5-yl)-2-methyl-propan-1-ol,
1-[4-Phenylamino-2-(5-piperazin-1-yl-pyridin-2-ylamino)-pyrimidin-5-yl]-ethanone,
4-[4-(3-Hydroxy-cyclopentylamino)-2-(5-piperazin-1-yl-pyridin-2-ylamino)-pyrimidin-5-yl]-pent-3-en-2-one,
4-[5-Cyano-2-(pyridin-2-ylamino)-pyrimidin-4-ylamino]-cyclohexanecarboxylic acid,
2-(4-Amino-5-nitro-pyrimidin-2-ylamino)-isonicotinic acid,
4-Amino-6-methyl-2-(pyridin-2-ylamino)-pyrimidine-5-carbaldehyde,
5-Iodo-N2-pyridin-2-yl-pyrimidine-2,4-diamine,
N-[5-Bromo-2-(5-piperazin-1-yl-pyridin-2-ylamino)-pyrimidin-4-yl]-acrylamide,
N2-(5-Piperazin-1-yl-pyridin-2-yl)-5-prop-1-ynyl-pyrimidine-2,4-diamine,
5-[2-(4-Fluoro-phenyl)-ethyl]-N2-(5-piperazin-1-yl-pyridin-2-yl)-pyrimidine-2,4-diamine,
[6-(4-Amino-5-propenyl-pyrimidin-2-ylamino)-pyridin-3-yloxy]-acetic acid,
5-Bromo-N4-cyclopentyl-N2-(5-pyrrolidin-1-yl-pyridin-2-yl)-pyrimidine-2,4-diamine,
N2-[5-(3-Amino-pyrrolidin-1-yl)-6-chloro-pyridin-2-yl]-5-bromo-N4-cyclopentyl-pyrimidine-2,4-diamine,
5-Bromo-N4-cyclopentyl-N2-(5-piperazin-1-yl-pyridin-2-yl)-pyrimidine-2,4-diamine,
5-Bromo-N4-cyclopentyl-N2-(1H-pyrrolo[3,2-b]pyridin-5-yl)-pyrimidine-2,4-diamine,
5-Bromo-N4-cyclopentyl-N2-(4,6-dichloro-5-piperazin-1-yl-pyridin-2-yl)-6-methyl-pyrimidine-2,4-diamine,
N2-{5-[Bis-(2-methoxy-ethyl)-amino]-pyridin-2-yl}-5-bromo-N4-cyclopentyl-pyrimidine-2,4-diamine,
5-Bromo-N4-phenyl-N2-(5-piperazin-1-yl-pyridin-2-yl)-pyrimidine-2,4-diamine,
3-[5-Bromo-2-(5-piperazin-1-yl-pyridin-2-ylamino)-pyrimidin-4-ylamino]-cyclopentanol,
N4-Cyclopentyl-5-iodo-N2-(5-pyrrolidin-1-yl-pyridin-2-yl)-pyrimidine-2,4-diamine,
N2-[5-(3-Amino-pyrrolidin-1-yl)-6-chloro-pyridin-2-yl]-N4-cyclopentyl-5-iodo-pyrimidine-2,4-diamine,
N4-Cyclopentyl-5-iodo-N2-(5-piperazin-1-yl-pyridin-2-yl)-pyrimidine-2,4-diamine,
N4-Cyclopentyl-5-iodo-N2-(1H-pyrrolo[3,2-b]pyridin-5-yl)-pyrimidine-2,4-diamine,
4-[6-(5-Bromo-4-cyclopentylamino-pyrimidin-2-ylamino)-pyridin-3-yl]-piperazine-1-carboxylic acid tert-butyl ester,
4-[6-(4-Cyclopentylamino-5-formyl-pyrimidin-2-ylamino)-pyridin-3-yl]-piperazine-1-carboxylic acid tert-butyl ester,
4-[6-(5-Acetyl-4-cyclopentylamino-pyrimidin-2-ylamino)-pyridin-3-yl]-piperazine-1-carboxylic acid tert-butyl ester,
2-[5-(4-tert-Butoxycarbonyl-piperazin-1-yl)-pyridin-2-ylamino]-4-cyclopentylamino-pyrimidine-5-carboxylic acid ethyl ester,
N-Cyclopentyl-N'-(5-piperazin-1-yl-pyridin-2-yl)-pyrimidine-4,6-diamine,
N-Isopropyl-N'-(5-piperazin-1-yl-pyridin-2-yl)-pyrimidine-4,6-diamine,
4-[6-(6-Cyclopentylamino-pyrimidin-4-ylamino)-pyridin-3-yl]-piperazine-1-carboxylic acid tert-butyl ester,
N-[5-(3-Amino-pyrrolidin-1-yl)-pyridin-2-yl]-N'-cyclopentyl-pyrimidine-4,6-diamine,
4-{6-[4-Cyclopentylamino-5-(1-methyl-3-oxo-but-1-enyl)-pyrimidin-2-ylamino]-pyridin-3-yl}-piperazine-1-carboxylic acid tert-butyl ester,
N-Cyclopentyl-N'-(5-piperazin-1-yl-pyridin-2-yl)-[1,3,5]triazine-2,4-diamine,
1-[4-Cyclopentylamino-2-(5-piperazin-1-yl-pyridin-2-ylamino)-pyrimidin-5-yl]-ethanone,
5-Bromo-N4-cyclopentyl-N2-(5-piperazin-1-yl-pyridin-2-yl)-pyridine-2,4-diamine,
4-Cyclopentylamino-6-(5-piperazin-1-yl-pyridin-2-ylamino)-nicotinonitrile,
N4-Cyclopentyl-5-nitro-N2-(5-piperazin-1-yl-pyridin-2-yl)-pylidine-2,4-diamine,
4-Cyclopentylamino-6-(5-piperazin-1-yl-pyridin-2-ylamino)-pyridine-3-carbaldehyde,
4-Cyclopentylamino-6-(5-piperazin-1-yl-pyridin-2-ylamino)-nicotinic acid ethyl ester,
4-Cyclopentylamino-6-(5-piperazin-1-yl-pyridin-2-ylamino)-nicotinic acid methyl ester,
[4-Cyclopentylamino-6-(5-piperazin-1-yl-pyridin-2-ylamino)-pyridin-3-yl]-methanol,
1-[4-Cyclopentylamino-6-(5-piperazin-1-yl-pyridin-2-ylamino)-pyridin-3-yl]-ethanone,
3-[4-Cyclopentylamino-6-(5-piperazin-1-yl-pyridin-2-ylamino)-pyridin-3-yl]-but-2-enoic acid ethyl ester,
(5-Cyclopentyl-5,6-dihydro-pyrido[2,3-e][1,2,4]triazin-3-yl)-(5-piperazin-1-yl-pyridin-2-yl)-amine,
(8-Cyclopentyl-7-methoxy-quinazolin-2-yl)-(5-piperazin-1-yl-pyridin-2-yl)-amine,
(8-Cyclopentyl-7-methoxy-pyrido[3,2-d]pyrimidin-2-yl)-(5-piperazin-1-yl-pyridin-2-yl)-amine,
6-Acetyl-8-cyclopentyl-2-(5-piperazin-1-yl-pyridin-2-ylamino)-8H-pteridin-7-one,
3-Acetyl-1-cyclopentyl-7-(5-piperazin-1-yl-pyridin-2-ylamino)-1H-pyrido[3,4-b]pyrazin-2-one,
1-Cyclopentyl-3-ethyl-4-methyl-7-(5-piperazin-1-yl-pyridin-2-ylamino)-3,4-dihydro-1H-pyrimido[4,5-d]pyrimidin-2-one,
1-Cyclopentyl-3-ethyl-4-methyl-7-(5-piperazin-1-yl-pyridin-2-ylamino)-3,4-dihydro-1H-pyrido[4,3-d]pyrimidin-2-one,
3-Acetyl-1-cyclopentyl-4-methyl-7-(5-piperazin-1-yl-pyridin-2-ylamino)-1H-[1,6]naphthyridin-2-one,
(9-Isopropyl-6-methyl-9H-purin-2-yl)-(5-piperazin-1-yl-pyridin-2-yl)-amine,
2-[9-Isopropyl-6-(5-piperazin-1-yl-pyridin-2-ylamino)-9H-purin-2-ylamino]-ethanol
N2-(4-Amino-cyclohexyl)-9-cyclopentyl-N6-(5-piperazin-1-yl-pyridin-2-yl)-9H-purine-2,6-diamine,
2-[9-Isopropyl-6-(5-piperazin-1-yl-pyridin-2-ylamino)-9H-purin-2-ylamino]-3-methyl-butan-1-ol,
(1-Isopropyl-4-methyl-1H-pyrazolo[3,4-d]pyrimidin-6-yl)-(5-piperazin-1-yl-pyridin-2-yl)-amine,
2-[1-Isopropyl-4-(5-piperazin-1-yl-pyridin-2-ylamino)-1H-pyrazolo[3,4-d]pyrimidin-6-ylamino]-ethanol,
N6-(4-Amino-cyclohexyl)-1-cyclopentyl-N4-(5-piperazin-1-yl-pyridin-2-yl)-1H-pyrazolo[3,4-d]pyrimidine-4,6-diamine,
2-[1-Isopropyl-4-(5-piperazin-1-yl-pyridin-2-ylamino)-1H-pyrazolo[3,4-d]pyrimidin-6-ylamino]-3-methyl-butan-1-ol,
5-Cyclopentyl-7-(1-hydroxy-ethyl)-8-methyl-3-(5-piperazin-1-yl-pyridin-2-ylamino)-5H-pyrido[3,2-c]pyridazin-6-one,
5-Cyclopentyl-8-methyl-3-(5-piperazin-1-yl-pyridin-2-ylamino)-5H-pyrido[3,2-c]pyridazin-6-one,
7-Benzyl-5-cyclopentyl-3-(5-piperazin-1-yl-pyridin-2-ylamino)-5H-pyrido[3,2-c]pyridazin-6-one,
(2-Ethyl-4-isopropyl-3-methoxy-[1,7]naphthyridin-6-yl)-pyridin-2-yl-amine,
(2,4-Diisopropyl-3-methoxy-[1,7]naphthyridin-6-yl)-(5-isopropenyl-pyridin-2-yl)-amine,
[4-(2-Ethylamino-pyridin-4-yl)-pyrimidin-2-yl]-(5-piperazin-1-yl-pyridin-2-yl)-amine,
[4-(5-Ethyl-2-methylamino-pyridin-4-yl)-pyrimidin-2-yl]-(5-morpholin-4-yl-pyridin-2-yl)-amine,
[5-Methoxy-4-(2-methylamino-pyridin-4-yl)-pyrimidin-2-yl]-(5-morpholin-4-yl-pyridin-2-yl)-amine, and
5-Fluoro-N4-isopropyl-N2-(5-piperazin-1-yl-pyridin-2-yl)-pyrimidine-2,4-diamine.

11. Use of a compound according to any one of claims 1 to 10, or a pharmaceutically acceptable salt, ester or amide thereof, in the manufacture of a medicament for the treatment of disorders or conditions caused by abnormal cell proliferation in a mammal.

12. Use according to claim 11 wherein the disorder is selected from the group consisting of vascular smooth muscle proliferation associated with atherosclerosis, postsurgical vascular stenosis and restenosis, and endometriosis.

13. Use of a compound according to any one of claims 1 to 10, or a pharmaceutically acceptable salt, ester or amide thereof, in the manufacture of a medicament for the treatment of disorder s or conditions caused by infections selected from the group consisting of viral infections such as DNA viruses like herpes and RNA viruses like HIV, and fungal infections in a mammal.

14. Use of a compound according to any one of claims 1 to 10, or a pharmaceutically acceptable salt, ester or amide thereof, in the manufacture of a medicament for the treatment of disorders selected from the group consisting of autoimmune diseases selected from the group consisting of psoriasis, inflammation like rheumatoid arthritis, lupus, type 1 diabetes, diabetic nephropathy, multiple sclerosis, glomerulonephritis, organ transplant rejection, including host versus graft disease in a mammal.

15. Use of a compound according to any one of claims 1 to 10, or a pharmaceutically acceptable salt, ester or amide thereof, in the manufacture of a medicament for the treatment of neurodegenerative disorders in a mammal.

16. Use according to claim 11 wherein the abnormal cell proliferation is a cancer selected from the group consisting of cancers of the breast, ovary, cervix, prostate, tests, esophagus, stomach, skin, lung, bone, colon, pancreas, thyroid, biliary passages, buccal cavity and pharynx (oral), lip, tongue, mouth, pharynx, small intestine, colon-rectum, large intestine, rectum, brain and central nervous system, glioblastoma, neuroblastoma, keratoacanthoma, epidermoid carcinoma, large cell carcinoma, adenocarcinoma, adenoma, follicular carcinoma, undifferentiated carcinoma, papillary carcinoma, seminoma, melanoma, sarcoma, bladder carcinoma, liver carcinoma, kidney carcinoma, myeloid disorders, lymphoid disorders, Hodgkin's, hairy cells, and leukemia.

## Patentansprüche

1. Verbindung der Formel I: 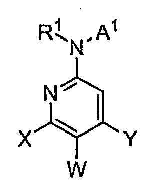 oder ein pharmazeutisch verträgliches Salz, ein pharmazeutisch verträglicher Ester oder ein pharmazeutisch verträgliches Amid davon,
worin:
A¹ ein monocyclisches oder bicyclisches heteroaromatisches Ringsystem ist, ausgewählt aus: 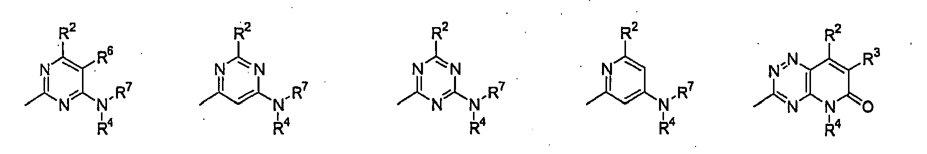 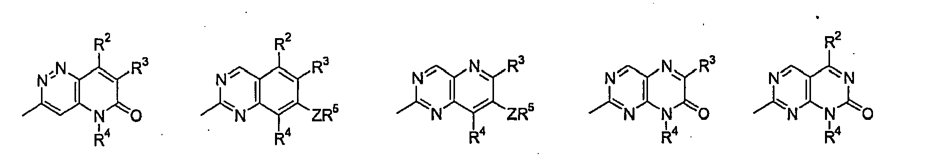 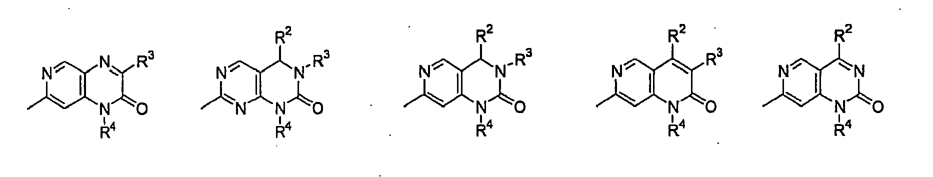 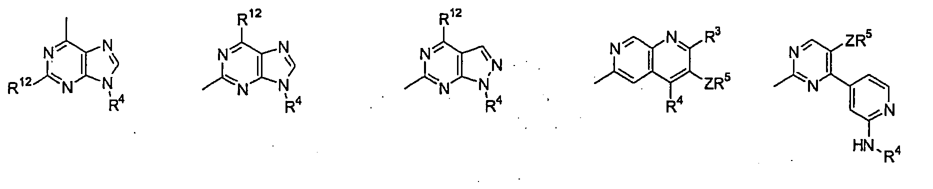
worin:
R¹ jeweils unabhängig Wasserstoff, C₁-C₆-Alkyl, C₁-C₆-Alkenyl, C₁-C₆-Acyl, Aryloxycarbonyl, C₁-C₁₀-Alkoxycarbonyl oder Tri-C₁-C₁₀-alkylsilyl ist;
R², R³, R⁴, R⁵, R⁸, R⁹, R¹⁰ und R¹¹ jeweils unabhängig ausgewählt sind aus Wasserstoff, C₁-C₁₀-Alkyl, C₁-C₁₀-Alkylamino, C₃-C₇-Cycloalkyl, Aryl, Heteroaryl und Heterocyclyl;
R⁶ jeweils unabhängig ausgewählt ist aus Wasserstoff, Halogen, Nitril, Nitro, C₁-C₈-Alkyl, C₂-C₈-Alkenyl, C₂-C₈-Alkinyl, C₁-C₁₀-Alkylcarbonyl, C₁-C₁₀-Akoxycarbonyl, C₃-C₇-Cycloalkyl, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C (O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-Aryl, (CR⁸R⁹)ₙ-Heteroaryl, -T(CH₂)ₘQR⁸, -C(O)T(CH₂)ₘQR⁸, NR⁸C(O)T(CH₂)ₘQR⁸, und -CR⁸=CR⁹C(O)R¹⁰;
R⁷ jeweils unabhängig Wasserstoff, C₁-C₁₀-Acyl, C₁-C₁₀-Alkoxycarbonyl, Aryloxycarbonyl, C₁-C₈-Alkyl oder C₂-C₈-Alkenyl ist;
R¹² jeweils unabhängig Wasserstoff, C₁-C₁₀-Acyl, Aryl-C₁-C₁₀-alkyl, C₁-C₁₀-Alkylamino oder Arylamino ist;
R⁸ und R⁹ gegebenenfalls eine carbocyclische Gruppe bilden können, die 3-7 Glieder, vorzugsweise 5-6 Glieder enthält von denen bis zu vier gegebenenfalls Heteroatome, unabhängig ausgewählt aus Sauerstoff, Schwefel und Stickstoff, sind, wobei die carbocyclische Gruppe unsubstituiert ist oder mit einer, zwei oder drei Gruppen substituiert ist, wobei die Gruppen jeweils unabhängig ausgewählt sind aus Halogen, Hydroxy, Hydroxy-C₁-C₁₀-alkyl, Nitril, C₁-C₄-Alkyl, C₁-C₄-Alkoxy, C₁-C₁₀-Alkoxycarbonyl, C₁-C₁₀-Alkylcarbonyl, C₁-C₁₀-Alkylcarbonylamino, Amino-C₁-C₁₀-alkyl, Trifluormethyl, N-Hydroxyacetamid, Trifluormethylalkyl, Amino oder Mono- oder Di-C₁-C₁₀-alkylamino, (CH₂)ₙC(O)NR¹⁰R¹¹, und O(CH₂)ₙC(O)OR¹⁰;
T jeweils unabhängig O, S, NR⁹, N(O)R⁹ oder CR⁹R¹⁰ ist;
Q jeweils unabhängig O, S, NR⁹, N(O)R⁹, CO₂, O(CH₂)ₙ-Heteroaryl, O(CH₂)ₙS(O)ₘR⁹, oder (CH₂)-Heteroaryl ist;
X und Y jeweils unabhängig ausgewählt sind aus Wasserstoff, Halogen, Nitril, C₁-C₆-Alkyl, C₁-C₆-Alkylcarbonyl, C₁-C₆-Alkoxycarbonyl, Nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR^{B}R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-Aryl, (CR⁸R⁹)ₙ-Heteroaryl, -T(CH₂)ₘQR⁸, -C(O)T(CH₂)ₘQR⁸, NR⁸C(O)T(CH₂)ₘQR⁸, und -CR⁸=CR⁹C(O)R¹⁰;
W ausgewählt ist aus Wasserstoff, Halogen, C₁-C₈-Alkyl, C₃-C₇-Cycloalkyl, C₁-C₈-Alkoxy, C₁-C₈-Alkoxyalkyl, C₁-C₈-Halogenalkyl, C₁-C₈-Hydroxyalkyl, C₂-C₈-Alkenyl, C₂-C₈-Alkinyl, Nitril, Nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹) ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-Aryl, (CR⁸R⁹)ₙ-Heteroaryl,
-T(CH₂)ₘQR⁸, -C(O)T(CH₂)ₘQR⁸, NR⁸C(O)T(CH₂)ₘQR⁹, und
-CR⁸=CR⁹C(O)R¹⁰;
W und eines von X oder Y gegebenenfalls einen aromatischen Ring bilden können, der bis zu drei Heteroatome enthält und gegebenenfalls mit bis zu 4 Gruppen substituiert ist, unabhängig ausgewählt aus Halogen, Hydroxy, Hydroxy-C₁-C₁₀-alkyl, C₁-C₄-Alkyl, C₁-C₄-Alkoxy, C₁-C₁₀-Alkoxycarbonyl, C₁-C₁₀-Alkylcarbonyl, C₁-C₁₀-Alkylcarbonylamino, und Amino-C₁-C₁₀-alkyl, Amino-C₁-C₁₀-alkylcarbonyl, Trifluormethyl, Trifluormethyl-C₁-C₁₀-alkyl, Trifluormethyl-C₁-C₁₀-alkylamino-C₁-C₁₀-alkyl, Amino, Mono- oder Di-C₁-C₁₀-alkylamino, N-Hydroxyacetamido, Aryl, Heteroaryl, Carboxy-C₁-C₁₀-alkyl, Nitril, NR⁸SO₂R⁹, C(O)NR⁸R⁹, NR⁸C(O)R⁹, C(O)OR⁸, C(O)NR⁸SO₂R⁹, (CH₂)ₙS(O)ₙR⁸, (CH₂)ₙ-Heteroaryl, O(CH₂)ₙ-Heteroaryl, (CH₂)ₙC(O)NR⁸R⁹, O(CH₂)ₙC(O)OR⁸, (CH₂)ₙSO₂NR⁸R⁹ und C(O)R⁸;
Z jeweils unabhängig 0 oder NR⁶ ist;
m eine ganze Zahl von 1-6 ist;
n eine ganze Zahl von 0-6 ist,
wobei der Ausdruck "Heterocyclyl" eine Cycloalkylgruppe bezeichnet, die auch wenigstens ein Heteroatom, ausgewählt aus 0, S, N und substituiertem Stickstoff, trägt;
der Ausdruck "Aryl" ein aromatisches Ringsystem ohne Heteroatome bezeichnet, das einen einzelnen Ring, mehrere Ringe oder mehrere kondensierte Ringe hat, von denen wenigstens einer aromatisch ist, das mono-, di- oder trisubstituiert sein kann;
der Ausdruck "Aryloxy" für "Aryl-O-" steht, worin "Aryl" wie oben definiert ist;
der Ausdruck "Heteroaryl" einen aromatischen Heterocyclus bezeichnet, der fünf oder sechs Ringglieder enthält, von denen 1 bis 4 Heteroatome, unabhängig ausgewählt aus N, S und O, sein können und wobei die Ringe unsubstituiert, monosubstituiert oder disubstituiert mit Substituenten sein können, unabhängig ausgewählt aus der Gruppe, bestehend aus Halogen, (C₁-C₄)-Alkyl und (C₁-C₄)-Alkoxy, gegebenenfalls substituiert mit ein bis drei Fluoratomen, und
der Ausdruck "Heteroaryloxy" für "Heteroaryl-O" steht, worin Heteroaryl wie oben definiert ist.

2. Verbindung gemäß Anspruch 1 mit der folgenden Struktur: 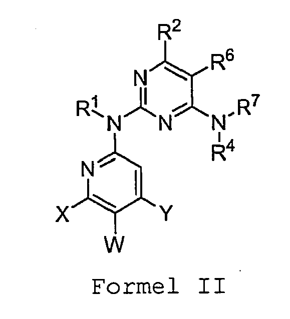 worin:
R¹, R², R⁴, R⁵, R⁶, R⁷, R⁸, R⁹, R¹⁰, R¹¹, T, Q, W, X, Y, m und n wie für Formel I definiert sind.

3. Verbindung gemäß Anspruch 1 oder Anspruch 2, wobei R¹ und R² jeweils unabhängig Wasserstoff sind.

4. Verbindung gemäß Anspruch 1 oder Anspruch 2, wobei R⁴ C₁-C₁₀-Alkyl ist.

5. Verbindung gemäß Anspruch 1 oder Anspruch 2, wobei R⁶ Halogen oder COR⁸ ist.

6. Verbindung gemäß Anspruch 1 oder Anspruch 2, wobei W NR⁸R⁹ ist.

7. Verbindung gemäß Anspruch 1, wobei Z O ist.

8. Verbindung gemäß Anspruch 1 oder Anspruch 2, wobei X und Y Wasserstoff sind.

9. Verbindung gemäß Anspruch 1 oder Anspruch 2, wobei R³ Halogen oder C₁-C₈-Alkyl ist.

10. Verbindung gemäß Anspruch 1, ausgewählt aus der Gruppe, bestehend aus:
4-Cyclopentylamino-2-(5-piperazin-1-yl-pyridin-2-ylamino)-pyrimidin-5-carbonitril,
N4-Cyclopentyl-5-nitro-N2-(5-piperazin-1-yl-pyridin-2-yl)-pyrimidin-2,4-diamin,
4-Cyclopentylamino-2-(5-piperazin-1-yl-pyridin-2-ylamino)-pyrimidin-5-carbaldehyd,
4-Cyclopentylamino-2-(5-piperazin-1-yl-pyridin-2-ylamino)-pyrimidin-5-carbonsäureethylester,
4-Cyclopentylamino-2-(5-piperazin-1-yl-pyridin-2-ylamino)-pyrimidin-5-carbonsäuremethylester,
[4-Cyclopentylamino-2-(5-piperazin-1-yl-pyridin-2-ylamino)-pyrimidin-5-yl]-methanol,
1-[4-Cyclopentylamino-2-(5-piperazin-1-yl-pyridin-2-ylamino)-pyrimidin-5-yl]-ethanon,
3-[4-Cyclopentylamino-2-(5-piperazin-1-yl-pyridin-2-ylamino)-pyrimidin-5-yl]-but-2-ensäureethylester,
4-Amino-2-(5-piperazin-1-yl-pyridin-2-ylamino)-pyrimidin-5-carbonitril,
5-Nitro-N2-(5-piperazin-1-yl-pyridin-2-yl)-pyrimidin-2,4-diamin,
4-Amino-2-(5-piperazin-1-yl-pyridin-2-ylamino)-pyrimidin-5-carbaldehyd,
4-Amino-2-(5-piperazin-1-yl-pyridin-2-ylamino)-pyrimidin-5-carbonsäureethylester,
4-Amino-2-(5-piperazin-1-yl-pyridin-2-ylamino)-pyrimidin-5-carbonsäuremethylester,
[4-Amino-2-(5-piperazin-1-yl-pyridin-2-ylamino)-pyrimidin-5-yl]-methanol,
1-[4-Amino-2-(5-piperazin-1-yl-pyridin-2-ylamino)-pyrimidin-5-yl]-ethanon,
3-[4-Amino-2-(5-piperazin-1-yl-pyridin-2-ylamino)-pyrimidin-5-yl]-but-2-ensäureethylester,
4-Cyclopentylamino-2-(5-pyrrolidin-1-yl-pyridin-2-ylamino)-pyrimidin-5-carbonitril,
N2-[5-(3-Aminopyrrolidin-1-yl)-pyridin-2-yl]-N4-cyclopentyl-5-nitro-pyrimidin-2,4-diamin,
4-Cyclopentylamino-2-(5-morpholin-4-yl-pyridin-2-ylamino)-pyrimidin-5-carbaldehyd,
4-Cyclopentylamino-2-(3,4,5,6-tetrahydro-2H-[1,3']-bi-pyridinyl-6'-ylamino)-pyrimidin-5-carbonsäureethylester,
4-Cyclopentylamino-6-methyl-2-(5-piperazin-1-yl-pyridin-2-ylamino)-pyrimidin-5-carbonsäuremethylester,
{2-[5-(Bismethoxymethylamino)-pyridin-2-ylamino]-4-cyclopentylaminopyrimidin-5-yl}-methanol,
1-[4-Benzylamino-2-(5-piperazin-1-yl-pyridin-2-ylamino)-pyrimidin-5-yl]-ethanon,
4-[4-Cyclopentylamino-2-(5-piperazin-1-yl-pyridin-2-ylamino)-pyrimidin-5-yl]-pent-3-en-2-on,
4-Amino-2-(pyridin-2-ylamino)-pyrimidin-5-carbonitril,
5-Nitro-N2-pyridin-2-yl-pyrimidin-2,4-diamin,
4-Amino-2-(pyridin-2-ylamino)-pyrimidin-5-carbaldehyd,
4-Amino-2-(pyridin-2-ylamino)-pyrimidin-5-carbonsäureethylester,
5-Brom-N2-(5-piperazin-1-yl-pyridin-2-yl)-pyrimidin-2,4-diamin,
[4-Amino-2-(5-morpholin-4-yl-pyridin-2-ylamino)-pyrimidin-5-yl]-methanol,
1-[4-Amino-2-(5-morpholin-4-yl-pyridin-2-ylamino)-pyrimidin-5-yl]-ethanon,
[6-(5-Acetyl-4-amino-pyrimidin-2-ylamino)-pyridin-3-yloxy]-essigsäure,
4-Cyclopentylamino-2-(4-hydroxymethyl-5-pyrrolidin-1-yl-pyridin-2-ylamino)-pyrimidin-5-carbonitril,
N2-[5-(3-Amino-pyrrolidin-1-yl)-6-chlor-pyridin-2-yl]-N4-cyclopentyl-5-nitropyrimidin-2,4-diamin,
2-(5-Brom-pyridin-2-ylamino)-4-cyclopentylaminopyrimidin-5-carbaldehyd,
4-Cyclopentylamino-2-(1H-pyrrolo[3,2-b]pyridin-5-ylamino)-pyrimidin-5-carbonsäureethylester,
4-Cyclopentylamino-2-(4,6-dichlor-5-piperazin-1-yl-pyridin-2-ylamino)-6-methylpyrimidin-5-carbonsäuremethylester,
2-(2-{5-[Bis-(2-methoxyethyl)-amino]-pyridin-2-ylamino}-4-cyclopentylaminopyrimidin-5-yl)-2-methylpropan-1-ol,
1-[4-Phenylamino-2-(5-piperazin-1-yl-pyridin-2-ylamino)-pyrimidin-5-yl]-ethanon,
4-[4-(3-Hydroxycyclopentylamino)-2-(5-piperazin-1-yl-pyridin-2-ylamino)-pyrimidin-5-yl]-pent-3-en-2-on,
4-[5-Cyano-2-(pyridin-2-ylamino)-pyrimidin-4-ylamino]-cyclohexancarbonsäure,
2-(4-Amino-5-nitro-pyrimidin-2-ylamino)-isonicotinsäure,
4-Amino-6-methyl-2-(pyridin-2-ylamino)-pyrimidine-5-carbaldehyd,
5-Iod-N2-pyridin-2-yl-pyrimidin-2,4-diamin,
N-[5-Brom-2-(5-piperazin-1-yl-pyridin-2-ylamino)-pyrimidin-4-yl]-acrylamid,
N2-(5-Piperazin-1-yl-pyridin-2-yl)-5-prop-1-inyl-pyrimidin-2,4-diamin,
5-[2-(4-Fluor-phenyl)-ethyl]-N2-(5-piperazin-1-yl-pyridin-2-yl)-pyrimidin-2,4-diamin,
[6-(4-Amino-5-propenyl-pyrimidin-2-ylamino)-pyridin-3-yloxy]-essigsäure,
5-Brom-N4-cyclopentyl-N2-(5-pyrrolidin-1-yl-pyridin-2-yl)-pyrimidin-2,4-diamin,
N2-[5-(3-Amino-pyrrolidin-1-yl)-6-chlor-pyridin-2-yl]-5-brom-N4-cyclopentylpyrimidin-2,4-diamin,
5-Brom-N4-cyclopentyl-N2-(5-piperazin-1-yl-pyridin-2-yl)-pyrimidin-2,4-diamin,
5-Brom-N4-cyclopentyl-N2-(1H-pyrrolo[3,2-b]pyridin-5-yl)-pyrimidin-2,4-diamin,'
5-Brom-N4-cyclopentyl-N2-(4,6-dichlor-5-piperazin-1-yl-pyridin-2-yl)-6-methylpyrimidin-2,4-diamin,
N2-{5-[Bis-(2-methoxy-ethyl)-amino]-pyridin-2-yl}-5-brom-N4-cyclopentylpyrimidin-2,4-diamin,
5-Brom-N4-phenyl-N2-(5-piperazin-1-yl-pyridin-2-yl)-pyrimidin-2,4-diamin,
3-[5-Brom-2-(5-piperazin-1-yl-pyridin-2-ylamino)-pyrimidin-4-ylamino]-cyclopentanol,
N4-Cyclopentyl-5-iod-N2-(5-pyrrolidin-1-yl-pyridin-2-yl)-pyrimidin-2,4-diamin,
N2-[5-(3-Amino-pyrrolidin-1-yl)-6-chlor-pyridin-2-yl]-N4-cyclopentyl-5-iodpyrimidin-2,4-diamin,
N4-Cyclopentyl-5-iod-N2-(5-piperazin-1-yl-pyridin-2-yl)-pyrimidin-2,4-diamin,
N4-Cyclopentyl-5-iod-N2-(1H-pyrrolo[3,2-b]pyridin-5-yl)-pyrimidin-2,4-diamin,
4-[6-(5-Brom-4-cyclopentylaminopyrimidin-2-ylamino)-pyridin-3-yl]-piperazin-1-carbonsäure-tert-butylester,
4-[6-(4-Cyclopentylamino-5-formylpyrimidin-2-ylamino)-pyridin-3-yl]-piperazin-1-carbonsäure-tert-butylester,
4-[6-(5-Acetyl-4-cyclopentylaminopyrimidin-2-ylamino)-pyridin-3-yl]-piperazin-1-carbonsäure-tert-butylester,
2-[5-(4-tert-Butoxycarbonyl-piperazin-1-yl)-pyridin-2-ylamino]-4-cyclopentylaminopyrimidin-5-carbonsäureethylester,
N-Cyclopentyl-N'-(5-piperazin-1-yl-pyridin-2-yl)-pyrimidin-4,6-diamin,
N-Isopropyl-N'-(5-piperazin-1-yl-pyridin-2-yl)-pyrimidin-4,6-diamin,
4-[6-(6-Cyclopentylaminopyrimidin-4-ylamino)-pyridin-3-yl]-piperazin-1-carbonsäureester-tert-butylester,
N-[5-(3-Aminopyrrolidin-1-yl)-pyridin-2-yl]-N'-cyclopentyl-pyrimidin-4,6-diamin,
4-{6-[4-Cyclopentylamino-5-(1-methyl-3-oxo-but-1-enyl)-pyrimidin-2-ylamino-pyridin-3-yl}-piperazin-1-carbonsäure-tert-butylester,
N-Cyclopentyl-N'-(5-piperazin-1-yl-pyridin-2-yl)-[1,3,5]triazin-2,4-diamin,
1-[4-Cyclopentylamino-2-(5-piperazin-1-yl-pyridin-2-ylamino)-pyrimidin-5-yl]-ethanon,
5-Brom-N4-cyclopentyl-N2-(5-piperazin-1-yl-pyridin-2-yl)-pyridin-2,4-diamin,
4-Cyclopentylamino-6-(5-piperazin-1-yl-pyridin-2-ylamino)-nicotinonitril,
N4-Cyclopentyl-5-nitro-N2-(5-piperazin-1-yl-pyridin-2-yl)-pyridin-2,4-diamin,
4-Cyclopentylamino-6-(5-piperazin-1-yl-pyridin-2-ylamino)-pyridin-3-carbaldehyd,
4-Cyclopentylamino-6-(5-piperazin-1-yl-pyridin-2-ylamino)-nicotinsäureethylester,
4-Cyclopentylamino-6-(5-piperazin-1-yl-pyridin-2-ylamino)-nicotinsäuremethylester,
[4-Cyclopentylamino-6-(5-piperazin-1-yl-pyridin-2-ylamino)-pyridin-3-yl]-methanol,
1-[4-Cyclopentylamino-6-(5-piperazin-1-yl-pyridin-2-ylamino)-pyridin-3-yl]-ethanon,
3-[4-Cyclopentylamino-6-(5-piperazin-1-yl-pyridin-2-ylamino)-pyridin-3-yl]-but-2-ensäureethylester,
(5-Cyclopentyl-5,6-dihydropyrido[2,3-e][1,2,4]triazin-3-yl)-(5-piperazin-1-yl-pyridin-2-yl)-amin,
(8-Cyclopentyl-7-methoxychinazolin-2-yl)-(5-piperazin-1-yl-pyridin-2-yl)-amin,
(8-Cyclopentyl-7-methoxypyrido[3,2-d]pyrimidin-2-yl)-(5-piperazin-1-yl-pyridin-2-yl)-amin,
6-Acetyl-8-cyclopentyl-2-(5-piperazin-1-yl-pyridin-2-ylamino)-8H-pteridin-7-on,
3-Acetyl-1-cyclopentyl-7-(5-piperazin-1-yl-pyridin-2-ylamino)-1H-pyrido[3,4-b]pyrazin-2-on,
1-Cyclopentyl-3-ethyl-4-methyl-7-(5-piperazin-1-yl-pyridin-2-ylamino)-3,4-dihydro-1H-pyrimido[4,5-d]pyrimidin-2-on,
1-Cyclopentyl-3-ethyl-4-methyl-7-(5-piperazin-1-yl-pyridin-2-ylamino)-3,4-dihydro-1H-pyrido[4,3-d]pyrimidin-2-on,
3-Acetyl-1-cyclopentyl-4-methyl-7-(5-piperazin-1-yl-pyridin-2-ylamino)-1H-[1,6]naphthyridin-2-on,
(9-Isopropyl-6-methyl-9H-purin-2-yl)-(5-piperazin-1-yl-pyridin-2-yl)-amin,
2-[9-Isopropyl-6-(5-piperazin-1-yl-pyridin-2-ylamino)-9H-purin-2-ylamino]-ethanol,
N2-(4-Amino-cyclohexyl)-9-cyclopentyl-N6-(5-piperazin-1-yl-pyridin-2-yl)-9H-purin-2,6-diamin,
2-[9-Isopropyl-6-(5-piperazin-1-yl-pyridin-2-ylamino)-9H-purin-2-ylamino]-3-methylbutan-1-ol,
(1 -Isopropyl-4-methyl-1H-pyrazolo[3,4-d]pyrimidin-6-yl)-(5-piperazin-1-yl-pyridin-2-yl)-amin,
2-[1-Isopropyl-4-(5-piperazin-1-yl-pyridin-2-ylamino)-1H-pyrazolo[3,4-d]pyrimidin-6-ylamino]-ethanol,
N6-(4-Amino-cyclohexyl)-1-cyclopentyl-N4-(5-piperazin-1-yl-pyridin-2-yl)-1H-pyrazolo[3,4-d]pyrimidin-4,6-diamin,
2-[1-Isopropyl-4-(5-piperazin-1-yl-pyridin-2-ylamino)-1H-pyrazolo[3,4-d]pyrimidin-6-ylamino]-3-methylbutan-1-ol,
5-Cyclopentyl-7-(1-hydroxyethyl)-8-methyl-3-(5-piperazin-1-yl-pyridin-2-ylamino)-5H-pyrido[3,2-c]pyridazin-6-on,
5-Cyclopentyl-8-methyl-3-(5-piperazin-1-yl-pyridin-2-ylamino)-5H-pyrido[3,2-c]pyridazin-6-on,
7-Benzyl-5-cyclopentyl-3-(5-piperazin-1-yl-pyridin-2-ylamino)-5H-pyrido[3,2-c]pyridazin-6-on,
(2-Ethyl-4-isopropyl-3-methoxy[1,7]naphthyridin-6-yl)-pyridin-2-yl-amin,
(2,4-Diisopropyl-3-methoxy[1,7]naphthyridin-6-yl)-(5-isopropenylpyridin-2-yl)-amin,
[4-(2-Ethylaminopyridin-4-yl)-pyrimidin-2-yl]-(5-piperazin-1-yl-pyridin-2-yl)-amin,
[4-(5-Ethyl-2-methylaminopyridin-4-yl)-pyrimidin-2-yl]-(5-morpholin-4-yl-pyridin-2-yl)-amin,
[5-Methoxy-4-(2-methylaminopyridin-4-yl)-pyrimidin-2-yl]-(5-morpholin-4-yl-pyridin-2-yl)-amin, und
5-Fluor-N4-isopropyl-N2-(5-piperazin-1-yl-pyridin-2-yl)-pyrimidin-2,4-diamin.

11. Verwendung einer Verbindung gemäß einem der Ansprüche 1 bis 10 oder eines pharmazeutisch verträglichen Salzes, Esters oder Amids davon bei der Herstellung eines Medikaments für die Behandlung von Störungen oder Zuständen, die durch abnormale Zellproliferation verursacht werden, bei einem Säuger.

12. Verwendung gemäß Anspruch 11, wobei die Störung aus der Gruppe, bestehend aus Proliferation des glatten Gefäßmuskels verbunden mit Atherosklerose, postchirurgischer Gefäßstenose und Restenose und Endometriose, ausgewählt ist.

13. Verwendung einer Verbindung gemäß einem der Ansprüche 1 bis 10 oder eines pharmazeutisch verträglichen Salzes, Esters oder Amids davon bei der Herstellung eines Medikaments zur Behandlung von Störungen oder Zuständen, die durch Infektionen verursacht sind, ausgewählt aus der Gruppe, bestehend aus Virusinfektionen, z.B. durch DNA-Viren wie Herpes und RNA-Viren wie HIV und Pilzinfektionen, bei einem Säuger.

14. Verwendung einer Verbindung gemäß einem der Ansprüche 1 bis 10 oder eines pharmazeutisch verträglichen Salzes, Esters oder Amids davon bei der Herstellung eines Medikaments für die Behandlung von Störungen, ausgewählt aus der Gruppe, bestehend aus Autoimmunerkrankungen, ausgewählt aus der Gruppe, bestehend aus Psoriasis, Entzündung wie rheumatoider Arthritis, Lupus, Typ 1-Diabetes, diabetischer Nephropathie,' multipler Sklerose, Glomerulonephritis, Organtransplantatabstoßung, einschließlich Host-versus-Kraft-Erkrankung, bei einem Säuger.

15. Verwendung einer Verbindung gemäß einem der Ansprüche 1 bis 10 oder eines pharmazeutisch verträglichen Salzes, Esters oder Amids davon bei der Herstellung eines Medikaments zur Behandlung von neurodegenerativen Störungen bei einem Säuger.

16. Verwendung gemäß Anspruch 11, wobei die abnormale Zellproliferation ein Krebs ist, ausgewählt aus der Gruppe, bestehend aus Krebs der Brust, der Eierstöcke, der Cervix, der Prostata, der Testis, des Ösophagus, des Magens, der Haut, der Lunge, des Knochens, des Colons, des Pancreas, der Schilddrüse, der Gallenwege, der Mundhöhle und des Pharynx (oraler), der Lippen, der Zunge, des Mundes, des Pharynx, des Dünndarms, des Colon-Rektums, des Dickdarms, des Rektums, des Gehirns und des zentralen Nervensystems, Glioblastom, Neuroblastom, Keratoacanthom, Epidermoidkarzinom, großzelligem Karzinom, Adenokarzinom, Adenom, Follikelkarzinom, undifferenziertem Karzinom, Papillenkarzinom, Seminom, Melanom, Sarkom, Blasenkarzinom, Leberkarzinom, Nierenkarzinom, Myeloidstörungen, Lymphoidstörungen, Hodgkinscher Erkrankung, Haarzell-Leukämie und Leukämie.

## Revendications

1. Composé de formule I : 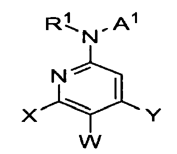 ou un de ses sels, esters ou amides pharmaceutiquement acceptables,
formule dans laquelle :
A¹ représente un système de noyau hétéroaromatique monocyclique ou bicyclique choisi entre : 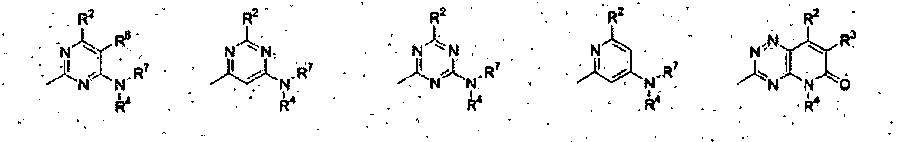 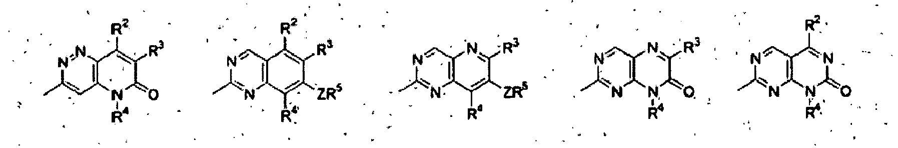 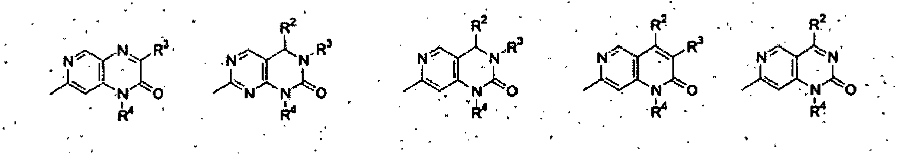 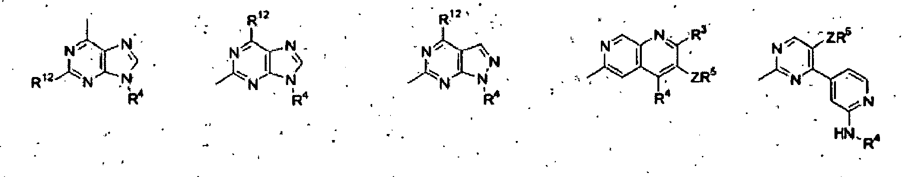
dans laquelle : ,
R¹ représente dans chaque cas, indépendamment, un atome d'hydrogène, un groupe alkyle en C₁ à C₆, alcényle en C₁ à C₆, acyle en C₁ à C₆, aryloxycarbonyle, (alkoxy en C₁ à C₁₀)carbonyle ou tri (alkyle en C₁ à C₁₀)silyle ;
R², R³, R⁴, R⁵, R⁸, R⁹, R¹⁰ et R¹¹ sont choisis dans chaque cas, indépendamment, entre un atome d'hydrogène, des groupes alkyle en C₁ à C₁₀, alkylamino en C₁ à C₁₀, cycloalkyle en C₃ à C₇, aryle, hétéroaryle et hétérocyclyle ;
R⁶ est choisi, indépendamment, dans chaque cas, entre un atome d'hydrogène, un atome d'halogène, des groupes nitrile, nitro, alkyle en C₁ à C₈, alcényle en C₂ à C₈, alcynyle en C₂ à C₈, (alkyle en C₁ à C₁₀)carbonyle, (alkoxy en C₁ à C₁₀)carbonyle, cycloalkyle en C₃ à C₇, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-aryle, (CR⁸R⁹)ₙ-hétéroaryle, -T(CH₂)ₘQR⁸, -C(O)T(CH₂)ₘQR⁸, NR⁸C(O)T(CH₂)ₘQR⁸, et -CR⁸=CR⁹C(O)R¹⁰ ;
R⁷ représente indépendamment, dans chaque cas, un atome d'hydrogène, un groupe acyle en C₁ à C₁₀, (alkoxy en C₁ à C₁₀)carbonyle, aryloxycarbonyle, alkyle en C₁ à C₈ ou alcényle en C₂ à C₈,
R¹² représente indépendamment, dans chaque cas, un atome d'hydrogène, un groupe acyle en C₁ à C₁₀, aryl(alkyle en C₁ à C₁₀), alkylamino en C₁ à C₁₀ ou arylamino ;
R⁸ et R⁹ peuvent former facultativement un groupe carbocyclique contenant 3 à 7 membres, de préférence 5 ou 6 membres, dont jusqu'à quatre représentent facultativement des hétéroatomes choisis indépendamment entre l'oxygène, le soufre et l'azote, le groupe carbocyclique étant non substitué ou substitué avec un, deux ou trois groupes, lesdits groupes étant choisis dans chaque cas, indépendamment, entre des groupes halogéno, hydroxy, hydroxyalkyle en C₁ à C₁₀, nitrile, alkyle en C₁ à C₄, alkoxy en C₁ à C₄, (alkoxy en C₁ à C₁₀)carbonyle, (alkyle en C₁ à C₁₀)carbonyle, (alkyle en C₁ à C₁₀)carbonylamino, aminoalkyle en C₁ à C₁₀, trifluorométhyle, N-hydroxyacétamide, trifluorométhylalkyle, amino ou mono- ou di-(alkyle en C₁ à C₁₀)amino, (CH₂)ₙC(O)NR¹⁰R¹¹ et O(CH₂)ₙC(O)OR¹⁰ ;
T représente dans chaque cas, indépendamment, O, S, un groupe NR⁹, N(O)R⁹ ou CR⁹R¹⁰ ;
Q représente dans chaque cas, indépendamment, O, S, un groupe NR⁹, N(O)R⁹, CO₂, O(CH₂)ₙ-hétéroaryle, O(CH₂)ₙS(O)ₘR⁹, ou (CH₂)-hétéroaryle ;
X et Y sont choisis dans chaque cas, indépendamment, entre un atome d'hydrogène, un atome d'halogène, des groupes nitrile, alkyle en C₁ à C₆, (alkyle en C₁ à C₆)carbonyle, (alkoxy en C₁ à C₆)carbonyle, nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹)ₙ-aryle, (CR⁸R⁹)ₙ-hétéroaryle , -T(CH₂)ₘQR⁸, -C(O)T(CH₂)ₘQR⁸, NR⁸C(O)T(CH₂)ₘQR⁸, et -CR⁸=CR⁹C(O)R¹⁰ ;
W est choisi entre un atome d'hydrogène, un atome d'halogène, des groupes alkyle en C₁ à C₈, cycloalkyle en C₃ à C₇, alkoxy en C₁ à C₈, alkoxyalkyle en C₁ à C₈, halogénalkyle en C₁ à C₈, hydroxyalkyle en C₁ à C₈, alcényle en C₂ à C₈, alcynyle en C₂ à C₈, nitrile, nitro, OR⁸, SR⁸, NR⁸R⁹, N(O)R⁸R⁹, P(O)(OR⁸)(OR⁹), (CR⁸R⁹)ₙNR¹⁰R¹¹, COR⁸, (CR⁸R⁹)ₙC(O)R¹⁰, CO₂R⁸, CONR⁸R⁹, C(O)NR⁸SO₂R⁹, NR⁸SO₂R⁹, C(O)NR⁸OR⁹, S(O)ₙR⁸, SO₂NR⁸R⁹, (CR⁸R⁹)ₙP(O)(OR¹⁰)(OR¹¹), (CR⁸R⁹) ₙ-aryle, (CR⁸R⁹)ₙ-hétéroaryle, -T(CH₂)ₘQR⁸, -C(O)T(CH₂)ₘQR⁸, NR⁸C(O)T(CH₂)ₘQR⁸, et -CR⁸=CR⁹C(O)R¹⁰ ;
W et un de X et Y peuvent former facultativement un noyau aromatique contenant jusqu'à trois hétéroatomes et facultativement substitué avec jusqu'à 4 groupes choisis indépendamment entre des groupes halogéno, hydroxy, hydroxyalkyle en C₁ à C₁₀, alkyle en C₁ à C₄, alkoxy en C₁ à C₄, (alkoxy en C₁ à C₁₀)carbonyle, (alkyle en C₁ à C₁₀)carbonyle, (alkyle en C₁ à C₁₀)carbonylamino et aminoalkyle en C₁ à C₁₀, amino(alkyle en C₁ à C₁₀)carbonyle, trifluorométhyle, trifluorométhyl(alkyle en C₁ à C₁₀), trifluorométhyl(alkyle en C₁ à C₁₀)amino(alkyle en C₁ à C₁₀), amino, mono- ou di-(alkyle en C₁ à C₁₀)amino, N-hydroxyacétamido, aryle, hétéroaryle, carboxy(alkyle en C₁ à C₁₀), nitrile, NR⁸SO₂R⁹, C(O)NR⁸R⁹, NR⁸C(O)R⁹, C(O)OR⁸, C(O)NR⁸SO₂R⁹, (CH₂)ₙS(O)ₙR⁸, (CH₂)ₙ-hétéroaryle, O(CH₂)ₙ-hétéroaryle, (CH₂)ₙC(O)NR⁸R⁹, O(CH₂)ₙC(O)OR⁸, (CH₂)ₙSO₂NR⁸R⁹ et C(O)R⁸ ;
Z représente dans chaque cas, indépendamment, O ou un groupe NR⁶,
m représente un nombre entier de 1 à 6,
n représente un nombre entier de 0 à 6,
le terme « hétérocyclyle » désigne un groupe cycloalkyle portant également au moins un hétéroatome choisi entre O, S, N ou un atome d'azote substitué,
le terme « aryle » désigne un système de noyau aromatique sans aucun hétéroatome, comprenant un noyau unique, des noyaux multiples ou des noyaux multiples condensés dans lesquels au moins un noyau est aromatique, qui peut être mono-, di- ou trisubstitué,
le terme « aryloxy » désigne un groupe «aryl-O- » dans lequel le terme « aryle » répond à la définition précitée,
le terme « hétéroaryle » désigne un hétérocycle aromatique contenant cinq ou six membres dans le noyau, dont 1 à 4 peuvent être des hétéroatomes choisis, indépendamment, entre N, S et O, noyaux qui peuvent être non substitués, monosubstitués ou disubstitués avec des substituants choisis, indépendamment, dans le groupe consistant en des substituants halogéno, alkyle en C₁ à C₄ et alkoxy en C₁ à C₄, facultativement substitués avec un à trois atomes de fluor et
le terme « hétéroaryloxy » désigne un groupe « hétéroaryl-O », dans lequel le terme « hétéroaryle » répond à la définition précitée.

2. Composé suivant la revendication 1, ayant la structure suivante : 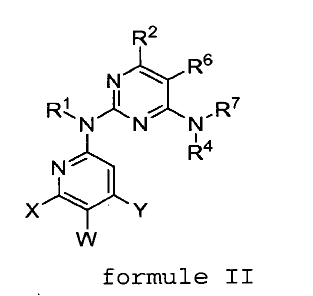 dans laquelle :
R¹, R², R⁴, R⁵, R⁶, R⁷, R⁸, R⁹, R¹⁰, R¹¹, T, Q, W, X, Y, m et n répondent aux définitions indiquées pour la formule I.

3. Composé suivant la revendication 1 ou la revendication 2, dans lequel R¹ et R² représentent indépendamment, dans chaque cas, un atome d'hydrogène.

4. Composé suivant la revendication 1 ou la revendication 2, dans lequel R⁴ représente un groupe alkyle en C₁ à C₁₀.

5. Composé suivant la revendication 1 ou la revendication 2, dans lequel R⁶ représente un atome d'halogène ou un groupe COR⁸.

6. Composé suivant la revendication 1 ou la revendication 2, dans lequel W représente un groupe NR⁸R⁹.

7. Composé suivant la revendication 1, dans lequel Z représente O.

8. Composé suivant la revendication 1 ou la revendication 2, dans lequel X et Y représentent un atome d'hydrogène.

9. Composé suivant la revendication 1 ou la revendication 2, dans lequel R³ représente un atome d'halogène ou un groupe alkyle en C₁ à C₈.

10. Composé suivant la revendication 1, choisi dans le groupe consistant en :
le 4-cyclopentylamino-2-(5-pipérazine-1-yl-pyridine-2-ylamino)pyrimidine-5-carbonitrile,
la N4-cyclopentyl-5-nitro-N2-(5-pipérazine-1-yl-pyridine-2-yl)pyrimidine-2,4-diamine,
le 4-cyclopentylamino-2-(5-pipérazine-1-yl-pyridine-2-ylamino)pyrimidine-5-carbaldéhyde,
l'ester éthylique d'acide 4-cyclopentylamino-2-(5-pipérazine-1-yl-pyridine-2-ylamino)pyrimidine-5-carboxylique,
l'ester méthylique d'acide 4-cyclopentylamino-2-(5-pipérazine-1-yl-pyridine-2-ylamino)pyrimidine-5-carboxylique,
[4-cyclopentylamino-2-(5-pipérazine-1-yl-pyridine-2-ylamino)pyrimidine-5-yl]méthanol,
la 1-[4-cyclopentylamino-2-(5-pipérazine-1-yl-pyridine-2-ylamino)pyrimidine-5-yl]éthanone,
l'ester éthylique d'acide 3-(4-cyclopentylamino-2-(5-pipérazine-1-yl-pyridine-2-ylamino)pyrimidine-5-yl]-but-2-énoïque,
le 4-amino-2-(5-pipérazine-1-yl-pyridine-2-ylamino)-pyrimidine-5-carbonitrile,
la 5-nitro-N2-(5-pipérazine-1-yl-pyridine-2-yl)-pyrimidine-2,4-diamine,
le 4-amino-2-(5-pipérazine-1-yl-pyridine-2-ylamino)pyrimidine-5-carbaldéhyde,
l'ester éthylique d'acide 4-amino-2-(5-pipérazine-1-yl-pyridine-2-ylamino)pyrimidine-5-carboxylique,
l'ester méthylique d'acide 4-amino-2-(5-pipérazine-1-yl-pyridine-2-ylamino)pyrimidine-5-carboxylique,
le [4-amino-2-(5-pipérazine-1-yl-pyridine-2-ylamino)-pyrimidine-5-yl]méthanol,
la 1-[4-amino-2--(5-pipérazine-1-yl-pyridine-2-ylamino)-pyrimidine-5-yl]éthanone,
l'ester éthylique d'acide 3-[4-amino-2-(5-pipérazine-1-yl-pyridine-2-ylamino)pyrimidine-5-yl]-but-2-énoïque,
le 4-cyclopentylamino-2-(5-pyrrolidine-1-yl-pyridine-2-ylamino)pyrimidine-5-carbonitrile,
la N2-[5-(3-aminopyrrolidine-1-yl)pyridine-2-yl]-N4-cyclopentyl-5-nitropyrimidïne-2,4-diamine,
le 4-cyclopentylamino-2-(5-morpholine-4-yl-pyridine-2-ylamino)pyrimidine-5-carbaldéhyde,
l'ester éthylique d'acide 4-cyclopentylamino-2-(3,4,5,6-tétrahydro-2H-[1,3']bipyridinyl-6'-ylamino)-pyrimidine-5-carboxylique,
l'ester méthylique d'acide 4-cyclopentylamino-6-méthyl-2-(5-pipérazine-1-yl-pyridine-2-ylamino)pyrimidine-5-carboxylique,
le {2-[5-(bis-méthoxyméthylamino)pyridine-2-ylamino]-4-cyclopentylaminopyridimine-5-yl}méthanol,
la 1-[4-benzylamino-2-(5-pipérazine-1-yl-pyridine-2-ylamino)pyrimidine-5-yl]éthanone,
la 4-[4-cyclopentylamino-2-(5-pipérazine-1-yl-pyridine-2-ylamino)pyrimidine-5-yl]pent-3-ène-2-one,
le 4-amino-2-(pyridine-2-ylamino)pyrimidine-5-carbonitrile,
la 5-nitro-N2-pyridine-2-yl-pyrimidine-2,4-diamine,
le 4-amino-2-(pyridine-2-ylamino)pyrimidine-5-carbaldéhyde,
l'ester éthylique d'acide 4-amino-2-(pyridine-2-ylamino)-pyrimidine-5-carboxylique,
la 5-bromo-N2-(5-pipérazine-1-yl-pyridine-2-yl)-pyrimidine-2,4-diamine,
le [4-amino-2-(5-morpholine-4-yl-pyridine-2-ylamino)-pyrimidine-5-yl]méthanol,
la 1-[4-amino-2-(5-morpholine-4-yl-pyridine-2-ylamino)-pyrimidine-5-yl]éthanone,
l'acide [6-(5-acétyl-4-aminopyrimidine-2-ylamino)-pyridine-3-yloxy]acétique,
le 4-cyclopentylamino-2-(4-hydroxyméthyl-5-pyrrolidine-1-yl-pyridine-2-ylamino)pyrimidine-5-carbonitrile,
le N2-[5-(3-aminopyrrolidine-1-yl)-6-chloropyridine-2-yl]-N4-cyclopentyl-5-nitropyrimidine-2,4-diamine,
le 2-(5-bromopyridine-2-ylamino)-4-cyclopentylamino-pyrimidine-5-carbaldéhyde,
l'ester éthylique d'acide 4-cyclopentylamino-2-(1H-pyrrolo[3,2-b]pyridine-5-ylamino)pyrimidine-5-carboxylique,
l'ester méthylique d'acide 4-cyclopentylamino-2-(4,6-dichloro-5-pipérazine-1-yl-pyridine-2-ylamino)-6-méthyl-pyrimidine-5-carboxylique,
le 2-(2-{5-[bis-(2-méthoxyéthyl)amino]pyridine-2-ylamino}-4-cyclopentylaminopyrimidine-5-yl)-2-méthylpropane-1-ol,
la 1-[4-phénylamino-2-(5-pipérazine-1-yl-pyridine-2-ylamino)pyrimidine-5-yl]éthanone,
la 4-[4-(3-hydroxycyclopentylamino)-2-(5-pipérazine-1-yl-pyridine-2-ylamino)pyrimidine-5-yl]pent-3-ène-2-one,
l'acide 4-[5-cyano-2-(pyridine-2-ylamino)pyrimidine-4-ylamino]cyclohexanecarboxylique,
l'acide 2-(4-amino-5-nitropyrimidine-2-ylamino)-isonicotinique,
le 4-amino-6-méthyl-2-(pyridine-2-ylamino)pyrimidine-5-carbaldéhyde,
la 5-iodo-N2-pyridine-2-yl-pyrimidine-2,4-diamine,
le N-[5-bromo-2-(5-pipérazine-1-yl-pyridine-2-ylamino)-pyrimidine-4-yl]acrylamide,
la N2(5-pipérazine-1-yl-pyridine-2-yl)-5-prop-1-ynyl-pyrimidine-2,4-diamine,
la 5-[2-(4-fluorophényl)éthyl]-N2-(5-pipérazine-1-yl-pyridine-2-yl)pyrimidine-2,4-diamine,
l'acide [6-(4-amino-5-propényl-pyrimidine-2-ylamino)-pyridine-3-yloxy]acétique,
la 5-bromo-N4-cyclopentyl-N2-(5-pyrrolidine-1-yl-pyridine-2-yl)pyrimidine-2,4-diamine,
la N2-[5-(3-aminopyrrolidine-1-yl)-6-chloropyridine-2-yl]-5-bromo-N4-cyclopéntylpyrimidine-2,4-diamine,
la 5-bromo-N4-cyclopentyl-N2-(5-pipérazine-1-yl-pyridine-2-yl)pyrimidine-2,4-diamine,
la 5-bromo-N4-cyclopentyl-N2-(1H-pyrrolo[3,2-b]-pyridine-5-yl)pyrimidine-2,4-diamine,
la 5-bromo-N4-cyclopentyl-N2-(4,6-dichloro-5-pipérazine-1-yl-pyridine-2-yl)-6-méthylpyrimidine-2,4-diamine,
la N2-{5-[bis-(2-méthoxyéthyl)amino]pyridine-2-yl}-5-bromo-N4-cyclopentylpyrimidine-2,4-diamine,
la 5-bromo-N4-phényl-N2-(5-pipérazine-1-yl-pyridine-2-yl)pyrimidine-2,4-diamine,
le 3-[5-bromo-2-(5-pipérazine-1-yl-pyridine-2-ylamino)-pyrimidine-4-ylamino]cyclopentanol,
la N4-cyclopentyl-5-iodo-N2-(5-pyrrolidine-1-yl-pyridine-2-yl)pyrimidine-2,4-diamine,
la N2-[5-(3-aminopyrrolidine-1-yl)-6-chloropyridiné-2-yl]-N4-cyclopentyl-5-iodopyrimidine-2,4-diamine,
la N4-cyclopentyl-5-iodo-N2-(5-pipéraziné-1-yl-pyridine-2-yl)pyrimidine-2,4-diamine,
la N4-cyclopentyl-5-iodo-N2-(1H-pyrrolo[3,2-b]-pyridine-5-yl)pyrimidine-2,4-diamine,
l'ester tertiobutylique d'acide 4-[6-(5-bromo-4-cyclo-pentylaminopyrimidine-2-ylamino)pyridine-3-yl]-pipérazine-1-carboxylique,
l'ester tertiobutylique d'acide 4-[6-(4-cyclopentylamino-5-formylpyrimidine-2-ylamino)pyridine-3-yl]pipérazine-1-carboxylique,
l'ester tertiobutylique d'acide 4-[6-(5-acétyl-4-cyclo-pentylaminopyrimidine-2-ylamino)pyridine-3-yl]pipérazine-1-carboxylique,
l'ester éthylique d'acide 2-[5-(4-tertiobutoxycarbonyl-pipérazine-1-yl)pyridine-2-ylamino]-4-cyclopentylaminopyrimidine-5-carboxylique,
la N-cyclopentyl-N'-(5-pipérazine-1-yl-pyridine-2-yl)-pyrimidine-4,6-diamine,
la N-isopropyl-N'-(5-pipérazine-1-yl-pyridine-2-yl)-pyrimidine-4,6-diamine,
l'ester tertiobutylique d'acide 4-[6-(6-cyclopentylamino-pyrimidine-4-ylamino)-pyridine-3-yl]-pipérazine-1-carboxylique,
la N-[5-(3-amino-pyrrolidine-1-yl)-pyridine-2-yl]-N'-cyclopentyl-pyrimidine-4,6-diamine,
l'ester tertiobutylique d'acide 4-{6-[4-cyclopentylamino-5-(1-méthyl-3-oxo-but-1-ényl)pyrimidine-2-ylamino]-pyridine-3-yl}pipérazine-1-carboxylique,
la N-cyclopentyl-N'-(5-pipérazine-1-yl-pyridine-2-yl)-[1,3,5]triazine-2,4-diamine,
la 1- [4-cyclopentylamino-2- (5-pipérazine-1-yl-pyridine-2-ylamino)-pyrimidine-5-yl]-éthanone,
la 5-bromo-N4-cyclopentyl-N2-(5-pipérazine-1-yl)-pyridine-2-yl)-pyridine-2,4-diamine,
la 4-cyclopentylamino-6-(5-pipérazine-1-yl-pyridine-2-ylamino)nicotinonitrile,
la N4-cyclopentyl-5-nitro-N2-(5-pipérazine-1-yl-pyridine-2-yl)pyridine-2,4-diamine,
le 4-cyclopentylamino-6-(5-pipérazine-1-yl-pyridine-2-ylamino)pyridine-3-carbaldéhyde,
l'ester éthylique d'acide 4-cyclopentylamino-6-(5-pipérazine-1-yl-pyridine-2-ylamino)nicotinique,
l'ester méthylique d'acide 4-cyclopentylamino-6-(5-pipérazine-1-yl-pyridine-2-ylamino)nicotinique,
le [4-cyclopentylamino-6-(5-pipérazine-1-yl-pyridine-2-ylamino)pyridine-3-yl]méthanol,
la 1-[4-cyclopentylamino-6-(5-pipérazine-1-yl-pyridine-2-ylamino)pyridine-3-yl]éthanone,
l'ester éthylique d'acide 3-[4-cyclopentylamino-6-(5-pipérazine-1-yl-pyridine-2-ylamino)pyridine-3-yl]but-2-énoïque,
la (5-cyclopentyl-5,6-dihydropyrido[2,3-e][1,2,4]triazine-3-yl)-(5-pipérazine-1-yl-pyridine-2-yl)amine,
la (8-cyclopentyl-7-méthoxyquinazoline-2-yl)-(5-pipérazine-1-yl-pyridine-2-yl)amine,
la (8-cyclopentyl-7-méthoxypyrido[3,2-d]pyrimidine-2-yl)-(5-pipérazine-1-yl-pyridine-2-yl)amine,
la 6-acétyl-8-cyclopentyl-2-(5-pipérazine-1-yl-pyridine-2-ylamino)-8H-ptéridine-7-one,
la 3-acétyl-1-cyclopentyl-7-(5-pipérazine-1-yl-pyridine-2-ylamino)-1H-pyrido[3,4-b]pyrazine-2-one,
la 1-cyclopentyl-3-éthyl-4-méthyl-7-(5-pipérazine-1-yl-pyridine-2-ylamino)-3,4-dihydro-1H-pyrimido[4,5-d]pyrimidine-2-one,
la 1-cyclopentyl-3-éthyl-4-méthyl-7-(5-pipérazine-1-yl-pyridine-2-ylamino)-3,4-dihydro-1H-pyrido[4,3-d]pyrimidine-2-one,
la 3-acétyl-1-cyclopentyl-4-méthyl-7-(5-pipérazine-1-yl-pyridine-2-ylamino)-1H-[1,6]naphtyridine-2-one,
la (9-isopropyl-6-méthyl-9H-purine-2-yl)-(5-pipérazine-1-yl-pyridine-2-yl)amine,
le 2-(9-isopropyl-6-(5-pipérazine-1-yl-pyridine-2-ylamino)-9H-purine-2-ylamino]éthanol,
la N2-(4-aminocyclohexyl)-9-cyclopentyl-N6-(5-pipérazine-1-yl-pyridine-2-yl)-9H-purine-2,6-diamine,
le 2-(9-isopropyl-6-(5-pipérazine-1-yl-pyridine-2-ylamino)-9H-purine-2-ylamino]-3-méthylbutane-1-ol,
la (1-isopropyl-4-méthyl-1H-pyrazolo[3,4-d]pyrimidine-6-yl)-(5-pipérazine-1-yl-pyridine-2-yl)amine,
le 2-(1-isopropyl-4-(5-pipérazine-1-yl-pyridine-2-ylamino)-1H-pyrazolo[3,4-d]pyrimidine-6-ylamino]éthanol,
la N6-(4-aminocyclohexyl)-1-cyclopentyl-N4-(5-pipérazine-1-yl-pyridine-2-yl)-1H-pyrazolo[3,4-d]pyrimidine-4,6-diamine,
le 2-[1-isopropyl-4-(5-pipérazine-1-yl-pyridine-2-ylamino)-1H-pyrazolo[3,4-d]pyrimidine-6-ylamino]-3-méthylbutane-1-ol,
la 5-cyclopentyl-7-(1-hydroxyéthyl)-8-méthyl-3-(5-pipérazine-1-yl-pyridine-2-ylamino)-5H-pyrido[3,2-c]pyridazine-6-one,
la 5-cyclopentyl-8-méthyl-3-(5-pipérazine-1-yl-pyridine-2-ylamino)-5H-pyrido(3,2-c]pyridazine-6-one,
la 7-benzyl-5-cyclopentyl-3-(5-pipérazine-1-yl-pyridine-2-ylamino)-5H-pyrido[3,2-c]pyridazine-6-one,
la (2-éthyl-4-isopropyl-3-méthoxy-(1,7]naphtyridine-6-yl)pyridine-2-yl-amine,
la (2,4-diisopropyl-3-méthoxy-[1,7]naphtyridine-6-yl)-(5-isopropénylpyridine-2-yl)amine,
la [4-(2-éthylaminopyridine-4-yl)pyrimidine-2-yl]-(5-pipérazine-1-yl-pyridine-2-yl)amine,
la [4-(5-éthyl-2-méthylaminopyridine-4-yl)pyrimidine-2-yl]-(5-morpholine-4-yl-pyridine-2-yl)amine,
la [5-méthoxy-4-(2-méthylaminopyridine-4-yl)pyrimidine-2-yl]-(5-morpholine-4-yl-pyridine-2-yl)amine, et
la 5-fluoro-N4-isopropyl-N2-(5-pipérazine-1-yl-pyridine-2-yl)pyrimidine-2,4-diamine.

11. Utilisation d'un composé suivant l'une quelconque des revendications 1 à 10, ou d'un de ses sels, esters ou amides pharmaceutiquement acceptables, dans la production d'un médicament destiné au traitement de troubles ou d'affections provoqués par une prolifération cellulaire anormale chez un mammifère.

12. Utilisation suivant la revendication 11, dans laquelle le trouble est choisi dans le groupe consistant en la prolifération du tissu musculaire lisse vasculaire associée à l'athérosclérose, la sténose et la resténose vasculaires postchirurgicales et l'endométriose.

13. Utilisation d'un composé suivant l'une quelconque des revendications 1 à 10, ou d'un de ses sels, esters ou amides pharmaceutiquement acceptables, dans la production d'un médicament destiné au traitement de troubles ou d'affections provoqués par des infections choisies dans le groupe consistant en des infections virales telles que des infections par des virus à ADN tels que les virus de l'herpès et des virus à ARN tels que le VIH, et des infections fongiques chez un mammifère.

14. Utilisation d'un composé suivant l'une quelconque des revendications 1 à 10, ou d'un de ses sels, esters ou amides pharmaceutiquement acceptables, dans la production d'un médicament destiné au traitement de troubles choisis dans le groupe consistant en des maladies auto-immunes choisies dans le groupe consistant en le psoriasis, une inflammation du type de la polyarthrite rhumatoïde, le lupus, le diabète de type I, la néphropathie diabétique, la sclérose en plaques, la glomérulonéphrite, le rejet d'un transplant d'organe, y compris la réaction de l'hôte contre le greffon, chez un mammifère.

15. Utilisation d'un composé suivant l'une quelconque des revendications 1 à 10, ou d'un de ses sels, esters ou amides pharmaceutiquement acceptables, dans la production d'un médicament destiné au traitement de troubles neurodégénératifs chez un mammifère.

16. Utilisation suivant la revendication 11, dans laquelle la prolifération cellulaire anormale est un cancer choisi dans le groupe consistant en les cancers du sein, des ovaires, du col de l'utérus, de la prostate, des testicules, de l'oesophage, de l'estomac, de la peau, du poumon, des os, du côlon, du pancréas, de la thyroïde, des voies biliaires, de la cavité buccale, et du pharynx (cancer buccal), des lèvres, de la langue, de la bouche, du pharynx, de l'intestin grêle, du côlon-rectum, du gros intestin, du rectum, du cerveau, du système nerveux central, un glioblastome, un neuroblastome, un kérato-acanthome, un carcinome épidermoïde, un carcinome à grandes cellules, un adénocarcinome, un adénome, un carcinome folliculaire, un carcinome indifférencié, un carcinome papillaire, un séminome, un mélanome, un sarcome, le carcinome de la vessie, le carcinome hépatique, le carcinome rénal, des troubles myéloïdes, des troubles lymphoïdes, la maladie d'Hodgkin, un cancer à tricholeucocytes et une leucémie.
